# EUROPEAN PATENT APPLICATION

(11) **EP 2 264 144 A1**
(43) Date of publication of application: **22.12.2010**
(21) Application number: 09715319.1
(22) Date of filing: 25.02.2009
(51) Int. Cl.: C12N 1/21, C12N 15/09, C12P 19/32

(54) **PROCESS FOR PRODUCTION OF 5'-GUANYLIC ACID**

(30) Priority: 25.02.2008 JP 2008043136
(71) Applicant: Ajinomoto Co., Inc., Tokyo 104-8315 (JP)
(72) Inventor: ASAHARA, Takayuki, Kawasaki-shi Kanagawa 210-8681 (JP); FUKADA, Hiroaki, Kawasaki-shi Kanagawa 210-8681 (JP); MATSUNO, Kiyoshi, Kawasaki-shi Kanagawa 210-8681 (JP)
(74) Representative: Strehl Schübel-Hopf & Partner
(86) International application number: PCT/JP2009/053358
(87) International publication number: WO 2009/107631

(57) **Abstract**

By reacting inosinic acid (IMP) with a bacterium which has been modified so that IMP dehydrogenase activity and 5'-guanylic acid (GMP) synthetase activity are enhanced, GMP is produced.

## Description

### Technical Field

The present invention relates to a method for producing 5'-guanylic acid and a novel microorganism used in production thereof. 5'-guanylic acid is useful as seasonings, medicines and raw materials thereof.

### Background Art

As methods for industrially producing 5'-guanylic acid (guanosine-5'-monophosphate, hereinafter also referred to as "GMP"), methods wherein guanosine is produced by a fermentation method and the obtained guanosine is enzymatically phosphorylated to obtain 5'-guanylic acid have been known (Patent Documents 1 to 4).

In addition, a method of production wherein a microorganism belonging to the genus *Escherichia* having an increased GMP synthetase activity and *Brevibacterium ammoniagenes* having a high activity to biosynthesize adenosine triphosphate (ATP) (hereinafter referred to as "regenerate ATP"), which is generated by glucose metabolism and necessary for the synthesis of GMP from 5'-xanthylic acid (XMP), are cultured in medium containing XMP and ammonia or glutamine, and GMP is generated and accumulated in the medium by converting XMP to GMP with high efficiency has been known (Non-patent document 1).

Meanwhile, methods for producing GMP by a fermentation method have been suggested. For example, Patent document 5 discloses a method for producing GMP characterized by culturing a mutant strain of the genus *Bacillus* having adenine auxotrophy and showing resistance to decoyinine or methionine sulfoxide, as well as having ability to produce GMP, followed by collecting GMP generated and accumulated in a medium. Also, Patent document 6 discloses a method for producing GMP characterized by culturing a strain of bacteria belonging to the genus *Escherichia* having ability to produce inosinic acid (inosine 5'-monophosphate, hereinafter referred to as "IMP") wherein two types of 5'-nucleotidase genes are deleted and IMP dehydrogenase gene and GMP synthetase gene are amplified, and collecting GMP generated and accumulated in a medium. Yet, in general, direct fermentation of GMP does not provide sufficient yield and is not necessarily practical compared with the above-described enzymatic method.

Thus far, no case producing GMP from IMP as a raw material by using bacteria belonging to the genus *Escherichia* with enhanced activities of IMP dehydrogenase and GMP synthetase has been reported.
Patent document 1: JP 07-231793
Patent document 2: JP 10-201481
Patent document 3: WO96/37603
Patent document 4: JP 2001-245676
Patent document 5: JP 56-12438
Patent document 6: JP 2002-355087
Non-patent document 1: Tatsuro Fujio et al., Biosci. Biotech. Biochem., 1997, Vol. 61, No. 5, p.840-845

### Disclosure of the Invention

An object of the present invention is to provide a method for producing GMP from IMP using a microorganism and to create a novel microorganism to be used in the method which can convert IMP to GMP with high efficiency.

In order to solve the above-described object, the inventors of the present invention intensively studied and found that by using a microorganism belonging to the genus *Escherichia* which has been modified so that IMP dehydrogenase activity and GMP synthetase activity are enhanced, IMP is converted to GMP with high efficiency and at high speed, and also succeeded in creation of a novel microorganism capable of converting IMP to GMP efficiently, thereby completed the present invention.

It is an aspect of the present invention to provide a method for producing 5'-guanylic acid, comprising:
generating 5'-guanylic acid by reacting inosinic acid with a microorganism having an ability to convert inosinic acid into 5'-guanylic acid; and
collecting 5'-guanylic acid, wherein said microorganism has been modified so that inosinic acid dehydrogenase activity and 5'-guanylic acid synthetase activity are enhanced.
It is another aspect of the present invention to provide the method as described above, wherein inosinic acid dehydrogenase activity and 5'-guanylic acid synthetase activity are enhanced by increasing expression of guaB gene and guaA gene.
It is another aspect of the present invention to provide the method as described above, wherein said guaA gene encodes a protein selected from the group consisting of
(A) A protein comprising the amino acid sequence of SEQ ID NO: 2, and
(B) A protein comprising the amino acid sequence of SEQ ID NO: 2 but which includes substitution, deletion, insertion, addition or inversion of one or several amino acids, and has 5'-guanylic acid synthetase activity.
It is another aspect of the present invention to provide the method as described above, wherein said guaB gene encodes a protein selected from the group consisting of
(C) A protein comprising the amino acid sequence of SEQ ID NO: 10, and
(D) A protein comprising the amino acid sequence of SEQ ID NO: 10 but which includes substitution, deletion, insertion, addition or inversion of one or several amino acids, and has inosinic acid dehydrogenase activity.
It is another aspect of the present invention to provide the method as described above, wherein said microorganism has been further modified so that one or more genes selected from ushA gene and aphA gene do not function normally.
It is another aspect of the present invention to provide the method as described above, wherein said microorganism has been further modified so that one or more genes selected from purR gene, add gene and purA gene do not function normally.
It is another aspect of the present invention to provide the method as described above, wherein said microorganism has been further modified so that nagD gene does not function normally.
It is another aspect of the present invention to provide the method as described above, wherein said microorganism is selected from the group consisting of bacteria belonging to the *Enterobacteriaceae* family, *Bacillus* bacteria and Coryneform bacteria
It is another aspect of the present invention to provide the method as described above, wherein said microorganism is an *Escherichia* bacterium.
It is another aspect of the present invention to provide the method as described above, wherein said microorganism is *Escherichia coli.*
It is another aspect of the present invention to provide a microorganism having an ability to convert inosinic acid into 5'-guanylic acid, which has been modified so that inosinic acid dehydrogenase activity and 5'-guanylic acid synthetase activity are enhanced by increasing expression of guaB gene and guaA gene, and has been further modified so that ushA gene, aphA gene and nagD gene do not function normally.
It is another aspect of the present invention to provide the microorganism as described above, which has been further modified so that one or more genes selected from purR gene, add gene and purA gene do not function normally.
It is another aspect of the present invention to provide the microorganism as described above, which is selected from the group consisting of bacteria belonging to the *Enterobacteriaceae* family, *Bacillus* bacteria and Coryneform bacteria.
It is another aspect of the present invention to provide the microorganism as described above which is an *Escherichia* bacterium.
It is another aspect of the present invention to provide the microorganism as described above which is an *Escherichia coli.*

### Brief Description of the Drawings

[Figure 1] Figure 1 is a graph showing change in the concentration of IMP, XMP and GMP when JM109ΔushAΔaphAΔpurR/pUC18-guaBA strain was reacted with IMP.
[Figure 2] Figure 2 is a graph showing change in the concentration of IMP (A) and GMP (B) when JM109ΔushAΔaphAΔpurR/pUC18-guaBA strain and JM109ΔushAΔaphAΔpurRΔnagD/pUC18-guaBA strain were reacted with IMP.
[Figure 3] (A) A graph showing change in the concentration of IMP and GMP when JM109ΔushAΔaphAΔpurRΔnagD/pUC18-guaBA strain was reacted with IMP. (B) A graph showing change in the concentration of XMP and GMP when JM109ΔushAΔaphAΔpurRΔnagD/pUC18-guaBA strain was reacted with XMP. (C) A graph showing the accumulated amount of GMP when JM109ΔushAΔaphAΔpurRΔnagD/pUC18-guaBA strain was reacted with IMP or XMP.
[Figure 4] (A) A graph showing change in the concentration of IMP, XMP and GMP when MG1655ΔushAΔaphAΔpurAΔpurRΔadd/pUC18-guaBA strain was reacted with IMP. (B) A graph showing the accumulated amount of GMP when MG1655ΔushAΔaphAΔpurAΔpurRΔadd/pUC18-guaBA strain was reacted with IMP or XMP.

### DESCRIPTION OF THE PREFERRED EMBODIMENTS

The present invention will now be described in detail.

### <I> Microorganism to be used in the method of the present invention

A microorganism to be used in the method of the present invention has been modified so that IMP dehydrogenase activity and GMP synthetase activity are enhanced and has an ability to convert IMP to GMP.

Examples of the microorganisms include bacteria belonging to the family Enterobacteriaceae, Coryneform bacteria and *Bacillus* bacteria.
Examples of the bacteria belonging to the family Enterobacteriaceae include *Escherichia* bacteria, *Pantoea* bacteria, *Enterobacter* bacteria, *Klebsiella* bacteria, *Serratia* bacteria, *Erwinia* bacteria, *Salmonella* bactetia and *Morganella* bacteria *Escherichia* bacteria are not restricted as long as they belongs to the genus *Fschenclua*, and include *Escherichia coli.* Yet, specifically, ones described in Neidhardt et al. (Neidhardt, FR. C. et al., Escherichia coli and Salmonella Typhimurium, American Society for Microbiology, Washington D. C., 1208, table 1) can be used. Also, examples of *Enterobacter* bacteria include *Enterobacter agglomerans*, *Enterobacter aerogenes* and the like, and examples of *Pantoea* bacteria include *Pantoea arumatis* and the like.

Examples of Coryneform bacteria include bacteria classified as Coryneform bacteria according to classification known to those skilled in the art of microorganisms, bacteria which used to be classified as the genus *Brevibacterium* but currently re-classified as the genus *Corynebacterium* (Int. J. Syst. Bacteriol., 41, 255 (1991)) and bacteria belonging to the genus *Brevibacterium* which is close to the genus *Corynebacterium*. Examples of such Coryneform bacteria are listed below.
*Corynebacterium acetoacidophilum*,
*Corynebacterium acetoglutamicum*,
*Corynebacterium alkanolyticum*,
*Corynebacterium callunae*,
*Corynebacterium glutamicum*,
*Corynebacterium lilium*,
*Corynebacterium melassecola*,
*Corynebacterium thermoaminogenes*,
*Corynebacterium harculis*,
*Brevibacterium divaricatum*,
*Brevibacterium flavum*,
*Brevibacterium immariophilum*,
*Brevibacterium lactofermentum*,
*Brevibacterium roseum*,
*Brevibacterium saccharolyticum*,
*Brevibacterium thiogenitalis*,
*Corynebacterium ammoniagenes*,
*Brevibacterium album*,
*Brevibacterium cerinum*,
*Microbacterium ammoniaphilum*,

As bacteria belonging to the genus *Bacillus*, bacteria classified as the genus *Bacillus* according to the classification known to those skilled in the art of microorganisms can be used. Specific examples include, but not limited to, *Bacillus subtilis, Bacillus amyloliquefaciens* and the like.

A method for enhancing activities of IMP dehydrogenase (a product of guaB gene) and GMP synthetase (a product of guaA gene) in the above-mentioned microorganisms will now be described.
In the present invention, "modified so that the IMP dehydrogenase activity and GMP synthetase activity are enhanced" means that IMP dehydrogenase activity and GMP synthetase activity are higher than those of an unmodified strain, for example a wild type strain, of microorganisms such as bacteria belonging to the genus *Escherichia*.

IMP dehydrogenase is an enzyme catalyzing the reaction below and "IMP dehydrogenase activity" refers to an activity catalyzing a reaction generating XMP from IMP. The IMP dehydrogenase activity can be measured, for example, by the method of Gilbert (Gilbert, H. J., Lowe, C. R and Drabble, W. T., Biochem J., 1979, Dec 1,183(3), 481-94).

NAD+ + IMP + H₂O → NADH H+ + XMP

Also, GMP synthetase is an enzyme (EC 6.3.4.1) catalyzing the reaction below and "GMP synthetase activity" means an activity catalyzing a reaction generating GMP form XMP.

ATP + XMP + NH₃ → AMP + pyrophosphoric acid + GMP

The GMP synthetase activity can be measured, for example, by examining a decreasing rate of NADH by the method of Spector (Spector, T., Methods Enzymol., 1978, 51, p219).

In order to increase IMP dehydrogenase activity and GMP synthetase activity, it is preferable to increase expression level of guaB gene and guaA gene encoding these enzymes respectively. An example of a method for increasing the expression level includes increasing the copy number of the DNA encoding IMP dehydrogenase and DNA encoding GMP synthetase in bacterial cells. In order to increase the copy number in the cells, DNA fragment each encoding IMP dehydrogenase or GMP synthetase may be linked to a vector functioning in microorganisms such as *Escherichia* bacteria to create a recombinant DNA, which is then introduced into a host for transformation. The copy number of guaB gene and guaA gene may be simultaneously or independently increased. In the case of *Escherichia coli*, guaB gene and guaA gene form an operon (GenBank accession No. M10101), so guaBA operon may be linked to a vector to create a recombinant DNA, which is then introduced into a host for transformation. The copy number of the genes encoding IMP dehydrogenase and GMP synthetase (guaB gene and guaA gene) increases in cells of a transformed strain, resulting in increased activities of IMP dehydrogenase and GMP synthetase.

The increase in the copy number in the cells can also be attained by allowing multiple copies of IMP dehydrogenase gene and GMP synthetase gene to exist on the chromosomal DNA of the above-mentioned host. Introduction of multiple copies of IMP dehydrogenase gene and GMP synthetase gene on the chromosomal DNA of bacteria belonging to the genus *Escherichia* can be carried out by homologous recombination by using a sequence existing on the chromosome with multiple copies as a target As the sequence existing on the chromosome with multiple copies, repetitive DNAs, inverted repeats present at the end of transposons or the like can be used. Alternatively, as disclosed in Japanese Patent Application Laid-Open Publication No. 2-109985, it is also possible that multiple copies of the desired gene can be introduced on the choromosomal DNA by integrating the desired gene on the transposon and transferring the gene. Either way, an increase in the copy number of IMP dehydrogenase gene and GMP synthetase gene in the transformant strain results in increased activities of IMP dehydrogenase and GMP synthetase.

Examples of the vector for introducing the gene above include plasmid vectors such as pSTV29, pMW218 or pUC19; and phage vectors such as λ 1059, λ BF101 or M13mp9. Also, examples of the transposon include Mu, Tn10 and Tn5.

Since nucleotide sequences of DNAs encoding IMP dehydrogenase and GMP synthetase are known, those DNAs can be obtained by synthesizing primers based on those sequences and amplifying by PCR using the chromosomal DNA of microorganisms such as an *Escherichia* bacterium as a template. An example of guaA gene of *Escherichia coli* includes one comprising the nucleotide sequence of SEQ ID NO: 1. An example of guaB gene of *Escherichia coli* includes one comprising the nucleotide sequence of SEQ ID NO: 9. As for these genes, the desired DNA fragments can be selected by preparing probes based on the nucleotide sequence and carrying out hybridization from a chromosomal DNA library of *Escherichia* bacterium. Alternatively, the DNA fragments encoding IMP dehydrogenase and GMP synthetase may be chemically synthesized based on the known nucleotide sequences. Also, guaBA operon of *Escherichia coli* can be cloned using primers of SEQ ID NOs: 31 and 32.

In addition, genes encoding proteins having functions equivalent to IMP dehydrogenase and GMP synthetase from microorganisms other than *Escherichia* bacteria may be obtained based on the above-mentioned nucleotide sequences.
An example of GMP synthetase gene (guaA) of *Bacillus subtilis* includes one comprising the nucleotide sequence of SEQ ID NO: 3. The amino acid sequence encoded by this nucleotide sequence is shown in SEQ ID NO: 4. An example ofGMP synthetase gene (guaA) of *Corynebacterium glutamicum* includes one comprising the nucleotide sequence shown in SEQ ID NO: 5. The amino acid sequence encoded by this nucleotide sequence is shown in SEQ ID NO: 6.
An example ofGMP synthetase gene (guaA) of *Corynebacterium ammoniagenes* includes one comprising the nucleotide sequence of SEQ ID NO: 7. The amino acid sequence encoded by this nucleotide sequence is shown in SEQ ID NO: 8.
An example of IMP dehydrogenase gene (guaB) of *Bacillus subtilis* includes one comprising the nucleotide sequence of SEQ ID NO: 11. The amino acid sequence encoded by this nucleotide sequence is shown in SEQ ID NO: 10.
An example of IMP dehydrogenase gene (guaB) of *Corynebacterium glutamicum* includes one comprising the nucleotide sequence of SEQ ID NO: 13. The amino acid sequence encoded by this nucleotide sequence is shown in SEQ ID NO: 14.
An example of IMP dehydrogenase gene (guaB) of *Corynebacterium ammoniagenes* includes one comprising the nucleotide sequence of SEQ ID NO: 15. The amino acid sequence encoded by this nucleotide sequence is shown in SEQ ID NO:16. As described above, since differences may exist in the nucleotide sequence of guaA gene and guaB gene by a genus, species or strain to which a microorganism belongs, guaA gene and guaB gene may be variants of the above-mentioned gene.

As long as the protein encoded by guaA gene has GMP synthetase activity, it may be one having a sequence identity of not less than 80%, preferably not less than 90%, more preferably 95%, particularly preferably not less than 98% to the entire amino acid sequence of SEQ ID NO: 2. Also, as long as the protein encoded by guaB gene has IMP dehydrogenase activity, it may be one having a sequence identity of not less than 80%, preferably not less than 90%, more preferably 95%, particularly preferably not less than 98% to the entire amino acid sequence of SEQ ID NO: 10. The identity of amino acid sequences and of nucleotide sequences can be determined by using, for example, algorithm BLAST by Karlin and Altschul (Pro. Natl. Acad. Sci. USA, 90, 5873 (1993)) or FASTA by Pearson (Methods Enzymol., 183, 63 (1990)). Based on this algorithm BLAST, programs called BLASTN and BLASTX have been developed (See http://www.ncbi.nlm.nih.gov).

Further, guaA gene and guaB gene to be used in the present invention are not restricted to wild type genes. As long as a function of an encoded protein, that is, GMP synthetase activity or IMP dehydrogenase activity, is not impaired, the gene may be a mutant or artificially modified one encoding a protein comprising the amino acid sequence of SEQ ID NO: 2 or 10 bu which includes substitution, deletion, insertion, addition or inversion of one or several amino acids at one or more positions. The term, "several" varies depending on the position of the amino acid residues in a spatial structure of the protein and the type of the amino acid residues, and specifically means 2 to 20 amino acids, preferably 2 to 10 amino acids, more preferably 2 to 5 amino acids. The substitution is preferably a conservative substitution, and the term "conservative substitution" means mutual substitution among Phe, Trp and Tyr in cases where the substitution site has an aromatic amino acid, among Leu, Ile and Val in cases where the substitution site has a hydrophobic amino acid, between Gln and Asn in cases where the substitution site has a polar amino acid, among Lys, Arg and His in cases where the substitution site has a basic amino acid, between Asp and Glu in cases where the substitution site has an acidic amino acid, and between Ser and Thr in cases where the substitution site has an amino acid having a hydroxyl group. Examples of the conservative substitution include substitution of Ala by Ser or Thr, substitution of Arg by Gln, His or Lys, substitution of Asn by Glu, Gln, Lys, His or Asp, substitution of Asp by Asn, Glu or Gln, substitution of Cys by Ser or Ala, substitution of Gln by Asn, Glu, Lys, His, Asp or Arg, substitution of Glu by Gly, Asn, Gln, Lys or Asp, substitution of Gly by Pro, substitution of His by Asn, Lys, Gln, Arg or Tyr, substitution of Ile by Leu, Met, Val or Phe, substitution of Leu by Ile, Met, Val or Phe, substitution of Lys by Asn, Glu, Gln, His or Arg, substitution of Met by Ile, Leu, Val or Phe, substitution of Phe by Trp, Tyr, Met, Ile or Leu, substitution of Ser by Thr or Ala, substitution of Thr by Ser or Ala, substitution of Trp by Phe or Tyr, substitution of Tyr by His, Phe or Trp, and substitution of Val by Met, Ile or Leu. Examples of the substitution, deletion, insertion, addition, inversion or the like of amino acids as described above also include those occurring due to naturally occurring mutations (mutants and variants), such as ones based on individual differences and species differences among microorganisms having guaA gene or guaB gene.

Also, guaA gene or guaB gene may be DNA capable of hybridizing with a complement of the nucleotide sequence of SEQ ID NO: 1 or 9 respectively, or with a probe which can be prepared from the sequence under stringent conditions and encoding a protein having GMP synthetase activity or IMP dehydrogenase activity. The term, "stringent conditions" herein refers to conditions under which the so-called specific hybrids form, and non-specific hybrids do not form. It is difficult to clearly express these conditions with numerical values. For example, stringent conditions include conditions under which DNAs having high identity, for instance, DNAs having an identity of not less than 80%, preferably not less than 90%, more preferably not less than 95%, particularly preferably not less than 98%, hybridize to each other, and DNAs having an identity of less than that do not hybridize to each other. Alternatively, the stringent conditions are exemplified by conditions where washing is carried out once, more preferably 2-3 times at a salt concentration and temperature corresponding to ordinary conditions of washing in Southern hybridization, i.e. 60°C, 1X SSC, 0.1% SDS, preferably 0.1X SSC, 0.1% SDS, more preferably 68°C, 0.1X SSC, 0.1% SDS.

Description of the above-mentioned gene variant is similarly applied to ushA gene, aphA gene, purR gene, add gene, purA gene and other genes, which are described later.

Introduction of DNA into microorganisms can be carried out by the method of C. T. chung (C. T. chung et al., Proc. Natl. Acad. Sci. USA, 86, 2172-2175 (1989)), the method of D. M. Morrison (Methods in Enzymology, 68, 326 (1979)), a method for increasing permeability of DNA by treating recipient bacterial cells with calcium chloride (Mandel, M. and Higa, A., J. Mol. Biol., 53,159 (1970)) or the like.

An increase in expression level of IMP dehydrogenase gene and GMP synthetase gene can also be attained by, besides the above-mentioned gene amplification, replacing an expression regulatory sequence, such as a promoter of IMP dehydrogenase gene and GMP synthetase gene, on the chromosomal DNA or a plasmid with a stronger one. For instance, a lac promoter, a trp promoter, a trc promoter and the like are known to be a strong promoter. Also, as disclosed in WO00/18935, by introducing nucleotide substitutions of several nucleotides in a promoter region of a gene, it can be altered to a stronger one. These promoter substitutions or modification strengthen expression of IMP dehydrogenase gene and GMP synthetase gene and increase activities of both proteins. The increase in expression level of guaB gene and guaA gene can be detected by Northern method, RT-PCR method or the like.

Since expression of guaBA operon encoding IMP dehydrogenase and GMP synthetase, as well as purine operon is suppressed by PurR protein, it is preferable that purR gene (GenBank accession J04212: SEQ ID NO: 17) encoding PurR protein be modified so as not to function normally, more preferably modified to decrease its expression in bacteria to be used in the method of the present invention. The modification of purR gene so that it does not function normally can be carried out by the method below. For instance, by a homologous recombination method using a gene recombination method (Experiments in Molecular Genetics, Cold Spring Harbor Laboratory press (1972); Matsuyama, S. and Mizushima, S., J. Bacteriol., 162,1196 (1985)), purR gene on the chromosome can be replaced with purR gene which does not function normally (hereinafter, also referred to as "disrupted purR gene"). In homologous recombination, when a plasmid with a sequence having a homology with a sequence on the chromosome or the like is introduced into bacteria cells, the recombination takes place at the sequence having the homology at a certain frequency and the introduced plasmid is entirely incorporated into the chromosome. Thereafter, if further recombination takes place at a site of the sequence having the homology on the chromosome, the plasmid drops. At that time, depending on a location at which the recombination takes place, the disrupted gene may be incorporated into the chromosome whereas the original normal gene may drop from the chromosome together with the plasmid. By selecting such a strain, a strain in which the normal purR gene on the chromosome has been replaced with the disrupted purR gene can be obtained.

Examples of such gene substitution using the homologous recombination include a method using linear DNA such as a combined method (see WO2005/010175) combining a method called "Red-driven integration" (Proc. Natl. Acad. Sci. USA, 2000, vol. 97, No. 12, p6640-6645) and an excision system derived from λ phage (J. Bacteriol. 2002 Sep; 184(18): 5200-3.) and a method using a plasmid containing temperature-sensitive replication origin (Proc. Natl. Acad. Sci. USA, 2000, vol. 97, No. 12, p6640-6645, U.S. Patent No. 6303383 or Japanese Patent Application Laid-Open Publication No. 05-007491).
Also, site-specific mutagenesis by gene substitution using the homologous recombination described above can be carried out by using a plasmid with no replication ability in a host. Also, the disruption of purR gene can be carried out by using a plasmid containing purR gene in which a marker gene such as drug resistance is inserted, the plasmid being incapable of replicating in the host microorganism. That is, in a transformant which has been transformed with the above-mentioned plasmid and acquired the drug resistance, the marker gene is incorporated in the chromosomal DNA. Since it is highly possible that this marker gene is incorporated by homologous recombination between purR gene sequence at both of its ends and these genes on the chromosome, the gene disrupted strain can be efficiently selected.

Specifically, the disrupted type purR gene to be used in the gene disruption can be obtained by decreasing or eliminating the activity of an encoded repressor or decreasing or eliminating transcription of purR gene by deleting a certain region of these genes by restriction enzyme digestion and ligation; by inserting another DNA fragment (such as a marker gene) in the gene; or introducing one or more nucleotide substitutions, deletions, insertions, additions or inversions in a nucleotide sequence of a coding region or promoter region of purR gene by a site-specific mutagenesis method (Kramer, W. and Frits, H. J., Methods in Enzymology, 154,350 (1987)) or by treatment with a chemical agent such as sodium hypochlorite or hydroxylamine (Shortle, D. and Nathans, D., Proc., Natl., Acad., Sci., U.S.A., 75,270 (1978)).

The sequence of purR gene is known and, based on the sequence, purR gene can be readily obtained by a PCR method or hybridization method. An example of purR gene of *E*. *coli* includes a gene encoding the amino acid sequence of SEQ ID NO: 18. For instance, purR gene can be obtained by PCR using primers of SEQ ID NOs: 19 and 20 from the chromosomal DNA of *Escherichia coli.*
Also, purR gene of other microorganisms can be obtained based on a known sequence or sequence homology with the above-mentioned purR gene of *E*. *coli* and used for modification of microorganisms.
purR gene may be one comprising an amino acid sequence having an identity of not less than 70%, preferably not less than 80%, more preferably not less than 90%, particularly preferably not less than 95% to the amino acid sequence of SEQ ID NO: 18.

The disruption of the desired gene can be confirmed by analyzing the gene on the chromosome by Southern blotting or a PCR method.

Further, a mutant strain which does not produce a functional PurR protein can also be obtained by irradiating microorganism with UV or treating it with a mutagen to be commonly used in mutagenesis such as N-methyl-N'-nitrosoguanidine (NTG) or nitrous acid. Also, by introducing one or more nucleotide substitution, deletion, insertion, addition, or inversion in a PurR binding sequence located upstream of guaBA operon promoter (M. I. Hutchings, W. T. Drabble, FEMS Microbiology Letters 187 (2000) 115-122), the PurR protein can be modified so as not to bind as usual.

It is preferable that the microorganism to be used in the method of the present invention be modified so that ushA gene and/or aphA gene do(does) not function normally. A mutant strain or gene recombinant strain of these genes can be obtained by modifying these genes such that activity of 5'-nucleotidase encoded by those genes decreases or disappears, or transcription of these genes decreases or disappears. Such a mutant strain or gene recombinant strain can be obtained in the same manner as the mutant strain or gene recombinant strain described above in which purR gene does not function normally.
The sequence ofushA gene is known and, based on the sequence, ushA gene can be readily obtained by a PCR method or hybridization method An example ofushA gene of *E*. *coli* includes a gene encoding the amino acid sequence of SEQ ID NO: 22.
Also, ushA gene of other microorganisms can be obtained based on a known sequence or sequence homology with the above-mentioned ushA gene of *E*. *coli* and used for modification of microorganisms.
And, ushA gene may be one comprising an amino acid sequence having an identity of not less than 70%, preferably not less than 80%, more preferably not less than 90%, particularly preferably not less than 95% to the amino acid sequence of SEQ ID NO: 22.
The sequence of aphA gene is known and, based on the sequence, aphA gene can be readily obtained by a PCR method or hybridization method. An example of aphA gene of *E*. *coli* includes a gene encoding the amino acid sequence of SEQ ID NO: 24.
Also, aphA gene of other microorganisms can be obtained based on a known sequence or sequence homology with the above-mentioned aphA gene of *E*. *coli* and used for modification of microorganisms.
And, aphA gene may be one comprising an amino acid sequence having an identity of not less than 70%, preferably not less than 80%, more preferably not less than 90%, particularly preferably not less than 95% to the amino acid sequence of SEQ ID NO: 24.

It is preferable that the microorganism to be used in the method of the present invention be modified so that add gene and/or purA gene do(does) not function normally. A mutant strain or gene recombinant strain of these genes can be obtained by modifying these genes such that activity of adenosine deaminase and/or succinyl AMP synthase encoded by those genes decreases or disappears, or transcription of these genes decreases or disappears. Such a mutant strain or gene recombinant strain can be obtained in the same manner as the mutant strain or gene recombinant strain described above in which purR gene does not function normally. The sequence of add gene is known and, based on the sequence, add gene can be readily obtained by a PCR method or hybridization method. An example of add gene of *E*. *coli* includes a gene encoding the amino acid sequence of SEQ ID NO: 38.
Also, add gene of other microorganisms can be obtained based on a known sequence or sequence homology with the above-mentioned add gene of *E*. *coli* and used for modification of microorganisms.
And, add gene may be one comprising an amino acid sequence having an identity of not less than 70%, preferably not less than 80%, more preferably not less than 90%, particularly preferably not less than 95% to the amino acid sequence of SEQ ID NO: 38.
The sequence itself of purA gene is known and, based on the sequence, purA gene can be readily obtained by a PCR method or hybridization method. An example of purA gene of *E*. *coli* includes a gene encoding the amino acid sequence of SEQ ID NO: 34. Also, purA gene of other microorganisms can be obtained based on a known sequence or sequence homology with the above-mentioned purA gene of *E*. *coli* and used for modification of microorganisms.
And, purA gene may be one comprising an amino acid sequence having an identity of not less than 70%, preferably not less than 80%, more preferably not less than 90%, particularly preferably not less than 95% to the amino acid sequence of SEQ ID NO: 34.

It is preferable that the microorganism to be used in the method of the present invention be modified so that nagD gene does not function normally. A mutant strain or gene recombinant strain of nagD gene can be obtained by modifying the genes such that activity of a protein encoded by the gene decreases or disappears, or transcription of the gene decreases or disappears. Such a mutant strain or gene recombinant strain can be obtained in the same manner as the mutant strain or gene recombinant strain described above in which purR gene does not function normally.
The sequence of nagD gene is known and, based on the sequence, nagD gene can be readily obtained by a PCR method or hybridization method. An example of nagD gene of *E*. *coli* includes a gene encoding the amino acid sequence of SEQ ID NO: 42.
It is known that nagD gene of *E*. *coli* exists in nagBACD operon which is involved in metabolizing N-acetylglucosamine and expression of this nagBACD operon gene is induced by adding N-acetylglucosamine, which is one of the sugars constituting major ingredients of bacterial cell walls, in a medium (Plumbridge JA., Mol. Micobiol. (1989) 3. 505-515). From conserved structural characteristics, NagD protein encoded by nagD of *E*. *coli* is known to belong to the family of haloacid dehalogenases (HAD). It has been reported that, according to *in vitro* experiments, the protein has a nucleotidase activity for GMP and 5'-uridylic acid (uridine 5'-monophosphate, hereinafter also referred to as "UMP") (Tremblay LW., Biochemistry, (2006) 45.1183-1193). There are 23 types of HAD family proteins on *E*. *coli* genome and a range of their substrate specificity is extremely broad (Kuznetsova et al., J. Biol. Chem., (2006) 281., 36149-36161). Therefore, physiological roles of nagD gene in cells are not known.
In the present invention, it has been discovered that further modification so that nagD gene does not function normally in a microorganism which has been modified so that expression of guaB gene and guaA gene increases, as well as ushA gene and aphA gene does not function normally resulted in efficient production of 5'-guanylic acid from inosinic acid.
Hence, the present invention provides, as a novel microorganism having an ability to convert inosinic acid to 5'-guanylic acid, a microorganism which has been modified so that inosinic acid dehydrogenase activity and 5'-guanylic acid synthetase activity are enhanced by increasing expression of guaB gene and guaA gene, as well as ushA gene, aphA gene and nagD gene do not function normally. It is preferable that, in this microorganism, one or more genes selected from purR gene, add gene and purA gene be further modified so as not to function normally.
And nagD gene is not limited to the gene of *E*. *coli*, nagD gene of other microorganisms can be obtained based on a known sequence or sequence homology with the above-mentioned nagD gene of *E*. *coli* and used for the modification. Thus, nagD gene may be one comprising an amino acid sequence having an identity of not less than 70%, preferably not less than 80%, more preferably not less than 90%, particularly preferably not less than 95% to the amino acid sequence of SEQ ID NO: 42.

### <II> Method for Producing GMP

GMP can be obtained by reacting IMP with the above-mentioned microorganism which has been modified so that IMP dehydrogenase activity and GMP synthetase activity are enhanced to convert IMP to GMP.

The reaction of IMP dehydrogenase or GMP synthetase requires NAD⁺ or ATP, respectively. NADH generated by the reaction of IMP dehydrogenase is again converted to NAD⁺ via an oxidative phosphorylated pathway. At that time, ATP is generated. It is therefore presumed that, by simultaneously enhancing activities of IMP dehydrogenase and GMP synthetase, a reaction from IMP to GMP proceeds efficiently.
In the present invention, the term, "reacting" includes to convert IMP to GMP by allowing the above-mentioned microorganism to exist in a medium to which IMP has been added and by using the microorganism as the so-called microorganism enzyme, as well as to generate GMP by adding IMP to a medium where the above-mentioned microorganism is culturing.
Preferably, by adding cells of the above-mentioned microorganism after the culturing to a reaction solution containing IMP or by adding IMP to a solution containing the above-mentioned microorganism, IMP can be efficiently converted to GMP, thereby GMP is produced and accumulated.

As a carbon source for culturing the microorganism, any of carbohydrates such as glucose, fructose, sucrose, molasses, blackstrap molasses or starch hydrolysate; alcohols such as ethanol, glycerin or sorbitol; organic acids such as pyruvic acid, lactic acid or acetic acid; amino acids such as glycine, alanine, glutamic acid or aspartic acid can be used, as long as it can be assimilated by the above-mentioned microorganism. As a nitrogen source, various inorganic and organic ammonium salts such as ammonia, ammonia chloride, ammonium sulfate, ammonium nitrate, ammonium carbonate, ammonium acetate or ammonium phosphate; urea, various amino acids, peptone, NZ amine, meat extract, yeast exact, corn steep liquor, casein hydrolysate, fish meal, digested material thereof or the like can be used. As an inorganic substance, potassium dihydrogen phosphate, potassium monophosphate, magnesium sulfate, magnesium phosphate, sodium chloride, ferrous sulfate, manganese sulfate, zinc sulfate, calcium carbonate or the like can be used. In cases where the microorganism requires specific trophic substances such as amino acids, nucleic acids or vitamins for its growth, an appropriate amount of those substances is added to the medium. The culture may be carried out in an appropriate range of pH 5.0 to 8.5 and 15°C to 45°C under aerobic condition for about 5 hours to 72 hours. In addition, for pH adjustment, an inorganic or organic, acidic or alkaline substance, as well as ammonia gas or the like can be used.

The conversion from IMP to GMP can be carried out by directly injecting the culture solution of the microorganism, by injecting only microbial cells after centrifugation of the culture solution or by injecting suspension in which the bacterial cells are suspended in an appropriate solution into the reaction solution containing IMP. In the method for producing GMP of the present invention, a processed product of the above-mentioned microbial cells can also be used. Examples of the processed product of microbial cells include immobilized microbial cells in which the microbial cells are immobilized using acrylamide, carrageenan or the like.

As a carbon source of the reaction solution, any of carbohydrates such as glucose, fructose, sucrose, molasses, blackstrap molasses or starch hydrolysate; alcohols such as ethanol, glycerin or sorbitol; organic acids such as pyruvic acid, lactic acid or acetic acid; amino acids such as glycine, alanine, glutamic acid or aspartic acid can be used, as long as it can be assimilated by the above-mentioned microorganisms. As a nitrogen source, various inorganic and organic ammonium salts such as ammonia, ammonia chloride, ammonium sulfate, ammonium nitrate, ammonium carbonate, ammonium acetate or ammonium phosphate; urea, various amino acids, peptone, NZ amine, meat extract, yeast extract, corn steep liquor, casein hydrolysate, fish meal, digested material thereof or the like can be used. As an inorganic substance, potassium dihydrogen phosphate, potassium monophosphate, magnesium sulfate, magnesium phosphate, sodium chloride, ferrous sulfate, manganese sulfate, zinc sulfate, calcium carbonate or the like can be used. In cases where the microorganism to be used requires specific trophic substances such as amino acids, nucleic acids or vitamins for its growth, an appropriate amount of these substances is added to the medium.

Further, it is preferable that permeability of nucleotide to cells be activated by adding an organic solvent to the reaction solution.
As the organic solvent which is an agent to activate membrane permeability of the nucleotide, xylene, toluene, benzene, fatty acid alcohol, ethyl acetate or the like can be used It is preferable that the concentration thereof be usually used at 0.1 to 30 ml/L. The reaction may be carried out in an appropriate range of pH 5.0 to 8.5 and 15°C to 45°C under aerobic or anaerobic condition for about 1 hour to 7 days. In addition, for pH adjustment, an inorganic or organic, acidic or alkaline substance, as well as ammonia gas or the like can be used. As the raw material, IMP, one which may be chemically synthesized, commercially available or produced by a fermentation method (WO96/30501, Japanese Patent Application Laid-Open Publication No. 2002-355087, Japanese Patent Application Laid-Open Publication No. 2003-325182 or the like) or the like can be used.

Collection ofGMP from the reaction solution can be usually carried out by combining an ion exchange resin method, a precipitation method and other known methods.

### [EXAMPLES]

The present invention will now be described more concretely by way of examples.

### Example 1

### <1-1> Construction of a strain where 5'-nucleotidase encoding ushA and aphA genes are deleted from JM109 strain

Construction of a strain which does not produce 5'-nucleotidase using as a parent strain *Escherichia coli* JM109 strain, which is commonly used as a host for DNA cloning, was first carried out. 5'-nucleotidase is encoded by ushA gene (Genbank Accession X03895 SEQ ID NO: 21) and aphA gene (Genbank Accession X86971 SEQ ID NO: 23).

Deletion of ushA and aphA genes encoding 5'-nucleotidase was carried out by a method called "Red-driven integration" which was originally developed by Datsenko and Wanner (Proc. Natl. Acad. Sci. USA, 2000, vol. 97, No. 12, p6640-6645) and an excision system derived from λ, phage (J. Bacteriol. 2002 Sep; 184(18): 5200-3. Interactions between integrase and excisionase in the phage lambda excisive nucleoprotein complex Cho EH, Gumport RI, Gardner JF.). According to the "Red-driven integration" method, a gene disrupted strain can be constructed in one step by using a PCR product which was obtained by using, as primers, synthetic oligonucleotides designed to have part of the desired gene in the 5'end and part of an antibiotic resistance gene in the 3' end. Further, by combining with the excision system derived from), phage, the antibiotic resistance gene incorporated in the gene distruptant strain can be removed.

### (1) Disruption of ushA gene

As a template of PCR, a plasmid pMW118-attL-Cm-attR was used. pMW118-attL-Cm-attR (WO200607803 is a plasmid in which attachment sites for λ phage, attL and attR genes as well as cat gene, which is an antibiotic resistance gene, are inserted in pMW118 (manufactured by Takara Bio Inc.) in the order of attL-cat-attR.

PCR was carried out using, as primers, synthetic oligonucleotides (SEQ ID NOs: 25 and 26) having a sequence corresponding to both ends of these attL and attR in the 3' end of the primer and a sequence corresponding to part of the desired gene, ushA, in the 5' wend of the primer.

The amplified PCR product was purified on agarose gel and introduced into *Escherichia coli* JM109 strain containing a plasmid pKD46 having a temperature-sensitive replication ability by electroporation The plasmid pKD46 (Proc. Natl. Acad. Sci. USA, 2000, vol. 97, No. 12, p6640-6645) contains a DNA fragment with a total of 2154 nucleotides of the λ phage (GenBank/EMBL Accession number J02459, the 31088th nucleotide to the 33241st nucleotide) containing a gene encoding Red reconbinase (γ, β and exo genes) of a λ Red homologous recombination system controlled by a arabinose-inducible P_{araB} promoter. The plasmid pKD46 is required to incorporate the PCR product into the chromosome of JM109 strain.

Competent cells for electroporation were prepared as follows. That is, *Escherichia coli* JM109 strain cultured in LB medium containing 100 mg/L of ampicillin at 30°C overnight was 100-fold diluted with 5 mL of SOB medium containing ampicillin (20 mg/L) and L-arabinose (1 mum) (Molecular Cloning: Laboratory Manual, Second Edition, Sambrook, J. et al., Cold Spring Harbor Laboratory Press (1989)). The obtained diluted cells were, while aerated, grown at 30°C until OD600 reached about 0.6. Thereafter, cells were concentrated 100-fold and washed three times with 10% glycerol so as to be used for electroporation. The electroporation was carried out using 70 µL of the obtained competent cells and about 100 ng of the PCR product. Cells after the electroporation were added with 1 mL of SOC medium (Molecular Cloning: Laboratory Manual, Second Edition, Sambmok, J. et al., Cold Spring Harbor Laboratory Press (1989)) and cultured at 37°C for 2.5 hours, followed by plate culture on L-agar medium containing Cm (Chloramphenicol) (25 mg/L) at 37°C to select Cm-resistant recombinants. Subsequently, in order to remove the pKD46 plasmid, they were subcultured twice on L-agar medium containing Cm at 42°C and ampicillin resistance of the obtained colonies was examined to obtain an ampicillin-sensitive strain in which pKD46 was cured.

The deletion of ushA gene in the mutant which was able to be distinguished by a chloramphenicol resistant gene was confirmed by PCR The obtained ushA-deleted strain was named JM1090ushA::att-cat strain.

Next, in order to remove att-cat gene which was introduced in ushA gene, the above-described pMW-intxis-ts was used as a helper plasmid. pMW-intxis-ts is a plasmid carrying a gene encoding integrase (Int) of the λ phage and a gene encoding excisionase (Xis), as well as having a temperature-sensitive replication ability. By the introduction of pMW-intxis-ts, attL or attR on the chromosome is recognized, recombination take place, and the gene between attL and attR is cut out, resulting in a structure in which only attL or attR sequence remains on the chromosome.

Competent cells of JM109AushA::att-cat strain obtained above were prepared in accordance with a conventional method, transformed with the helper plasmid pMW-intxis-ts, and subjected to plate culture on Lager medium containing 50 mg/L of ampicillin at 30°C to select an ampicillin resistant strain. Next, in order to remove pMW-intxis-ts plasmid, subculture was carried out twice on L-agar medium at 42°C, ampicillin and chloramphenicol resistance of the obtained colony was examined to obtain a chloramphenicol- and ampicillin-sensitive strain where ushA is disrupted and att-cat and pMW-intxis-ts were cured. This strain was named JM109ΔushA.

### (2) Deletion of aphA gene of JM109ΔushA strain

Deletion of aphA gene in JM109ΔushA strain was carried out according to the above method and using primers of SEQ ID NOs: 27 and 28 as primers for aphA disruption. Thereby, JM109ΔushAΔaphA which was ushA and aphA disrupted strain was obtained.

### <1-2> Construction of purR gene (a gene encoding repressor of purine operon and guaBA operon) disrupted strain from JM109ΔushAΔaphA strain

Subsequently, using JM109ΔushAΔaphA strain as a parent strain, construction of a strain which does not produce a repressor of guaBA operon, PurR, was carried out. PurR was encoded by purR gene (Genbank Accession J04212, SEQ ID NO: 17). The construction was carried out according to the above-described method of disruption of ushA and aphA genes and using the primers of SEQ ID NOs: 29 and 30 for purR disruption. Thereby, JM109ΔushAΔaphAΔpurR was obtained.

### <1-3> Construction of a plasmid pUC 18-guaBA for expression of IMP dehydrogenase and GMP synthetase and introduction of the plasmid into JM109ΔushAΔaphAΔpurR strain

The plasmid pUC18-guaBA for expression of IMP dehydrogenase and GMP synthetase was constructed as follows. It is known that guaB and guaA genes encoding the two enzymes constitute an operon (guaBA) in *Escherichia coli.* Thus, PCR was carried out using primers of SEQ ID NO: 31 and SEQ ID NO: 32 to amplify guaBA operon of *Escherichia coli.* After the amplified fragment was purified, restriction sites formed at both ends were cleaved with SacI and KpnI. The cleaved fragment was ligated with pUC18 which was also cleaved with SacI and KpnI to obtain a plasmid pUC 18-guaBA in which guaBA gene was inserted. This plasmid was introduced into the above-mentioned JM109ΔushAΔaphAΔpurR strain to obtain JM109ΔushAΔaphAΔpurR/pUC18-guaBA strain.

### <1-4> Conversion from IMP to GMP using JM109ΔushAΔaphAΔpurR/pUC18-guaBA strain

For the above-mentioned bacterial cells, evaluation of a conversion reaction from IMP to GMP was carried out. Hereinafter, a method for preparing bacterial cells, a method for reaction, composition of a reaction solution and a method of analysis for the evaluation of a conversion reaction from IMP to GMP are described.

### [Method for Preparing Bacterial Cells]

JM1090ΔushAΔaphAΔpurR/pUC18-guaBA strain was evenly spread on an LB medium plate and cultured at 37°C overnight Next day, bacterial cells corresponding to 1/32 of the plate were seeded in a 500-ml Sakaguchi flask filled with 20 ml of LB medium and cultured at 37°C overnight. Bacterial cells obtained by centrifuging the culture solution ofLB (600 ml) were used for a 60 ml of a reaction solution.

### [Method for Reaction]

The above-described bacterial cells from 600 ml of LB after the culturing were scraped with a medicine spoon and thereafter seeded in 60 ml of the reaction solution described later to initiate a reaction. The reaction was carried out at 42°C while pH was maintained at 7.2 by adding sodium hydroxide.

### [Composition of Reaction Solution]

60mM IMP
50 g/L Glucose
9.2 g/L Sodium hexametaphosphate
5gJL, MgSO₄·7H₂O
6.6 g/L (NH₄)₂SO₄
10 g/L KH₂PO₄
5 ml/L Xylene

### [Method of analysis]

The reaction solution (500 µl) was sampled with time and centrifuged at 15,000 rpm for 5 minutes. The supernatant was diluted with NaOH to terminate the reaction. The resulting solution was filtered and 300 µl of the filtrate was subjected to HPLC analysis. Conditions of the analysis are as follows:
Column: Two of Asahipak GS-220 (diameter 7.6 mm, 50 cm) were connected.
Eluent 0.2 MNaH₂PO₄ (pH 3.98)
Temperature: 55°C
Flow rate: 1 ml/min

### Detection: Ultraviolet (254 nm) absorption

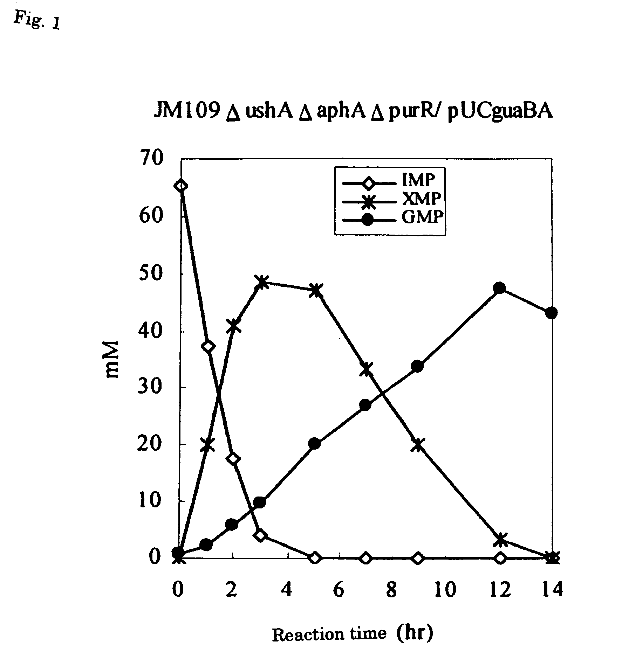

The results are shown in Figure 1. It was shown that JM109ΔushAΔaphAΔpurR/pUC18-guaBA accumulated about 16.2 g/L ofGMP at maximum in the reaction solution by 12 hours of the reaction.

### Example 2

### <2-1> Construction of nagD gene disrupted strain from JM109ΔushAΔaphAΔpurR strain

Using JM109ΔushAΔaphAΔpurR strain which was obtained in Example 1 < 1-2 > as a parent strain, construction of a strain which does not produce NagD was carried out. NagD was encoded by nagD gene (Genbank Accession X14135, SEQ ID NO: 41). The nagD gene disruption was carried out according to the above-described method of disruption of ushA, aphA and purR genes and using primers of SEQ ID NOs: 43 and 44 for nagD disruption. Thereby, JM109ΔushAΔaphAΔpurRΔnagD was obtained.

### <2-2> Introduction of plasmid pUC18-guaBA for expression of IMP dehydrogenase and GMP synthetase into JM109ΔushAΔaphAΔpurR strain and JM109ΔushAΔaphAΔpurRΔnagD strain

The plasmid pUC18-guaBA was introduced into the above-mentioned JM109ΔushAΔaphAΔpurR strain and JM109ΔushAΔaphAΔpurRΔnagD strain to obtain JM109ΔushAΔaphAΔpurR/pUC18-guaBA strain and JM109ΔushAΔaphAΔpurRΔnagD/pUC18-guaBA strain, respectively.

### <2-3> Conversion from IMP to GMP using JM109ΔushAΔaphAΔpurR/pUC18-guaBA strain and JM109ΔushAΔaphAΔpurRΔnagD/pUC18-guaBA strain

For the above-mentioned bacterial cells, evaluation of a conversion reaction from IMP to GMP was carried out. Hereinafter, a method for preparing bacterial cells, a method for reaction, composition of a reaction solution and a method of analysis for the conversion reaction from IMP to GMP are described.

### [Method for Preparing Bacterial Cells]

JM109ΔushAΔaphAApurR/pUC18-guaBA strain or JM109ΔushAΔaphAΔpurRΔnagD/pUC18-guaBA strain was evenly spread on an LB medium plate and cultured at 37°C overnight. Next day, bacterial cells corresponding to 1/32 of the plate were seeded in a 500-ml Sakaguchi flask filled with 20 ml of LB medium and cultured at 37°C overnight.

### [Method for Reaction]

The above-described culture solution was centrifuged and bacterial cells corresponding to a dry weight of 0.8 g were seeded in 60 ml of a reaction solution to initiate a reaction. The reaction was carried out at 42°C while pH was maintained at 7.2 by adding sodium hydroxide.

### [Composition of Reaction Solution for Conversion Reaction from IMP to GMP]

50 mM IMP
50 g/L Glucose
9.2 g/L Sodium hexametaphosphate
5 g/L MgSO₄·7H₂O
6.6 g/L (NH₄)₂SO₄
10 g/L KH₂PO₄
3 ml/L Xylene

### [Method of Analysis]

The reaction solution (500 µl) was sampled with time and diluted with NaOH to terminate the reaction. The resulting solution was filtered and 300 µl of the filtrate was subjected to HPLC analysis. Conditions of the analysis are as follows:
Column: Two of Asahipak GS-220 (diameter 7.6 mm, 50 cm) were connected.
Eluent: 0.2 M NaH₂PO₄ (pH 3.98)
Temperature: 55°C
Flow rate: 1 ml/min
Detection: Ultraviolet (254 nm) absorption

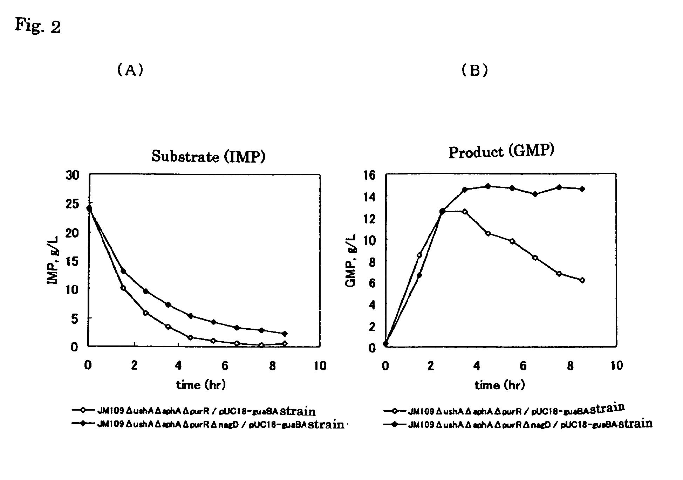

The results are shown in Figure 2. It was shown that JM109ΔushAΔaphAΔpurR/pUC18-guaBA strain accumulated about 12.5 g/L of GMP at ,maximum in the reaction solution whereas JM109ΔushAΔaphAΔpurRΔnagD/pUC18-guaBA strain accumulated about 14.81 g/L of GMP at maximum.

### Example 3

### Conversion from IMP to GMP and from XMP to GMP using JM109ΔushAΔaphAΔpurRΔnagD/pUC18-guaBA strain described in Example 2 and comparison thereof

For JM109ΔushAΔaphAΔpurRΔnagD/pUC18-guaBA strain described in Example 2, a conversion reaction from IMP to GMP and conversion reaction from XMP to GMP were evaluated. A method for preparing bacterial cells, a method for reaction, composition of a solution for a conversion reaction from IMP to GMP and a method of analysis for the evaluation of the conversion reaction were employed in accordance with Example 2. In the conversion reaction from XMP to GMP, the same concentration of XMP, instead of IMP in the composition of the solution for the conversion reaction from IMP to GMP, was used.

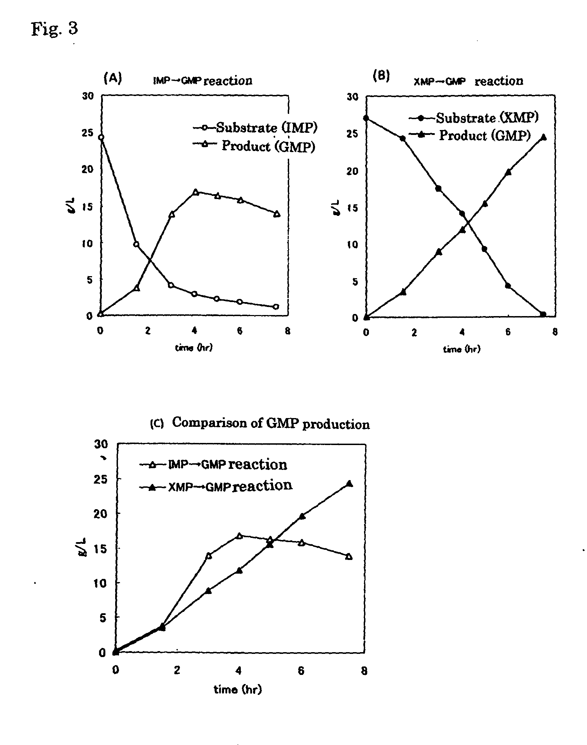

The results are shown in Figure 3. It was shown that up to about 16.9 g/L of GMP was accumulated in the reaction solution by 4 hours of the reaction in the conversion from IMP to GMP whereas about 24.4 g/L of GMP at maximum was accumulated in the reaction solution by 7.5 hours of the reaction in the conversion from XMP to GMP, and thus the production rate of GMP in the reaction with IMP as a substrate is higher than that in the reaction with XMP as the substrate.

### Example 4

### <4-1> Construction of 5'-nucleotidase encoding ushA and aphA disrupted strain from MG1655 strain

Using *Escherichia coli* wild type MG1655 strain as a parent strain, construction of a strain which does not produce 5'-nucleotidase, UshA and AphA was carried out. The deletion of ushA and aphA genes encoding 5'-nucleotidase was carried out by a method called "Red-driven integration" and an excision system derived from λ phage in accordance with Example 1. The produced strain was named MG1655ΔushAΔaphA.

### <A-2> Construction of succinyl AMP synthase encoding purA gene disrupted strain from MG1655ΔushAΔaphA strain

Subsequently, using MG1655ΔushAΔaphA strain as a parent strain, construction of a strain which does not produce succinyl AMP synthase was carried out. Succinyl AMP synthase of *E. coli* was encoded by purA gene (Genbank Accession J04199, SEQ ID NO: 33). The construction was carried out according to the method of disruption of ushA, aphA and purR genes shown in Example 1 and using primers of SEQ ID NOs: 35 and 36 for purA disruption. Thereby, MG1655ΔushAΔaphAΔpurA was obtained.

### < 4-3 > Construction of purR gene (a gene encoding represser of purine operon and guaBA operon) disrupted strain from MG1655ΔushAΔaphAΔpurA strain

Subsequently, using MG1655ΔushAΔaphAΔpurA strain as a parent strain, construction of a strain which does not produce a repressor of guaBA operon, PurR was carried out. The disruption of purR gene was carried out in accordance with Example 1 and using primers of SEQ ID NOs: 29 and 30 for purR disruption. Thereby, MG1655ΔushAΔaphAΔpurAΔpurR was obtained.

### <4-4> Construction of adenosine deaminase encoding add gene disrupted strain from MG1655ΔushAΔaphAΔpurR strain

Subsequently, using MG1655ΔushAΔaphAΔpurAΔpurR strain as a parent strain, construction of a strain which does not produce adenosine deaminase was carried out. Adenosine deaminase of *E. coli* is encoded by add gene (Genbank Accession No. M59033, SEQ ID NO: 37). The construction was carried out according to the method of disruption of ushA, aphA and purR genes shown in Example 1 and using primers of SEQ ID NOs: 39 and 40 for add disruption. Thereby, MG1655ΔushAΔaphAΔpurAΔpurRΔadd strain was obtained.

### <4-5> Introduction of a plasmid pUC18-guaBA for expression of IMP dehydrogenase and GMP synthetase into MG1655ΔushAΔaphAΔpurAΔpurRΔadd strain

pUC18-guaBA shown in Example 1 was introduced into the above-mentioned
MG1655ΔushAΔaphAΔpurAΔpurRΔadd strain to obtain
MG1655ΔushAΔaphAΔpurAΔpurRΔadd/pUC18-guaBA strain.

### <4-b> Conversion from IMP or XMP to GMP using MG1655ΔushAΔaphAΔpurAΔpurRΔadd/pUC18-guaBA strain

As for the above-mentioned bacterial cells, evaluation of a conversion reaction from IMP or XMP to GMP was carried out Hereinafter, a method for preparing bacterial cells, a method for reaction, composition of a reaction solution and a method of analysis for the evaluation of the conversion reaction are described.

### [Method for Preparing Bacterial Cells]

MG1655ΔushAΔaphAΔpurAΔpurRΔadd/pUC18-guaBA strain was evenly spread on an LB medium plate and cultured at 37°C overnight Next day, bacterial cells corresponding to 1/32 of the plate were seeded in a 500-ml Sakaguchi flask filled with 20 ml of LB medium and cultured at 37°C overnight. Bacterial cells obtained by centrifuging the culture solution of LB (600 ml) were used for a 60 ml of a reaction solution.

### [Method for Reaction]

The above-described bacterial cells from 600 ml of LB after the culturing were scraped with a medicine spoon and thereafter seeded in 60 ml of the reaction solution described later to initiate a reaction, The reaction was carried out at 42°C while pH was maintained at 7.2 by adding sodium hydroxide.

### [Composition of Reaction Solution]

50 mM IMP or 50 mM XMP
50 g/L Glucose
9.2 g/L Sodium hexametaphosphate
5 g/L MgSO₄·7H₂O
6.6 g/L (NH₄)₂SO₄
10 g/L KH₂PO₄
5 ml/L Xylene

### [Method of Analysis]

The reaction solution (500 µl) was sampled with time and diluted with NaOH to terminate the reaction. The resulting solution was filtered and 300 µl of the filtrate was subjected to HPLC analysis. Conditions of the analysis are as follows:
Column: Two of Asahipak GS-220 (diameter 7.6 mm, 50 cm) were connected.
Eluent: 0.2 M NaH₂PO₄ (pH 3.98)
Temperature: 55°C
Flow rate: 1 ml/min
Detection: Ultraviolet (254 nm) absorption

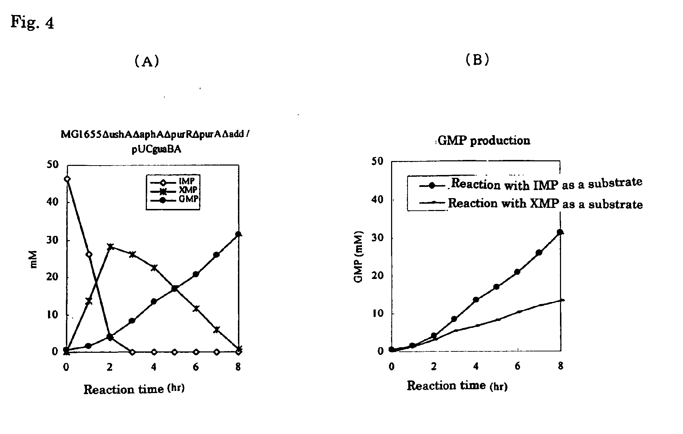

The results are shown in Figure 4. It was shown that the production rate of GMP in the reaction with IMP as a substrate is higher than that in the reaction with XMP as the substrate.

### Industrial Applicability

According to the present invention, GMP which is useful as seasonings, pharmaceuticals and raw materials thereof can be efficiently produced.

### [Explanation of the Sequence Listing]

SEQ ID NO: 1: Nucleotide sequence of *E*. *coli* GMP synthetase (GMPS) gene (guaA)
SEQ ID NO: 2: Amino acid sequence of *E*. *coli* GMPS
SEQ ID NO: 3: Nucleotide sequence of *B. subtilis* GMPS gene (guaA)
SEQ ID NO: 4: Amino acid sequence of *B*. *subtilis* GMPS
SEQ ID NO: 5: Nucleotide sequence of *C*. *glutamicum* GMPS gene (guaA)
SEQ ID NO: 6: Amino acid sequence of *C*. *glutamicum* GMPS
SEQ ID NO: 7: Nucleotide sequence of C. *ammoniagenes* GMPS gene (guaA)
SEQ ID NO: 8: Amino acid sequence of *C*. *ammnoniagenes* GMPS
SEQ ID NO: 9: Nucleotide sequence of *E*. *coli* IMP dehydrogenase (IMPDH) gene (guaB)
SEQ ID NO: 10: Amino acid sequence of *E*. *coli* IMPDH
SEQ ID NO: 11: Nucleotide sequence of *B. subtilis* IMPDH gene (guaB)
SEQ ID NO: 12: Amino acid sequence of *B*. *subtilis* IMPDH
SEQ ID NO: 13: Nucleotide sequence of *C*. *glutamicum* IMPDH gene (guaB)
SEQ ID NO: 14: Amino acid sequence of *C*. *glutamicum* IMPDH
SEQ ID NO: 15: Nucleotide sequence of *C*. *ammoniagenes* IMPDH gene (guaB)
SEQ ID NO: 16: Amino acid sequence of *C*. *ammoniagenes* IMPDH
SEQ ID NO: 17: Nucleotide sequence of *E. coli* purR
SEQ ID NO: 18: Amino acid sequence of *E*. *coli* PurR
SEQ ID NO: 19: Primer sequence for cloning of *E*. *coli* purR
SEQ ID NO: 20: Primer sequence for cloning of *E*. *coli* purR
SEQ ID NO: 21: Nucleotide sequence of *E*. *coli* ushA
SEQ ID NO: 22: Amino acid sequence of *E*. *coli* UshA
SEQ ID NO: 23: Nucleotide sequence of *E*. *coli* aphA
SEQ ID NO: 24: Amino acid sequence of *E*. *coli* AphA
SEQ ID NO: 25: Primer sequence for disruption of *E*. *coli* ushA gene
SEQ ID NO: 26: Primer sequence for disruption of *E*. *coli* ushA gene
SEQ ID NO: 27: Primer sequence for disruption of *E*. *coli* aphA gene
SEQ ID NO: 28: Primer sequence for disruption of *E*. *coli* aphA gene
SEQ ID NO: 29: Primer sequence for disruption of *E*. *coli* purR gene
SEQ NO: 30: Primer sequence for disruption of *E*. *coli* purR gene
SEQ ID NO: 31: Primer sequence for cloning of *E*. *coli* guaBA
SEQ ID NO: 32: Primer sequence for cloning of *E*. *coli* guaBA
SEQ ID NO: 33: Nucleotide sequence of *E*. *coli* purA
SEQ ID NO: 34: Amino acid sequence of *E*. *coli* PurA
SEQ NO: 35: Primer sequence for disruption of *E*. *coli* purA gene
SEQ ID NO: 36: Primer sequence for disruption of *E*. *coli* purA gene
SEQ ID NO: 37: Nucleotide sequence of *E*. *coli* add
SEQ ID NO: 38: Amino acid sequence of *E*. *coli* Add
SEQ ID NO: 39: Primer sequence for disruption of *E*. *coli* add gene
SEQ ID NO: 40: Primer sequence for disruption of *E*. *coli* add gene
SEQ ID NO: 41: Nucleotide sequence of *E*. *coli* nagD
SEQ ID NO: 42: Amino acid sequence of *E*. *coli* NagD
SEQ ID NO: 43: Primer sequence for disruption of *E*. *coli* nagD gene
SEQ ID NO: 44: Primer sequence for disruption of *E*. *coli* nagD gene

## Claims

1. A method for producing 5'-guanylic acid, comprising:
generating 5'-guanylic acid by reacting inosinic acid with a microorganism having an ability to convert inosinic acid into 5'-guanylic acid; and
collecting 5'-guanylic acid, wherein said microorganism has been modified so that inosinic acid dehydrogenase activity and 5'-guanylic acid synthetase activity are enhanced.

2. The method according to claim 1, wherein inosinic acid dehydrogenase activity and 5'-guanylic acid synthetase activity are enhanced by increasing expression of guaB gene and guaA gene.

3. The method according to claim 2, wherein
said guaA gene encodes a protein selected from the group consisting of
(A) A protein comprising the amino acid sequence of SEQ ID NO: 2, and
(B) A protein comprising the amino acid sequence of SEQ ID NO: 2 but which includes substitution, deletion, insertion, addition or inversion of one or several amino acids, and has 5'-guanylic acid synthetase activity.

4. The method according to claim 2 or 3, wherein said guaB gene encodes a protein selected from the group consisting of:
(C) A protein comprising the amino acid sequence of SEQ ID NO: 10, and
(D) A protein comprising the amino acid sequence of SEQ ID NO: 10 but which includes substitution, deletion, insertion, addition or inversion of one or several amino acids, and has inosinic acid dehydrogenase activity.

5. The method according to any one of claims 1 to 4, wherein said microorganism has been further modified so that one or more genes selected from ushA gene and aphA gene do not function normally.

6. The method according to any one of claims 1 to 5, wherein said microorganism has been further modified so that one or more genes selected from purR gene, add gene and purA gene do not function normally.

7. The method according to any one of claims 1 to 6, wherein said microorganism has been further modified so that nagD gene does not function normally.

8. The method according to any one of claims 1 to 7, wherein said microorganism is selected from the group consisting of bacteria belonging to the *Enterobacteriaceae* family, *Bacillus* bacteria and Coryneform bacteria.

9. The method according to claim 8, wherein said microorganism is an *Escherichia* bacterium.

10. The method according to claim 9, wherein said microorganism is *Escherichia coli.*

11. A microorganism having an ability to convert inosinic acid into 5'-guanylic acid, which has been modified so that inosinic acid dehydrogenase activity and 5'-guanylic acid synthetase activity are enhanced by increasing expression of guaB gene and guaA gene, and has been further modified so that ushA gene, aphA gene and nagD gene do not function normally.

12. The microorganism according to claim 11, which has been further modified so that one or more genes selected from purR gene, add gene and purA gene do not function normally.

13. The microorganism according to claim 11 or 12, which is selected from the group consisting of bacteria belonging to the *Enterobacteriaceae* family, *Bacillus* bacteria and Coryneform bacteria

14. The microorganism according to claim 13 which is an *Escherichia* bacterium.

15. The microorganism according to claim 14 which is an *Escherichia coli.*


SEQUENCE LISTING

<110>  Ajinomoto Co., Inc.

<120>  PROCESS FOR PRODUCTION OF 5'-GUANYLIC ACID

<130>  EPA-64971

<150>  JP2008-043136
<151>  2008-02-25

<160>  44

<170>  PatentIn version 3.3

<210>  1
<211>  1578
<212>  DNA
<213>  Escherichia coli


<220>
<221>  CDS
<222>  (1)..(1575)

<400>  1
atg acg gaa aac att cat aag cat cgc atc ctc att ctg gac ttc ggt      48
Met Thr Glu Asn Ile His Lys His Arg Ile Leu Ile Leu Asp Phe Gly
1               5                   10                  15
tct cag tac act caa ctg gtt gcg cgc cgc gtg cgt gag ctg ggt gtt      96
Ser Gln Tyr Thr Gln Leu Val Ala Arg Arg Val Arg Glu Leu Gly Val
            20                  25                  30
tac tgc gaa ctg tgg gcg tgg gat gtg aca gaa gca caa att cgt gac      144
Tyr Cys Glu Leu Trp Ala Trp Asp Val Thr Glu Ala Gln Ile Arg Asp
        35                  40                  45
ttc aat cca agc ggc att att ctt tcc ggc ggc ccg gaa agt act act      192
Phe Asn Pro Ser Gly Ile Ile Leu Ser Gly Gly Pro Glu Ser Thr Thr
    50                  55                  60
gaa gaa aac agt ccg cgt gcg ccg cag tat gtc ttt gaa gca ggc gta      240
Glu Glu Asn Ser Pro Arg Ala Pro Gln Tyr Val Phe Glu Ala Gly Val
65                  70                  75                  80
ccg gta ttc ggc gtt tgc tat ggc atg cag acc atg gca atg cag ttg      288
Pro Val Phe Gly Val Cys Tyr Gly Met Gln Thr Met Ala Met Gln Leu
                85                  90                  95
ggc ggt cac gtt gaa gcc tct aac gaa cgt gaa ttt ggc tac gcg cag      336
Gly Gly His Val Glu Ala Ser Asn Glu Arg Glu Phe Gly Tyr Ala Gln
            100                 105                 110
gtt gaa gtc gta aac gac agc gca ctg gtt cgc ggt atc gaa gat gcg      384
Val Glu Val Val Asn Asp Ser Ala Leu Val Arg Gly Ile Glu Asp Ala
        115                 120                 125
ctg acc gca gac ggt aaa ccg ctg ctc gat gtc tgg atg agc cac ggc      432
Leu Thr Ala Asp Gly Lys Pro Leu Leu Asp Val Trp Met Ser His Gly
    130                 135                 140
gat aaa gtt acc gct att ccg tcc gac ttc atc acc gta gcc agc acc      480
Asp Lys Val Thr Ala Ile Pro Ser Asp Phe Ile Thr Val Ala Ser Thr
145                 150                 155                 160
gaa agc tgc ccg ttt gcc att atg gct aac gaa gaa aaa cgc ttc tat      528
Glu Ser Cys Pro Phe Ala Ile Met Ala Asn Glu Glu Lys Arg Phe Tyr
                165                 170                 175
ggc gta cag ttc cac ccg gaa gtg act cat acc cgc cag ggt atg cgc      576
Gly Val Gln Phe His Pro Glu Val Thr His Thr Arg Gln Gly Met Arg
            180                 185                 190
atg ctg gag cgt ttt gtg cgt gat atc tgc cag tgt gaa gcc ctg tgg      624
Met Leu Glu Arg Phe Val Arg Asp Ile Cys Gln Cys Glu Ala Leu Trp
        195                 200                 205
acg cca gcg aaa att atc gac gat gct gta gct cgc atc cgc gag cag      672
Thr Pro Ala Lys Ile Ile Asp Asp Ala Val Ala Arg Ile Arg Glu Gln
    210                 215                 220
gta ggc gac gat aaa gtc atc ctc ggc ctc tct ggt ggt gtg gat tcc      720

Val Gly Asp Asp Lys Val Ile Leu Gly Leu Ser Gly Gly Val Asp Ser
225                 230                 235                 240
tcc gta acc gca atg ctg ctg cac cgc gct atc ggt aaa aac ctg act      768
Ser Val Thr Ala Met Leu Leu His Arg Ala Ile Gly Lys Asn Leu Thr
                245                 250                 255
tgc gta ttc gtc gac aac ggc ctg ctg cgc ctc aac gaa gca gag cag      816
Cys Val Phe Val Asp Asn Gly Leu Leu Arg Leu Asn Glu Ala Glu Gln
            260                 265                 270
gtt ctg gat atg ttt ggc gat cac ttt ggt ctt aac att gtt cac gta      864
Val Leu Asp Met Phe Gly Asp His Phe Gly Leu Asn Ile Val His Val
        275                 280                 285
ccg gca gaa gat cgc ttc ctg tca gcg ctg gct ggc gaa aac gat ccg      912
Pro Ala Glu Asp Arg Phe Leu Ser Ala Leu Ala Gly Glu Asn Asp Pro
    290                 295                 300
gaa gca aaa cgt aaa atc atc ggt cgc gtt ttc gtt gaa gta ttc gat      960
Glu Ala Lys Arg Lys Ile Ile Gly Arg Val Phe Val Glu Val Phe Asp
305                 310                 315                 320
gaa gaa gcg ctg aaa ctg gaa gac gtg aag tgg ctg gcg cag ggc acc     1008
Glu Glu Ala Leu Lys Leu Glu Asp Val Lys Trp Leu Ala Gln Gly Thr
                325                 330                 335
atc tac cct gac gtt atc gaa tct gcg gcg tct gca acc ggt aaa gca     1056
Ile Tyr Pro Asp Val Ile Glu Ser Ala Ala Ser Ala Thr Gly Lys Ala
            340                 345                 350
cac gtc atc aaa tct cac cac aac gtg ggc ggc ctg ccg aaa gag atg     1104
His Val Ile Lys Ser His His Asn Val Gly Gly Leu Pro Lys Glu Met
        355                 360                 365
aag atg ggc ctg gtt gaa ccg ctg aaa gag ctg ttc aaa gac gaa gtg     1152
Lys Met Gly Leu Val Glu Pro Leu Lys Glu Leu Phe Lys Asp Glu Val
    370                 375                 380
cgt aag att ggt ctg gag ctg ggc ctg ccg tac gac atg ctg tac cgt     1200
Arg Lys Ile Gly Leu Glu Leu Gly Leu Pro Tyr Asp Met Leu Tyr Arg
385                 390                 395                 400
cac ccg ttc ccg gga cca ggc ctt ggc gtt cgt gtt ctg ggt gaa gtg     1248
His Pro Phe Pro Gly Pro Gly Leu Gly Val Arg Val Leu Gly Glu Val
                405                 410                 415
aag aaa gag tac tgt gac ctg ctg cgc cgt gct gac gcc atc ttc att     1296
Lys Lys Glu Tyr Cys Asp Leu Leu Arg Arg Ala Asp Ala Ile Phe Ile
            420                 425                 430
gaa gaa ctg cgt aaa gcg gac ctg tac gac aaa gtc agc cag gcg ttc     1344
Glu Glu Leu Arg Lys Ala Asp Leu Tyr Asp Lys Val Ser Gln Ala Phe
        435                 440                 445
act gtg ttc ctg ccg gta cgt tcc gtt ggc gta atg ggc gat ggt cgt     1392
Thr Val Phe Leu Pro Val Arg Ser Val Gly Val Met Gly Asp Gly Arg
    450                 455                 460
aag tat gac tgg gtt gtc tct ctg cgt gct gtc gaa acc atc gac ttt     1440
Lys Tyr Asp Trp Val Val Ser Leu Arg Ala Val Glu Thr Ile Asp Phe
465                 470                 475                 480
atg acc gca cac tgg gcg cat ctg ccg tac gat ttc ctc ggt cgc gtt     1488
Met Thr Ala His Trp Ala His Leu Pro Tyr Asp Phe Leu Gly Arg Val
                485                 490                 495
tcc aac cgc att atc aat gaa gtg aac ggt att tcc cgc gtg gtg tat     1536
Ser Asn Arg Ile Ile Asn Glu Val Asn Gly Ile Ser Arg Val Val Tyr
            500                 505                 510
gac atc agc ggc aag ccg cca gct acc att gag tgg gaa tga             1578
Asp Ile Ser Gly Lys Pro Pro Ala Thr Ile Glu Trp Glu
        515                 520                 525

<210>  2
<211>  525
<212>  PRT
<213>  Escherichia coli

<400>  2
Met Thr Glu Asn Ile His Lys His Arg Ile Leu Ile Leu Asp Phe Gly
1               5                   10                  15
Ser Gln Tyr Thr Gln Leu Val Ala Arg Arg Val Arg Glu Leu Gly Val
            20                  25                  30
Tyr Cys Glu Leu Trp Ala Trp Asp Val Thr Glu Ala Gln Ile Arg Asp

35                  40                  45
Phe Asn Pro Ser Gly Ile Ile Leu Ser Gly Gly Pro Glu Ser Thr Thr
    50                  55                  60
Glu Glu Asn Ser Pro Arg Ala Pro Gln Tyr Val Phe Glu Ala Gly Val
65                  70                  75                  80
Pro Val Phe Gly Val Cys Tyr Gly Met Gln Thr Met Ala Met Gln Leu
                85                  90                  95
Gly Gly His Val Glu Ala Ser Asn Glu Arg Glu Phe Gly Tyr Ala Gln
            100                 105                 110
Val Glu Val Val Asn Asp Ser Ala Leu Val Arg Gly Ile Glu Asp Ala
        115                 120                 125
Leu Thr Ala Asp Gly Lys Pro Leu Leu Asp Val Trp Met Ser His Gly
    130                 135                 140
Asp Lys Val Thr Ala Ile Pro Ser Asp Phe Ile Thr Val Ala Ser Thr
145                 150                 155                 160
Glu Ser Cys Pro Phe Ala Ile Met Ala Asn Glu Glu Lys Arg Phe Tyr
                165                 170                 175
Gly Val Gln Phe His Pro Glu Val Thr His Thr Arg Gln Gly Met Arg
            180                 185                 190
Met Leu Glu Arg Phe Val Arg Asp Ile Cys Gln Cys Glu Ala Leu Trp
        195                 200                 205
Thr Pro Ala Lys Ile Ile Asp Asp Ala Val Ala Arg Ile Arg Glu Gln
    210                 215                 220
Val Gly Asp Asp Lys Val Ile Leu Gly Leu Ser Gly Gly Val Asp Ser
225                 230                 235                 240
Ser Val Thr Ala Met Leu Leu His Arg Ala Ile Gly Lys Asn Leu Thr
                245                 250                 255
Cys Val Phe Val Asp Asn Gly Leu Leu Arg Leu Asn Glu Ala Glu Gln
            260                 265                 270
Val Leu Asp Met Phe Gly Asp His Phe Gly Leu Asn Ile Val His Val
        275                 280                 285
Pro Ala Glu Asp Arg Phe Leu Ser Ala Leu Ala Gly Glu Asn Asp Pro
    290                 295                 300
Glu Ala Lys Arg Lys Ile Ile Gly Arg Val Phe Val Glu Val Phe Asp
305                 310                 315                 320
Glu Glu Ala Leu Lys Leu Glu Asp Val Lys Trp Leu Ala Gln Gly Thr
                325                 330                 335
Ile Tyr Pro Asp Val Ile Glu Ser Ala Ala Ser Ala Thr Gly Lys Ala
            340                 345                 350
His Val Ile Lys Ser His His Asn Val Gly Gly Leu Pro Lys Glu Met
        355                 360                 365
Lys Met Gly Leu Val Glu Pro Leu Lys Glu Leu Phe Lys Asp Glu Val
    370                 375                 380
Arg Lys Ile Gly Leu Glu Leu Gly Leu Pro Tyr Asp Met Leu Tyr Arg
385                 390                 395                 400
His Pro Phe Pro Gly Pro Gly Leu Gly Val Arg Val Leu Gly Glu Val
                405                 410                 415
Lys Lys Glu Tyr Cys Asp Leu Leu Arg Arg Ala Asp Ala Ile Phe Ile
            420                 425                 430
Glu Glu Leu Arg Lys Ala Asp Leu Tyr Asp Lys Val Ser Gln Ala Phe
        435                 440                 445
Thr Val Phe Leu Pro Val Arg Ser Val Gly Val Met Gly Asp Gly Arg
    450                 455                 460
Lys Tyr Asp Trp Val Val Ser Leu Arg Ala Val Glu Thr Ile Asp Phe
465                 470                 475                 480
Met Thr Ala His Trp Ala His Leu Pro Tyr Asp Phe Leu Gly Arg Val
                485                 490                 495
Ser Asn Arg Ile Ile Asn Glu Val Asn Gly Ile Ser Arg Val Val Tyr
            500                 505                 510
Asp Ile Ser Gly Lys Pro Pro Ala Thr Ile Glu Trp Glu
        515                 520                 525

<210>  3
<211>  1542
<212>  DNA
<213>  Bacillus subtilis

<220>
<221> CDS
<222> (1)..(1539)

<400> 3
atg aca aag tta gtg aat gaa atg att ctt gtc ctt gat ttc ggc agt 48
Met Thr Lys Leu Val Asn Glu Met Ile Leu Val Leu Asp Phe Gly Ser
1               5                   10                  15
cag tat aac cag ctg att aca cgc cgt atc cgt gaa ttc ggt gtt tac 96
Gln Tyr Asn Gln Leu Ile Thr Arg Arg Ile Arg Glu Phe Gly Val Tyr
            20                  25                  30
agc gag ctg cat cca cat aca ttg acg gct gaa gaa att aaa aaa atg 144
Ser Glu Leu His Pro His Thr Leu Thr Ala Glu Glu Ile Lys Lys Met
        35                  40                  45
aat cca aaa gga att att tta tcc ggc ggt cca aac agt gtg tat gat 192
Asn Pro Lys Gly Ile Ile Leu Ser Gly Gly Pro Asn Ser Val Tyr Asp
    50                  55                  60
gaa aac tct ttc cgc tgt gac gag aaa atc ttc gag ctt gat att cct 240
Glu Asn Ser Phe Arg Cys Asp Glu Lys Ile Phe Glu Leu Asp Ile Pro
65                  70                  75                  80
gtt ttg gga att tgc tac ggc atg cag ctg atg act cat tac ctt ggc 288
Val Leu Gly Ile Cys Tyr Gly Met Gln Leu Met Thr His Tyr Leu Gly
                85                  90                  95
ggt aaa gtt gaa gcg gca agc cag cgt gaa tac gga aaa gca aac atc 336
Gly Lys Val Glu Ala Ala Ser Gln Arg Glu Tyr Gly Lys Ala Asn Ile
            100                 105                 110
cgc atc gaa ggc aca cct gat ttg ttc aga gat ctt ccg aat gaa caa 384
Arg Ile Glu Gly Thr Pro Asp Leu Phe Arg Asp Leu Pro Asn Glu Gln
        115                 120                 125
gtg gtt tgg atg agc cac ggc gat ttg gtt gta gaa gtt cct gaa ggc 432
Val Val Trp Met Ser His Gly Asp Leu Val Val Glu Val Pro Glu Gly
    130                 135                 140
ttc act gtt gac gcg aca agc cat cac tgc ccg aac tca gca atg agc 480
Phe Thr Val Asp Ala Thr Ser His His Cys Pro Asn Ser Ala Met Ser
145                 150                 155                 160
aaa gcg gac aaa aaa tgg tat ggc gtt cag ttc cac ccg gaa gtg cgc 528
Lys Ala Asp Lys Lys Trp Tyr Gly Val Gln Phe His Pro Glu Val Arg
                165                 170                 175
cac tct gaa tac ggc aat gat ctt ctg aaa aac ttt gta ttc ggt gtt 576
His Ser Glu Tyr Gly Asn Asp Leu Leu Lys Asn Phe Val Phe Gly Val
            180                 185                 190
tgc gaa tgc gaa ggc gaa tgg tca atg gag aac ttt atc gaa atc gaa 624
Cys Glu Cys Glu Gly Glu Trp Ser Met Glu Asn Phe Ile Glu Ile Glu
        195                 200                 205
atg caa aaa atc cgt gaa acg gtc gga gac aaa cag gtt ctt tgc gcg 672
Met Gln Lys Ile Arg Glu Thr Val Gly Asp Lys Gln Val Leu Cys Ala
    210                 215                 220
cta agc ggc ggc gtt gat tcc tct gtt gtt gct gtt ttg att cat aaa 720
Leu Ser Gly Gly Val Asp Ser Ser Val Val Ala Val Leu Ile His Lys
225                 230                 235                 240
gcg atc ggc gac cag ctg act tgt atc ttt gta gac cat ggt ctt ctc 768
Ala Ile Gly Asp Gln Leu Thr Cys Ile Phe Val Asp His Gly Leu Leu
                245                 250                 255
cgt aaa ggc gaa gct gag ggt gtt atg aaa aca ttc agc gaa ggc ttt 816
Arg Lys Gly Glu Ala Glu Gly Val Met Lys Thr Phe Ser Glu Gly Phe
            260                 265                 270
aac atg aat gtg att aaa gta gac gca aaa gat cga ttc tta aac aaa 864
Asn Met Asn Val Ile Lys Val Asp Ala Lys Asp Arg Phe Leu Asn Lys
        275                 280                 285
cta aaa ggc gtt tct gat cct gag caa aaa cgc aaa atc atc ggt aat 912
Leu Lys Gly Val Ser Asp Pro Glu Gln Lys Arg Lys Ile Ile Gly Asn
    290                 295                 300
gaa ttc att tac gtg ttt gat gat gaa gcg gac aag ctc aaa ggc atc 960
Glu Phe Ile Tyr Val Phe Asp Asp Glu Ala Asp Lys Leu Lys Gly Ile
305                 310                 315                 320
gac tac ctt gca caa ggt acg ctt tac aca gat atc atc gag agc ggt 1008
Asp Tyr Leu Ala Gln Gly Thr Leu Tyr Thr Asp Ile Ile Glu Ser Gly
                325                 330                 335
aca gca acg gcg caa acg atc aaa tct cac cac aat gtc ggc gga ctt 1056

Thr Ala Thr Ala Gln Thr Ile Lys Ser His His Asn Val Gly Gly Leu
            340                 345                 350
cct gaa gac atg cag ttt gag ctg atc gag ccg tta aat acg ctc ttc 1104
Pro Glu Asp Met Gln Phe Glu Leu Ile Glu Pro Leu Asn Thr Leu Phe
        355                 360                 365
aaa gac gaa gtg cgc gcg ctt ggc aca gag ctc ggc att ccg gat gaa 1152
Lys Asp Glu Val Arg Ala Leu Gly Thr Glu Leu Gly Ile Pro Asp Glu
    370                 375                 380
atc gta tgg cgt cag ccg ttc cca gga ccg gga ctc gga atc cgc gtt 1200
Ile Val Trp Arg Gln Pro Phe Pro Gly Pro Gly Leu Gly Ile Arg Val
385                 390                 395                 400
ctt ggc gaa gta aca gaa gaa aaa ctt gaa atc gtt cgt gaa tca gat 1248
Leu Gly Glu Val Thr Glu Glu Lys Leu Glu Ile Val Arg Glu Ser Asp
                405                 410                 415
gca atc ttg cgt gaa gaa att gca aat cac ggc tta gag cgt gat atc 1296
Ala Ile Leu Arg Glu Glu Ile Ala Asn His Gly Leu Glu Arg Asp Ile
            420                 425                 430
tgg caa tac ttc act gtt ctt cct gac atc cgc agc gtt ggt gtt atg 1344
Trp Gln Tyr Phe Thr Val Leu Pro Asp Ile Arg Ser Val Gly Val Met
        435                 440                 445
ggt gac gca aga aca tat gat tac aca atc gga atc cgc gcc gtt aca 1392
Gly Asp Ala Arg Thr Tyr Asp Tyr Thr Ile Gly Ile Arg Ala Val Thr
    450                 455                 460
tca atc gac ggc atg aca tct gac tgg gcg cgt atc ccg tgg gat gtg 1440
Ser Ile Asp Gly Met Thr Ser Asp Trp Ala Arg Ile Pro Trp Asp Val
465                 470                 475                 480
ctt gaa gtc att tcg aca cgt atc gta aac gaa gtg aag cac att aac 1488
Leu Glu Val Ile Ser Thr Arg Ile Val Asn Glu Val Lys His Ile Asn
                485                 490                 495
cgc gtg gtg tat gat att aca agt aag ccg cct gcg acg att gag tgg 1536
Arg Val Val Tyr Asp Ile Thr Ser Lys Pro Pro Ala Thr Ile Glu Trp
            500                 505                 510
gaa taa 1542
Glu

<210> 4
<211> 513
<212> PRT
<213> Bacillus subtilis

<400> 4

Met Thr Lys Leu Val Asn Glu Met Ile Leu Val Leu Asp Phe Gly Ser
1               5                   10                  15
Gln Tyr Asn Gln Leu Ile Thr Arg Arg Ile Arg Glu Phe Gly Val Tyr
            20                  25                  30
Ser Glu Leu His Pro His Thr Leu Thr Ala Glu Glu Ile Lys Lys Met
        35                  40                  45
Asn Pro Lys Gly Ile Ile Leu Ser Gly Gly Pro Asn Ser Val Tyr Asp
    50                  55                  60
Glu Asn Ser Phe Arg Cys Asp Glu Lys Ile Phe Glu Leu Asp Ile Pro
65                  70                  75                  80
Val Leu Gly Ile Cys Tyr Gly Met Gln Leu Met Thr His Tyr Leu Gly
                85                  90                  95
Gly Lys Val Glu Ala Ala Ser Gln Arg Glu Tyr Gly Lys Ala Asn Ile
            100                 105                 110
Arg Ile Glu Gly Thr Pro Asp Leu Phe Arg Asp Leu Pro Asn Glu Gln
        115                 120                 125
Val Val Trp Met Ser His Gly Asp Leu Val Val Glu Val Pro Glu Gly
    130                 135                 140
Phe Thr Val Asp Ala Thr Ser His His Cys Pro Asn Ser Ala Met Ser
145                 150                 155                 160
Lys Ala Asp Lys Lys Trp Tyr Gly Val Gln Phe His Pro Glu Val Arg
                165                 170                 175
His Ser Glu Tyr Gly Asn Asp Leu Leu Lys Asn Phe Val Phe Gly Val
            180                 185                 190
Cys Glu Cys Glu Gly Glu Trp Ser Met Glu Asn Phe Ile Glu Ile Glu

195                 200                 205
Met Gln Lys Ile Arg Glu Thr Val Gly Asp Lys Gln Val Leu Cys Ala
    210                 215                 220
Leu Ser Gly Gly Val Asp Ser Ser Val Val Ala Val Leu Ile His Lys
225                 230                 235                 240
Ala Ile Gly Asp Gln Leu Thr Cys Ile Phe Val Asp His Gly Leu Leu
                245                 250                 255
Arg Lys Gly Glu Ala Glu Gly Val Met Lys Thr Phe Ser Glu Gly Phe
            260                 265                 270
Asn Met Asn Val Ile Lys Val Asp Ala Lys Asp Arg Phe Leu Asn Lys
        275                 280                 285
Leu Lys Gly Val Ser Asp Pro Glu Gln Lys Arg Lys Ile Ile Gly Asn
    290                 295                 300
Glu Phe Ile Tyr Val Phe Asp Asp Glu Ala Asp Lys Leu Lys Gly Ile
305                 310                 315                 320
Asp Tyr Leu Ala Gln Gly Thr Leu Tyr Thr Asp Ile Ile Glu Ser Gly
                325                 330                 335
Thr Ala Thr Ala Gln Thr Ile Lys Ser His His Asn Val Gly Gly Leu
            340                 345                 350
Pro Glu Asp Met Gln Phe Glu Leu Ile Glu Pro Leu Asn Thr Leu Phe
        355                 360                 365
Lys Asp Glu Val Arg Ala Leu Gly Thr Glu Leu Gly Ile Pro Asp Glu
    370                 375                 380
Ile Val Trp Arg Gln Pro Phe Pro Gly Pro Gly Leu Gly Ile Arg Val
385                 390                 395                 400
Leu Gly Glu Val Thr Glu Glu Lys Leu Glu Ile Val Arg Glu Ser Asp
                405                 410                 415
Ala Ile Leu Arg Glu Glu Ile Ala Asn His Gly Leu Glu Arg Asp Ile
            420                 425                 430
Trp Gln Tyr Phe Thr Val Leu Pro Asp Ile Arg Ser Val Gly Val Met
        435                 440                 445
Gly Asp Ala Arg Thr Tyr Asp Tyr Thr Ile Gly Ile Arg Ala Val Thr
    450                 455                 460
Ser Ile Asp Gly Met Thr Ser Asp Trp Ala Arg Ile Pro Trp Asp Val
465                 470                 475                 480
Leu Glu Val Ile Ser Thr Arg Ile Val Asn Glu Val Lys His Ile Asn
                485                 490                 495
Arg Val Val Tyr Asp Ile Thr Ser Lys Pro Pro Ala Thr Ile Glu Trp
            500                 505                 510
Glu

<210>  5
<211>  1572
<212>  DNA
<213>  Corynebacterium glutamicum

<220>
<221>  CDS
<222>  (1)..(1569)

<400>  5
gtg agc ctt cag aca aat cat cgc cca gta ctc gtc gtt gac ttc ggc      48
Val Ser Leu Gln Thr Asn His Arg Pro Val Leu Val Val Asp Phe Gly
1               5                   10                  15
gca cag tac gcg cag ctg atc gca cgt cgt gtg cgt gag gcc ggc atc      96
Ala Gln Tyr Ala Gln Leu Ile Ala Arg Arg Val Arg Glu Ala Gly Ile
            20                  25                  30
tac tcc gaa gtc atc ccg cac acc gcc acc gca gac gat gtg cgc gct     144
Tyr Ser Glu Val Ile Pro His Thr Ala Thr Ala Asp Asp Val Arg Ala
        35                  40                  45
aaa aat gca gca gcc ctc gtc ctt tcc ggt ggc cca tcc tcc gtg tat     192
Lys Asn Ala Ala Ala Leu Val Leu Ser Gly Gly Pro Ser Ser Val Tyr
    50                  55                  60
gcc gag gga gca cca tcc ctt gac gct gag att ctt gat ctc gga ttg     240
Ala Glu Gly Ala Pro Ser Leu Asp Ala Glu Ile Leu Asp Leu Gly Leu
65                  70                  75                  80

cca gta ttt ggc att tgc tac ggc ttc caa gcc atg acc cac gcg ctt 288
Pro Val Phe Gly Ile Cys Tyr Gly Phe Gln Ala Met Thr His Ala Leu
                85                  90                  95
ggt ggc acc gtt gcc aac acc ggt aag cgc gaa tac gga cgc acc gac 336
Gly Gly Thr Val Ala Asn Thr Gly Lys Arg Glu Tyr Gly Arg Thr Asp
            100                 105                 110
atc aac gtt gcc ggt ggc gtc ctc cac gaa ggc ctc gaa gcc tgc cac 384
Ile Asn Val Ala Gly Gly Val Leu His Glu Gly Leu Glu Ala Cys His
        115                 120                 125
aag gtg tgg atg agc cac ggc gac gcc gtc tct gaa gcc cca gaa ggt 432
Lys Val Trp Met Ser His Gly Asp Ala Val Ser Glu Ala Pro Glu Gly
    130                 135                 140
ttc gta gtc acc gcg tcc tcc gaa ggt gcg cct gtc gca gct ttc gaa 480
Phe Val Val Thr Ala Ser Ser Glu Gly Ala Pro Val Ala Ala Phe Glu
145                 150                 155                 160
aac aag gaa cgc aaa atg gct ggc gtg cag tac cac cca gag gta ttg 528
Asn Lys Glu Arg Lys Met Ala Gly Val Gln Tyr His Pro Glu Val Leu
                165                 170                 175
cac tca cca cac ggc cag gca gtt ctg acc cgc ttc ctc act gag atc 576
His Ser Pro His Gly Gln Ala Val Leu Thr Arg Phe Leu Thr Glu Ile
            180                 185                 190
gca ggt cta gag cag aac tgg acc gca gca aac atc gct gaa gaa ctc 624
Ala Gly Leu Glu Gln Asn Trp Thr Ala Ala Asn Ile Ala Glu Glu Leu
        195                 200                 205
atc gaa aag gtc cgc gag cag atc ggc gaa gat ggc cgc gct att tgt 672
Ile Glu Lys Val Arg Glu Gln Ile Gly Glu Asp Gly Arg Ala Ile Cys
    210                 215                 220
ggc cta tcc ggt ggt gtg gac tcc gct gtt gcc ggt gct ttg gtg cag 720
Gly Leu Ser Gly Gly Val Asp Ser Ala Val Ala Gly Ala Leu Val Gln
225                 230                 235                 240
cgc gcc att ggt gac cgt ttg acc tgt gtc ttt gtt gac cac ggt ctg 768
Arg Ala Ile Gly Asp Arg Leu Thr Cys Val Phe Val Asp His Gly Leu
                245                 250                 255
ctg cgt gcc ggt gag cgc gag cag gtg gaa aaa gac ttc gtc gca gca 816
Leu Arg Ala Gly Glu Arg Glu Gln Val Glu Lys Asp Phe Val Ala Ala
            260                 265                 270
acc ggc gcc aag ctg gtt acc gtt gat gag cgc cag gca ttc cta tcc 864
Thr Gly Ala Lys Leu Val Thr Val Asp Glu Arg Gln Ala Phe Leu Ser
        275                 280                 285
aag ctg gcc gga gtt acc gaa cca gaa gca aag cgc aag gct atc ggc 912
Lys Leu Ala Gly Val Thr Glu Pro Glu Ala Lys Arg Lys Ala Ile Gly
    290                 295                 300
gct gag ttc atc cgc tcc ttc gag cgc gca gtt gcc ggt gtg ctg gaa 960
Ala Glu Phe Ile Arg Ser Phe Glu Arg Ala Val Ala Gly Val Leu Glu
305                 310                 315                 320
gaa gct cca gaa ggt tcc acc gtg gac ttc ctg gtt cag ggc acc ctg 1008
Glu Ala Pro Glu Gly Ser Thr Val Asp Phe Leu Val Gln Gly Thr Leu
                325                 330                 335
tac cca gac gtc gtg gaa tcc ggt ggt gga tct ggt acc gca aac atc 1056
Tyr Pro Asp Val Val Glu Ser Gly Gly Gly Ser Gly Thr Ala Asn Ile
            340                 345                 350
aag agc cac cac aac gtc ggt gga ctg cca gac gat gtg gaa ttc aag 1104
Lys Ser His His Asn Val Gly Gly Leu Pro Asp Asp Val Glu Phe Lys
        355                 360                 365
ctt gtt gag cca ctg cgt gac ctc ttc aaa gac gaa gtc cgt gcc gtt 1152
Leu Val Glu Pro Leu Arg Asp Leu Phe Lys Asp Glu Val Arg Ala Val
    370                 375                 380
ggc cgt gaa ctt ggc ctg cct gag gaa atc gtt ggc cgc cag cca ttc 1200
Gly Arg Glu Leu Gly Leu Pro Glu Glu Ile Val Gly Arg Gln Pro Phe
385                 390                 395                 400
cca gga cca gga ctt ggt atc cgc atc atc ggt gaa gtc acc gaa gat 1248
Pro Gly Pro Gly Leu Gly Ile Arg Ile Ile Gly Glu Val Thr Glu Asp
                405                 410                 415
cgc cta gaa acc ctc cgc cac gct gac ctg atc gcc cgc acc gag ctc 1296
Arg Leu Glu Thr Leu Arg His Ala Asp Leu Ile Ala Arg Thr Glu Leu
            420                 425                 430
acc gaa gcc gga ctt gac ggc gtg atc tgg cag tgc cca gta gtc ctc 1344
Thr Glu Ala Gly Leu Asp Gly Val Ile Trp Gln Cys Pro Val Val Leu
        435                 440                 445

ctg gca gat gtc cgc tct gtt ggt gtt caa ggc gat ggc cgc acc tac      1392
Leu Ala Asp Val Arg Ser Val Gly Val Gln Gly Asp Gly Arg Thr Tyr
    450                 455                 460
gga cac cca atc gtg ctg cgc cca gtg tct tcc gaa gac gca atg acc      1440
Gly His Pro Ile Val Leu Arg Pro Val Ser Ser Glu Asp Ala Met Thr
465                 470                 475                 480
gcc gac tgg acc cgc ctg cca tac gag gtt ctg gag aag atc tcc acc      1488
Ala Asp Trp Thr Arg Leu Pro Tyr Glu Val Leu Glu Lys Ile Ser Thr
                485                 490                 495
cgc atc acc aac gaa gtt cca gat gtg aac cgc gtg gtg ctg gac gta      1536
Arg Ile Thr Asn Glu Val Pro Asp Val Asn Arg Val Val Leu Asp Val
            500                 505                 510
acc tcc aag cca cca gga acc atc gaa tgg gag tag                      1572
Thr Ser Lys Pro Pro Gly Thr Ile Glu Trp Glu
        515                 520


<210>  6
<211>  523
<212>  PRT
<213>  Corynebacterium glutamicum

<400>  6

Val Ser Leu Gln Thr Asn His Arg Pro Val Leu Val Val Asp Phe Gly
1               5                   10                  15
Ala Gln Tyr Ala Gln Leu Ile Ala Arg Arg Val Arg Glu Ala Gly Ile
            20                  25                  30
Tyr Ser Glu Val Ile Pro His Thr Ala Thr Ala Asp Asp Val Arg Ala
        35                  40                  45
Lys Asn Ala Ala Ala Leu Val Leu Ser Gly Gly Pro Ser Ser Val Tyr
    50                  55                  60
Ala Glu Gly Ala Pro Ser Leu Asp Ala Glu Ile Leu Asp Leu Gly Leu
65                  70                  75                  80
Pro Val Phe Gly Ile Cys Tyr Gly Phe Gln Ala Met Thr His Ala Leu
                85                  90                  95
Gly Gly Thr Val Ala Asn Thr Gly Lys Arg Glu Tyr Gly Arg Thr Asp
            100                 105                 110
Ile Asn Val Ala Gly Gly Val Leu His Glu Gly Leu Glu Ala Cys His
        115                 120                 125
Lys Val Trp Met Ser His Gly Asp Ala Val Ser Glu Ala Pro Glu Gly
    130                 135                 140
Phe Val Val Thr Ala Ser Ser Glu Gly Ala Pro Val Ala Ala Phe Glu
145                 150                 155                 160
Asn Lys Glu Arg Lys Met Ala Gly Val Gln Tyr His Pro Glu Val Leu
                165                 170                 175
His Ser Pro His Gly Gln Ala Val Leu Thr Arg Phe Leu Thr Glu Ile
            180                 185                 190
Ala Gly Leu Glu Gln Asn Trp Thr Ala Ala Asn Ile Ala Glu Glu Leu
        195                 200                 205
Ile Glu Lys Val Arg Glu Gln Ile Gly Glu Asp Gly Arg Ala Ile Cys
    210                 215                 220
Gly Leu Ser Gly Gly Val Asp Ser Ala Val Ala Gly Ala Leu Val Gln
225                 230                 235                 240
Arg Ala Ile Gly Asp Arg Leu Thr Cys Val Phe Val Asp His Gly Leu
                245                 250                 255
Leu Arg Ala Gly Glu Arg Glu Gln Val Glu Lys Asp Phe Val Ala Ala
            260                 265                 270
Thr Gly Ala Lys Leu Val Thr Val Asp Glu Arg Gln Ala Phe Leu Ser
        275                 280                 285
Lys Leu Ala Gly Val Thr Glu Pro Glu Ala Lys Arg Lys Ala Ile Gly
    290                 295                 300
Ala Glu Phe Ile Arg Ser Phe Glu Arg Ala Val Ala Gly Val Leu Glu
305                 310                 315                 320
Glu Ala Pro Glu Gly Ser Thr Val Asp Phe Leu Val Gln Gly Thr Leu
                325                 330                 335
Tyr Pro Asp Val Val Glu Ser Gly Gly Gly Ser Gly Thr Ala Asn Ile
            340                 345                 350
Lys Ser His His Asn Val Gly Gly Leu Pro Asp Asp Val Glu Phe Lys

                355                 360                 365
Leu Val Glu Pro Leu Arg Asp Leu Phe Lys Asp Glu Val Arg Ala Val
        370                 375                 380
Gly Arg Glu Leu Gly Leu Pro Glu Glu Ile Val Gly Arg Gln Pro Phe
385                 390                 395                 400
Pro Gly Pro Gly Leu Gly Ile Arg Ile Ile Gly Glu Val Thr Glu Asp
                405                 410                 415
Arg Leu Glu Thr Leu Arg His Ala Asp Leu Ile Ala Arg Thr Glu Leu
            420                 425                 430
Thr Glu Ala Gly Leu Asp Gly Val Ile Trp Gln Cys Pro Val Val Leu
        435                 440                 445
Leu Ala Asp Val Arg Ser Val Gly Val Gln Gly Asp Gly Arg Thr Tyr
    450                 455                 460
Gly His Pro Ile Val Leu Arg Pro Val Ser Ser Glu Asp Ala Met Thr
465                 470                 475                 480
Ala Asp Trp Thr Arg Leu Pro Tyr Glu Val Leu Glu Lys Ile Ser Thr
                485                 490                 495
Arg Ile Thr Asn Glu Val Pro Asp Val Asn Arg Val Val Leu Asp Val
            500                 505                 510
Thr Ser Lys Pro Pro Gly Thr Ile Glu Trp Glu
        515                 520

<210>  7
<211>  1575
<212>  DNA
<213>  Corynebacterium ammoniagenes

<220>
<221>  CDS
<222>  (1)..(1572)

<400>  7
gtg act caa cct gca aca act ccg cgc cca gtc ctc gtg gtg gat ttc      48
Val Thr Gln Pro Ala Thr Thr Pro Arg Pro Val Leu Val Val Asp Phe
1               5                   10                  15
ggt gcc caa tac gca cag ctg att gct cgt cgc gta cgt gag gca tcg      96
Gly Ala Gln Tyr Ala Gln Leu Ile Ala Arg Arg Val Arg Glu Ala Ser
            20                  25                  30
att tac tcc gag gta gtc cca cat tcc gcc acc gtt aaa gag att aaa      144
Ile Tyr Ser Glu Val Val Pro His Ser Ala Thr Val Lys Glu Ile Lys
        35                  40                  45
gct aaa aac cct gca gct ttg att ttg tcc ggt ggc ccg tcc tct gtt      192
Ala Lys Asn Pro Ala Ala Leu Ile Leu Ser Gly Gly Pro Ser Ser Val
    50                  55                  60
tat gcc gat ggc gcc ccg caa tta aag cct gaa ctg ctc gag ctt ggt      240
Tyr Ala Asp Gly Ala Pro Gln Leu Lys Pro Glu Leu Leu Glu Leu Gly
65                  70                  75                  80
gtg cca gtc ttt ggc atc tgc tac ggc ttc caa gcc atg aac cat gct      288
Val Pro Val Phe Gly Ile Cys Tyr Gly Phe Gln Ala Met Asn His Ala
                85                  90                  95
ttg ggt ggc aac gtt gcg caa acc ggt gac cgt gaa tac ggc cgc acc      336
Leu Gly Gly Asn Val Ala Gln Thr Gly Asp Arg Glu Tyr Gly Arg Thr
            100                 105                 110
gaa atc acc cat acc ggt ggt gtg ctg cac gac ggc tta gaa gaa aac      384
Glu Ile Thr His Thr Gly Gly Val Leu His Asp Gly Leu Glu Glu Asn
        115                 120                 125
cac aag gtc tgg atg tcc cac ggt gat gct gtg gat aag gca cct gag      432
His Lys Val Trp Met Ser His Gly Asp Ala Val Asp Lys Ala Pro Glu
    130                 135                 140
ggc ttt acc gtg acc gca tcg tcg gct ggt gcg ccg gtt gca gcg atg      480
Gly Phe Thr Val Thr Ala Ser Ser Ala Gly Ala Pro Val Ala Ala Met
145                 150                 155                 160
gaa tgc gtg gcc aag caa atg gct ggt gtg caa tac cac ccc gag gtt      528
Glu Cys Val Ala Lys Gln Met Ala Gly Val Gln Tyr His Pro Glu Val
                165                 170                 175
atg cat tcc cca cac gga cag gaa gta ctc gtt cgc ttc ctc acc gag      576
Met His Ser Pro His Gly Gln Glu Val Leu Val Arg Phe Leu Thr Glu

180                 185                 190
gta gca ggg cta gag cag acc tgg acc tcg gca aat att gcg cag cag      624
Val Ala Gly Leu Glu Gln Thr Trp Thr Ser Ala Asn Ile Ala Gln Gln
        195                 200                 205
ctt atc gat gat gtc cgc gcg caa atc ggc cct gaa ggc cgc gct att      672
Leu Ile Asp Asp Val Arg Ala Gln Ile Gly Pro Glu Gly Arg Ala Ile
    210                 215                 220
tgt ggc ctg tcg ggc ggc gtg gac tcc gca gtc gct gca gcg ctc gtg      720
Cys Gly Leu Ser Gly Gly Val Asp Ser Ala Val Ala Ala Ala Leu Val
225                 230                 235                 240
cag cgc gcc att ggc gac cgt ttg acc tgt gtg ttc gtg gac cac ggt      768
Gln Arg Ala Ile Gly Asp Arg Leu Thr Cys Val Phe Val Asp His Gly
                245                 250                 255
ctg ctg cgc gcc ggt gag cgt gag cag gta gaa aag gac ttc gtg gct      816
Leu Leu Arg Ala Gly Glu Arg Glu Gln Val Glu Lys Asp Phe Val Ala
            260                 265                 270
tcg act ggt gcg aag ctg att acc gcg cat gaa gct gat gct ttc ttg      864
Ser Thr Gly Ala Lys Leu Ile Thr Ala His Glu Ala Asp Ala Phe Leu
        275                 280                 285
tct aag ctc gcc ggt gtt acc gat cct gag gct aag cgc aag gct atc      912
Ser Lys Leu Ala Gly Val Thr Asp Pro Glu Ala Lys Arg Lys Ala Ile
    290                 295                 300
ggc gcg gaa ttc atc cgt tcc ttt gag cgt gct gtg gca cag gct ttg      960
Gly Ala Glu Phe Ile Arg Ser Phe Glu Arg Ala Val Ala Gln Ala Leu
305                 310                 315                 320
gaa gaa tct cct gaa gac tcc aca gtg gac ttc ctg gtc cag ggc acc     1008
Glu Glu Ser Pro Glu Asp Ser Thr Val Asp Phe Leu Val Gln Gly Thr
                325                 330                 335
ttg tat ccg gat gtg gtt gaa tcc ggc ggc ggt gac ggc acc gca aat     1056
Leu Tyr Pro Asp Val Val Glu Ser Gly Gly Gly Asp Gly Thr Ala Asn
            340                 345                 350
atc aag tcc cac cac aat gtt ggc ggc ctg cca gac gat gtc gaa ttc     1104
Ile Lys Ser His His Asn Val Gly Gly Leu Pro Asp Asp Val Glu Phe
        355                 360                 365
gaa ctc gtc gag cca cta cgc ctg ctg ttt aag gac gaa gtc cgt gcc     1152
Glu Leu Val Glu Pro Leu Arg Leu Leu Phe Lys Asp Glu Val Arg Ala
    370                 375                 380
gtc ggc cgc gag ctc ggc ctg cct gag gaa atc gtt gcc cgc cag cca     1200
Val Gly Arg Glu Leu Gly Leu Pro Glu Glu Ile Val Ala Arg Gln Pro
385                 390                 395                 400
ttc cct ggc cct ggc cta ggc atc cgc atc atc ggt gaa gtc acc gag     1248
Phe Pro Gly Pro Gly Leu Gly Ile Arg Ile Ile Gly Glu Val Thr Glu
                405                 410                 415
gag cgt cta gaa atc ctg cgt caa gca gac ctg att gcg cgt acc gag     1296
Glu Arg Leu Glu Ile Leu Arg Gln Ala Asp Leu Ile Ala Arg Thr Glu
            420                 425                 430
ctg acc aac gct ggc ctc gac ggt gat atc tgg cag tgc cca gtc gta     1344
Leu Thr Asn Ala Gly Leu Asp Gly Asp Ile Trp Gln Cys Pro Val Val
        435                 440                 445
ctg ctt gcc gat gtc cgc tcc gtc gga gtc caa ggc gac ggc cgc acc     1392
Leu Leu Ala Asp Val Arg Ser Val Gly Val Gln Gly Asp Gly Arg Thr
    450                 455                 460
tac ggc cac cca atc gtg ctg cgc cca gtg tca tcc gag gat gcc atg     1440
Tyr Gly His Pro Ile Val Leu Arg Pro Val Ser Ser Glu Asp Ala Met
465                 470                 475                 480
acc gcc gac tgg acc cgc gtt cct tac gac gtc cta gag aaa atc tcc     1488
Thr Ala Asp Trp Thr Arg Val Pro Tyr Asp Val Leu Glu Lys Ile Ser
                485                 490                 495
acc cgc att acc aac gaa gtc aac gac gtc aac cgc gtg gtc gtc gac     1536
Thr Arg Ile Thr Asn Glu Val Asn Asp Val Asn Arg Val Val Val Asp
            500                 505                 510
atc acc tcc aag cca ccg gga acc atc gag tgg gag taa                 1575
Ile Thr Ser Lys Pro Pro Gly Thr Ile Glu Trp Glu
        515                 520

<210>  8
<211>  524
<212>  PRT

<213>  Corynebacterium ammoniagenes

<400>  8
Val Thr Gln Pro Ala Thr Thr Pro Arg Pro Val Leu Val Val Asp Phe
1               5                   10                  15
Gly Ala Gln Tyr Ala Gln Leu Ile Ala Arg Arg Val Arg Glu Ala Ser
            20                  25                  30
Ile Tyr Ser Glu Val Val Pro His Ser Ala Thr Val Lys Glu Ile Lys
        35                  40                  45
Ala Lys Asn Pro Ala Ala Leu Ile Leu Ser Gly Gly Pro Ser Ser Val
    50                  55                  60
Tyr Ala Asp Gly Ala Pro Gln Leu Lys Pro Glu Leu Leu Glu Leu Gly
65                  70                  75                  80
Val Pro Val Phe Gly Ile Cys Tyr Gly Phe Gln Ala Met Asn His Ala
                85                  90                  95
Leu Gly Gly Asn Val Ala Gln Thr Gly Asp Arg Glu Tyr Gly Arg Thr
            100                 105                 110
Glu Ile Thr His Thr Gly Gly Val Leu His Asp Gly Leu Glu Glu Asn
        115                 120                 125
His Lys Val Trp Met Ser His Gly Asp Ala Val Asp Lys Ala Pro Glu
    130                 135                 140
Gly Phe Thr Val Thr Ala Ser Ser Ala Gly Ala Pro Val Ala Ala Met
145                 150                 155                 160
Glu Cys Val Ala Lys Gln Met Ala Gly Val Gln Tyr His Pro Glu Val
                165                 170                 175
Met His Ser Pro His Gly Gln Glu Val Leu Val Arg Phe Leu Thr Glu
            180                 185                 190
Val Ala Gly Leu Glu Gln Thr Trp Thr Ser Ala Asn Ile Ala Gln Gln
        195                 200                 205
Leu Ile Asp Asp Val Arg Ala Gln Ile Gly Pro Glu Gly Arg Ala Ile
    210                 215                 220
Cys Gly Leu Ser Gly Gly Val Asp Ser Ala Val Ala Ala Ala Leu Val
225                 230                 235                 240
Gln Arg Ala Ile Gly Asp Arg Leu Thr Cys Val Phe Val Asp His Gly
                245                 250                 255
Leu Leu Arg Ala Gly Glu Arg Glu Gln Val Glu Lys Asp Phe Val Ala
            260                 265                 270
Ser Thr Gly Ala Lys Leu Ile Thr Ala His Glu Ala Asp Ala Phe Leu
        275                 280                 285
Ser Lys Leu Ala Gly Val Thr Asp Pro Glu Ala Lys Arg Lys Ala Ile
    290                 295                 300
Gly Ala Glu Phe Ile Arg Ser Phe Glu Arg Ala Val Ala Gln Ala Leu
305                 310                 315                 320
Glu Glu Ser Pro Glu Asp Ser Thr Val Asp Phe Leu Val Gln Gly Thr
                325                 330                 335
Leu Tyr Pro Asp Val Val Glu Ser Gly Gly Gly Asp Gly Thr Ala Asn
            340                 345                 350
Ile Lys Ser His His Asn Val Gly Gly Leu Pro Asp Asp Val Glu Phe
        355                 360                 365
Glu Leu Val Glu Pro Leu Arg Leu Leu Phe Lys Asp Glu Val Arg Ala
    370                 375                 380
Val Gly Arg Glu Leu Gly Leu Pro Glu Glu Ile Val Ala Arg Gln Pro
385                 390                 395                 400
Phe Pro Gly Pro Gly Leu Gly Ile Arg Ile Ile Gly Glu Val Thr Glu
                405                 410                 415
Glu Arg Leu Glu Ile Leu Arg Gln Ala Asp Leu Ile Ala Arg Thr Glu
            420                 425                 430
Leu Thr Asn Ala Gly Leu Asp Gly Asp Ile Trp Gln Cys Pro Val Val
        435                 440                 445
Leu Leu Ala Asp Val Arg Ser Val Gly Val Gln Gly Asp Gly Arg Thr
    450                 455                 460
Tyr Gly His Pro Ile Val Leu Arg Pro Val Ser Ser Glu Asp Ala Met
465                 470                 475                 480
Thr Ala Asp Trp Thr Arg Val Pro Tyr Asp Val Leu Glu Lys Ile Ser
                485                 490                 495
Thr Arg Ile Thr Asn Glu Val Asn Asp Val Asn Arg Val Val Val Asp
            500                 505                 510
Ile Thr Ser Lys Pro Pro Gly Thr Ile Glu Trp Glu
        515                 520

<210>  9
<211>  1467
<212>  DNA
<213>  Escherichia coli


<220>
<221>  CDS
<222>  (1)..(1464)

<400>  9
atg cta cgt atc gct aaa gaa gct ctg acg ttt gac gac gtt ctc ctc        48
Met Leu Arg Ile Ala Lys Glu Ala Leu Thr Phe Asp Asp Val Leu Leu
1               5                   10                  15
gtt cct gct cac tct acc gtt ctg ccg aat act gct gac ctc agc acc        96
Val Pro Ala His Ser Thr Val Leu Pro Asn Thr Ala Asp Leu Ser Thr
            20                  25                  30
cag ctg acg aaa act att cgt ctg aat atc cct atg ctt tcc gca gca       144
Gln Leu Thr Lys Thr Ile Arg Leu Asn Ile Pro Met Leu Ser Ala Ala
        35                  40                  45
atg gat acc gta acg gaa gcg cgc ctg gct att gct ctg gct cag gaa       192
Met Asp Thr Val Thr Glu Ala Arg Leu Ala Ile Ala Leu Ala Gln Glu
    50                  55                  60
ggc ggt atc ggc ttt atc cac aaa aac atg tcc att gaa cgc cag gca       240
Gly Gly Ile Gly Phe Ile His Lys Asn Met Ser Ile Glu Arg Gln Ala
65                  70                  75                  80
gaa gaa gtt cgc cgt gtg aaa aaa cac gaa tct ggt gtg gtg act gat       288
Glu Glu Val Arg Arg Val Lys Lys His Glu Ser Gly Val Val Thr Asp
                85                  90                  95
ccg cag act gtt ctg cca acc acg acg ctg cgc gaa gtg aaa gaa ctg       336
Pro Gln Thr Val Leu Pro Thr Thr Thr Leu Arg Glu Val Lys Glu Leu
            100                 105                 110
acc gag cgt aac ggt ttt gcg ggc tat ccg gtc gtt acc gaa gaa aac       384
Thr Glu Arg Asn Gly Phe Ala Gly Tyr Pro Val Val Thr Glu Glu Asn
        115                 120                 125
gaa ctg gtg ggt att atc acc ggt cgt gac gtg cgt ttt gtt acc gac       432
Glu Leu Val Gly Ile Ile Thr Gly Arg Asp Val Arg Phe Val Thr Asp
    130                 135                 140
ctg aac cag ccg gtt agc gtt tac atg acg ccg aaa gag cgt ctg gtc       480
Leu Asn Gln Pro Val Ser Val Tyr Met Thr Pro Lys Glu Arg Leu Val
145                 150                 155                 160
acc gtg cgt gaa ggt gaa gcc cgt gaa gtg gtg ctg gca aaa atg cac       528
Thr Val Arg Glu Gly Glu Ala Arg Glu Val Val Leu Ala Lys Met His
                165                 170                 175
gaa aaa cgc gtt gaa aaa gcg ctg gtg gtt gat gac gaa ttc cac ctg       576
Glu Lys Arg Val Glu Lys Ala Leu Val Val Asp Asp Glu Phe His Leu
            180                 185                 190
atc ggc atg atc acc gtg aaa gac ttc cag aaa gcg gaa cgt aaa ccg       624
Ile Gly Met Ile Thr Val Lys Asp Phe Gln Lys Ala Glu Arg Lys Pro
        195                 200                 205
aac gcc tgt aaa gac gag caa ggc cgt ctg cgt gtt ggt gca gcg gtt       672
Asn Ala Cys Lys Asp Glu Gln Gly Arg Leu Arg Val Gly Ala Ala Val
    210                 215                 220
ggc gca ggt gcg ggt aac gaa gag cgt gtt gac gcg ctg gtt gcc gca       720
Gly Ala Gly Ala Gly Asn Glu Glu Arg Val Asp Ala Leu Val Ala Ala
225                 230                 235                 240
ggc gtt gac gtt ctg ctg atc gac tcc tcc cac ggt cac tca gaa ggt       768
Gly Val Asp Val Leu Leu Ile Asp Ser Ser His Gly His Ser Glu Gly
                245                 250                 255
gta ctg caa cgt atc cgt gaa acc cgt gct aaa tat ccg gat ctg caa       816
Val Leu Gln Arg Ile Arg Glu Thr Arg Ala Lys Tyr Pro Asp Leu Gln
            260                 265                 270
att atc ggc ggc aac gtg gca aca gct gca ggt gca cgc gct ctg gca       864
Ile Ile Gly Gly Asn Val Ala Thr Ala Ala Gly Ala Arg Ala Leu Ala
        275                 280                 285
gaa gct ggt tgc agt gcg gtt aaa gtc ggc att ggc cct ggc tct atc       912
Glu Ala Gly Cys Ser Ala Val Lys Val Gly Ile Gly Pro Gly Ser Ile

                290                 295                 300
tgt aca act cgt atc gtg act ggc gtc ggt gtt ccg cag att acc gct        960
Cys Thr Thr Arg Ile Val Thr Gly Val Gly Val Pro Gln Ile Thr Ala
305                 310                 315                 320
gtt gct gac gca gta gaa gcc ctg gaa ggc acc ggt att ccg gtt atc        1008
Val Ala Asp Ala Val Glu Ala Leu Glu Gly Thr Gly Ile Pro Val Ile
                325                 330                 335
gct gat ggc ggt att cgc ttc tcc ggc gac atc gcc aaa gct atc gcc        1056
Ala Asp Gly Gly Ile Arg Phe Ser Gly Asp Ile Ala Lys Ala Ile Ala
            340                 345                 350
gct ggc gca agc gcg gtg atg gta ggt tcc atg ctg gcg ggt act gaa        1104
Ala Gly Ala Ser Ala Val Met Val Gly Ser Met Leu Ala Gly Thr Glu
        355                 360                 365
gaa tct ccg ggt gaa atc gaa ctc tac cag ggc cgt tct tac aaa tct        1152
Glu Ser Pro Gly Glu Ile Glu Leu Tyr Gln Gly Arg Ser Tyr Lys Ser
    370                 375                 380
tac cgt ggt atg ggt tcc ctg ggc gcg atg tcc aaa ggt tcc tct gac        1200
Tyr Arg Gly Met Gly Ser Leu Gly Ala Met Ser Lys Gly Ser Ser Asp
385                 390                 395                 400
cgt tat ttc cag agc gat aac gct gcc gac aaa ctg gtg ccg gaa ggt        1248
Arg Tyr Phe Gln Ser Asp Asn Ala Ala Asp Lys Leu Val Pro Glu Gly
                405                 410                 415
atc gaa ggt cgc gta gcc tat aaa ggt cgc ctg aaa gag atc att cac        1296
Ile Glu Gly Arg Val Ala Tyr Lys Gly Arg Leu Lys Glu Ile Ile His
            420                 425                 430
cag cag atg ggc ggc ctg cgc tcc tgt atg ggt ctg acc ggc tgt ggt        1344
Gln Gln Met Gly Gly Leu Arg Ser Cys Met Gly Leu Thr Gly Cys Gly
        435                 440                 445
act atc gac gaa ctg cgt act aaa gcg gag ttt gta cgt atc agc ggt        1392
Thr Ile Asp Glu Leu Arg Thr Lys Ala Glu Phe Val Arg Ile Ser Gly
    450                 455                 460
gcg ggc att cag gaa agc cac gtt cac gac gtg acc att act aaa gag        1440
Ala Gly Ile Gln Glu Ser His Val His Asp Val Thr Ile Thr Lys Glu
465                 470                 475                 480
tcc ccg aac tac cgt ctg ggc tcc tga                                    1467
Ser Pro Asn Tyr Arg Leu Gly Ser
                485


<210>  10
<211>  488
<212>  PRT
<213>  Escherichia coli

<400>  10
Met Leu Arg Ile Ala Lys Glu Ala Leu Thr Phe Asp Asp Val Leu Leu
1               5                   10                  15
Val Pro Ala His Ser Thr Val Leu Pro Asn Thr Ala Asp Leu Ser Thr
            20                  25                  30
Gln Leu Thr Lys Thr Ile Arg Leu Asn Ile Pro Met Leu Ser Ala Ala
        35                  40                  45
Met Asp Thr Val Thr Glu Ala Arg Leu Ala Ile Ala Leu Ala Gln Glu
    50                  55                  60
Gly Gly Ile Gly Phe Ile His Lys Asn Met Ser Ile Glu Arg Gln Ala
65                  70                  75                  80
Glu Glu Val Arg Arg Val Lys Lys His Glu Ser Gly Val Val Thr Asp
                85                  90                  95
Pro Gln Thr Val Leu Pro Thr Thr Thr Leu Arg Glu Val Lys Glu Leu
            100                 105                 110
Thr Glu Arg Asn Gly Phe Ala Gly Tyr Pro Val Val Thr Glu Glu Asn
        115                 120                 125
Glu Leu Val Gly Ile Ile Thr Gly Arg Asp Val Arg Phe Val Thr Asp
    130                 135                 140
Leu Asn Gln Pro Val Ser Val Tyr Met Thr Pro Lys Glu Arg Leu Val
145                 150                 155                 160
Thr Val Arg Glu Gly Glu Ala Arg Glu Val Val Leu Ala Lys Met His
                165                 170                 175
Glu Lys Arg Val Glu Lys Ala Leu Val Val Asp Asp Glu Phe His Leu
            180                 185                 190

Ile Gly Met Ile Thr Val Lys Asp Phe Gln Lys Ala Glu Arg Lys Pro
        195                 200                 205
Asn Ala Cys Lys Asp Glu Gln Gly Arg Leu Arg Val Gly Ala Ala Val
    210                 215                 220
Gly Ala Gly Ala Gly Asn Glu Glu Arg Val Asp Ala Leu Val Ala Ala
225                 230                 235                 240
Gly Val Asp Val Leu Leu Ile Asp Ser Ser His Gly His Ser Glu Gly
                245                 250                 255
Val Leu Gln Arg Ile Arg Glu Thr Arg Ala Lys Tyr Pro Asp Leu Gln
            260                 265                 270
Ile Ile Gly Gly Asn Val Ala Thr Ala Ala Gly Ala Arg Ala Leu Ala
        275                 280                 285
Glu Ala Gly Cys Ser Ala Val Lys Val Gly Ile Gly Pro Gly Ser Ile
    290                 295                 300
Cys Thr Thr Arg Ile Val Thr Gly Val Gly Val Pro Gln Ile Thr Ala
305                 310                 315                 320
Val Ala Asp Ala Val Glu Ala Leu Glu Gly Thr Gly Ile Pro Val Ile
                325                 330                 335
Ala Asp Gly Gly Ile Arg Phe Ser Gly Asp Ile Ala Lys Ala Ile Ala
            340                 345                 350
Ala Gly Ala Ser Ala Val Met Val Gly Ser Met Leu Ala Gly Thr Glu
        355                 360                 365
Glu Ser Pro Gly Glu Ile Glu Leu Tyr Gln Gly Arg Ser Tyr Lys Ser
    370                 375                 380
Tyr Arg Gly Met Gly Ser Leu Gly Ala Met Ser Lys Gly Ser Ser Asp
385                 390                 395                 400
Arg Tyr Phe Gln Ser Asp Asn Ala Ala Asp Lys Leu Val Pro Glu Gly
                405                 410                 415
Ile Glu Gly Arg Val Ala Tyr Lys Gly Arg Leu Lys Glu Ile Ile His
            420                 425                 430
Gln Gln Met Gly Gly Leu Arg Ser Cys Met Gly Leu Thr Gly Cys Gly
        435                 440                 445
Thr Ile Asp Glu Leu Arg Thr Lys Ala Glu Phe Val Arg Ile Ser Gly
    450                 455                 460
Ala Gly Ile Gln Glu Ser His Val His Asp Val Thr Ile Thr Lys Glu
465                 470                 475                 480
Ser Pro Asn Tyr Arg Leu Gly Ser
                485

<210>  11
<211>  1467
<212>  DNA
<213>  Bacillus subtilis

<220>
<221>  CDS
<222>  (1)..(1464)

<400>  11
atg tgg gaa agt aaa ttt tca aaa gaa ggc tta acg ttc gac gat gtg        48
Met Trp Glu Ser Lys Phe Ser Lys Glu Gly Leu Thr Phe Asp Asp Val
1               5                   10                  15
ctg ctt gta cca gca aag tct gag gta ctt ccg cgt gat gtg gat tta        96
Leu Leu Val Pro Ala Lys Ser Glu Val Leu Pro Arg Asp Val Asp Leu
            20                  25                  30
tct gta gaa ctt aca aaa acg tta aag cta aat att cct gtc atc agc       144
Ser Val Glu Leu Thr Lys Thr Leu Lys Leu Asn Ile Pro Val Ile Ser
        35                  40                  45
gca ggt atg gac act gta aca gaa tca gca atg gca att gca atg gca       192
Ala Gly Met Asp Thr Val Thr Glu Ser Ala Met Ala Ile Ala Met Ala
    50                  55                  60
aga cag ggc ggc ttg ggc atc att cac aaa aat atg tcc att gaa cag       240
Arg Gln Gly Gly Leu Gly Ile Ile His Lys Asn Met Ser Ile Glu Gln
65                  70                  75                  80
cag gct gaa caa gtt gat aaa gta aag cgt tct gag cgc ggc gtt atc       288
Gln Ala Glu Gln Val Asp Lys Val Lys Arg Ser Glu Arg Gly Val Ile
                85                  90                  95

aca aat ccc ttc ttt tta act cct gat cac caa gta ttt gat gcg gag 336
Thr Asn Pro Phe Phe Leu Thr Pro Asp His Gln Val Phe Asp Ala Glu
            100                 105                 110
cat ttg atg ggg aaa tac aga att tcc ggt gtt ccg att gta aat aac 384
His Leu Met Gly Lys Tyr Arg Ile Ser Gly Val Pro Ile Val Asn Asn
        115                 120                 125
gaa gaa gac cag aag ctt gtt gga att att aca aac cgt gac ctt cgt 432
Glu Glu Asp Gln Lys Leu Val Gly Ile Ile Thr Asn Arg Asp Leu Arg
    130                 135                 140
ttt att tct gac tac tca atg aaa atc agc gac gtc atg acg aaa gaa 480
Phe Ile Ser Asp Tyr Ser Met Lys Ile Ser Asp Val Met Thr Lys Glu
145                 150                 155                 160
gag cta gtt act gca tct gta gga act act ctg gat gaa gct gaa aag 528
Glu Leu Val Thr Ala Ser Val Gly Thr Thr Leu Asp Glu Ala Glu Lys
                165                 170                 175
att ttg cag aaa cat aaa att gaa aag ctt cct ctc gta gat gac cag 576
Ile Leu Gln Lys His Lys Ile Glu Lys Leu Pro Leu Val Asp Asp Gln
            180                 185                 190
aat aaa tta aaa ggt ctt atc aca att aaa gac att gaa aaa gtc att 624
Asn Lys Leu Lys Gly Leu Ile Thr Ile Lys Asp Ile Glu Lys Val Ile
        195                 200                 205
gag ttc ccg aac tca tct aaa gac att cac ggc cgc ctg atc gtt ggc 672
Glu Phe Pro Asn Ser Ser Lys Asp Ile His Gly Arg Leu Ile Val Gly
    210                 215                 220
gcg gca gtt ggt gta act ggc gat aca atg act cgc gtc aaa aag ctt 720
Ala Ala Val Gly Val Thr Gly Asp Thr Met Thr Arg Val Lys Lys Leu
225                 230                 235                 240
gtt gaa gcc aat gtt gat gtg att gtt atc gat aca gct cac gga cac 768
Val Glu Ala Asn Val Asp Val Ile Val Ile Asp Thr Ala His Gly His
                245                 250                 255
tct caa ggc gtt tta aac aca gtc aca aaa atc cgt gaa acg tat ccc 816
Ser Gln Gly Val Leu Asn Thr Val Thr Lys Ile Arg Glu Thr Tyr Pro
            260                 265                 270
gaa tta aac att att gct gga aac gtg gca aca gct gaa gcg aca aga 864
Glu Leu Asn Ile Ile Ala Gly Asn Val Ala Thr Ala Glu Ala Thr Arg
        275                 280                 285
gcg ctt atc gaa gct gga gca gac gtt gtc aaa gtt gga ata ggg cct 912
Ala Leu Ile Glu Ala Gly Ala Asp Val Val Lys Val Gly Ile Gly Pro
    290                 295                 300
ggt tca att tgt act aca cgt gtt gta gcc ggc gtg ggt gtt ccg caa 960
Gly Ser Ile Cys Thr Thr Arg Val Val Ala Gly Val Gly Val Pro Gln
305                 310                 315                 320
att aca gca att tat gat tgt gcg act gaa gca aga aaa cac ggc aaa 1008
Ile Thr Ala Ile Tyr Asp Cys Ala Thr Glu Ala Arg Lys His Gly Lys
                325                 330                 335
aca atc atc gcc gac ggt ggg att aaa ttc tct ggc gat atc act aaa 1056
Thr Ile Ile Ala Asp Gly Gly Ile Lys Phe Ser Gly Asp Ile Thr Lys
            340                 345                 350
gca ttg gca gcc ggc gga cat gct gtt atg ctc gga agc ttg ctt gca 1104
Ala Leu Ala Ala Gly Gly His Ala Val Met Leu Gly Ser Leu Leu Ala
        355                 360                 365
ggc aca tca gaa agc cct ggt gaa act gaa atc tac caa ggc aga aga 1152
Gly Thr Ser Glu Ser Pro Gly Glu Thr Glu Ile Tyr Gln Gly Arg Arg
    370                 375                 380
ttt aag gta tac cgc ggc atg gga tca gtt gct gca atg gaa aaa gga 1200
Phe Lys Val Tyr Arg Gly Met Gly Ser Val Ala Ala Met Glu Lys Gly
385                 390                 395                 400
agt aaa gac cgt tac ttc caa gaa gaa aac aaa aaa ttt gtt cct gaa 1248
Ser Lys Asp Arg Tyr Phe Gln Glu Glu Asn Lys Lys Phe Val Pro Glu
                405                 410                 415
gga att gaa gga cgc aca cct tac aaa ggg cca gtt gaa gaa acc gtt 1296
Gly Ile Glu Gly Arg Thr Pro Tyr Lys Gly Pro Val Glu Glu Thr Val
            420                 425                 430
tat cag cta gtc gga ggc ctt cgt tct ggt atg ggg tat tgc ggg tcc 1344
Tyr Gln Leu Val Gly Gly Leu Arg Ser Gly Met Gly Tyr Cys Gly Ser
        435                 440                 445
aaa gat ctg cgt gcg cta aga gaa gaa gct cag ttc att cgc atg act 1392
Lys Asp Leu Arg Ala Leu Arg Glu Glu Ala Gln Phe Ile Arg Met Thr
    450                 455                 460

ggc gca gga ctt cgc gaa agc cat ccg cat gac gta cag att aca aaa 1440
Gly Ala Gly Leu Arg Glu Ser His Pro His Asp Val Gln Ile Thr Lys
465                 470                 475                 480
gaa tca cct aac tat aca att tca taa 1467
Glu Ser Pro Asn Tyr Thr Ile Ser
                485

<210> 12
<211> 488
<212> PRT
<213> Bacillus subtilis

<400> 12
Met Trp Glu Ser Lys Phe Ser Lys Glu Gly Leu Thr Phe Asp Asp Val
1               5                   10                  15
Leu Leu Val Pro Ala Lys Ser Glu Val Leu Pro Arg Asp Val Asp Leu
            20                  25                  30
Ser Val Glu Leu Thr Lys Thr Leu Lys Leu Asn Ile Pro Val Ile Ser
        35                  40                  45
Ala Gly Met Asp Thr Val Thr Glu Ser Ala Met Ala Ile Ala Met Ala
    50                  55                  60
Arg Gln Gly Gly Leu Gly Ile Ile His Lys Asn Met Ser Ile Glu Gln
65                  70                  75                  80
Gln Ala Glu Gln Val Asp Lys Val Lys Arg Ser Glu Arg Gly Val Ile
                85                  90                  95
Thr Asn Pro Phe Phe Leu Thr Pro Asp His Gln Val Phe Asp Ala Glu
            100                 105                 110
His Leu Met Gly Lys Tyr Arg Ile Ser Gly Val Pro Ile Val Asn Asn
        115                 120                 125
Glu Glu Asp Gln Lys Leu Val Gly Ile Ile Thr Asn Arg Asp Leu Arg
    130                 135                 140
Phe Ile Ser Asp Tyr Ser Met Lys Ile Ser Asp Val Met Thr Lys Glu
145                 150                 155                 160
Glu Leu Val Thr Ala Ser Val Gly Thr Thr Leu Asp Glu Ala Glu Lys
                165                 170                 175
Ile Leu Gln Lys His Lys Ile Glu Lys Leu Pro Leu Val Asp Asp Gln
            180                 185                 190
Asn Lys Leu Lys Gly Leu Ile Thr Ile Lys Asp Ile Glu Lys Val Ile
        195                 200                 205
Glu Phe Pro Asn Ser Ser Lys Asp Ile His Gly Arg Leu Ile Val Gly
    210                 215                 220
Ala Ala Val Gly Val Thr Gly Asp Thr Met Thr Arg Val Lys Lys Leu
225                 230                 235                 240
Val Glu Ala Asn Val Asp Val Ile Val Ile Asp Thr Ala His Gly His
                245                 250                 255
Ser Gln Gly Val Leu Asn Thr Val Thr Lys Ile Arg Glu Thr Tyr Pro
            260                 265                 270
Glu Leu Asn Ile Ile Ala Gly Asn Val Ala Thr Ala Glu Ala Thr Arg
        275                 280                 285
Ala Leu Ile Glu Ala Gly Ala Asp Val Val Lys Val Gly Ile Gly Pro
    290                 295                 300
Gly Ser Ile Cys Thr Thr Arg Val Val Ala Gly Val Gly Val Pro Gln
305                 310                 315                 320
Ile Thr Ala Ile Tyr Asp Cys Ala Thr Glu Ala Arg Lys His Gly Lys
                325                 330                 335
Thr Ile Ile Ala Asp Gly Gly Ile Lys Phe Ser Gly Asp Ile Thr Lys
            340                 345                 350
Ala Leu Ala Ala Gly Gly His Ala Val Met Leu Gly Ser Leu Leu Ala
        355                 360                 365
Gly Thr Ser Glu Ser Pro Gly Glu Thr Glu Ile Tyr Gln Gly Arg Arg
    370                 375                 380
Phe Lys Val Tyr Arg Gly Met Gly Ser Val Ala Ala Met Glu Lys Gly
385                 390                 395                 400
Ser Lys Asp Arg Tyr Phe Gln Glu Glu Asn Lys Lys Phe Val Pro Glu
                405                 410                 415
Gly Ile Glu Gly Arg Thr Pro Tyr Lys Gly Pro Val Glu Glu Thr Val
            420                 425                 430
Tyr Gln Leu Val Gly Gly Leu Arg Ser Gly Met Gly Tyr Cys Gly Ser

        435                 440                 445
Lys Asp Leu Arg Ala Leu Arg Glu Glu Ala Gln Phe Ile Arg Met Thr
    450                 455                 460
Gly Ala Gly Leu Arg Glu Ser His Pro His Asp Val Gln Ile Thr Lys
465                 470                 475                 480
Glu Ser Pro Asn Tyr Thr Ile Ser
                485

<210> 13
<211> 1521
<212> DNA
<213> Corynebacterium glutamicum

<220>
<221> CDS
<222> (1)..(1518)

<400> 13
atg aca acc cag agc cga gtt tct acc gga gga gac aac cca aac aag 48
Met Thr Thr Gln Ser Arg Val Ser Thr Gly Gly Asp Asn Pro Asn Lys
1               5                   10                  15
gtt gcc ctt gtt gga tta acc ttt gat gac gta ctt ttg ctt cca gat 96
Val Ala Leu Val Gly Leu Thr Phe Asp Asp Val Leu Leu Leu Pro Asp
            20                  25                  30
gcg tcg gac gtt gtt cct tca gag gta gat acc tcg acg cag tta aca 144
Ala Ser Asp Val Val Pro Ser Glu Val Asp Thr Ser Thr Gln Leu Thr
        35                  40                  45
cgt aat att cgc ctt aac acc cct att ctt tct gcc gca atg gat act 192
Arg Asn Ile Arg Leu Asn Thr Pro Ile Leu Ser Ala Ala Met Asp Thr
    50                  55                  60
gtc acc gag gct cgc atg gct atc ggc atg gca cgc cat ggc ggc att 240
Val Thr Glu Ala Arg Met Ala Ile Gly Met Ala Arg His Gly Gly Ile
65                  70                  75                  80
ggt gtt ttg cac cgc aac ctg tct att caa gag cag gca gaa aac gtt 288
Gly Val Leu His Arg Asn Leu Ser Ile Gln Glu Gln Ala Glu Asn Val
                85                  90                  95
gag ctg gtg aag cgt tct gag tct gga atg gtc act gat cct gtt acc 336
Glu Leu Val Lys Arg Ser Glu Ser Gly Met Val Thr Asp Pro Val Thr
            100                 105                 110
tgt act cct gac atg agc atc caa gaa gtg gat gat ctg tgt gca cgc 384
Cys Thr Pro Asp Met Ser Ile Gln Glu Val Asp Asp Leu Cys Ala Arg
        115                 120                 125
ttc cgc att tcc ggt ctg cct gtt gtt gat gag gcc gga aag ttg gtt 432
Phe Arg Ile Ser Gly Leu Pro Val Val Asp Glu Ala Gly Lys Leu Val
    130                 135                 140
ggt att tgc acc aac cgc gat atg cgt ttt gaa agc gac atg aac cgt 480
Gly Ile Cys Thr Asn Arg Asp Met Arg Phe Glu Ser Asp Met Asn Arg
145                 150                 155                 160
cgt gtc gct gaa gtt atg acc cca atg cct ttg gtt gtt gct gaa gag 528
Arg Val Ala Glu Val Met Thr Pro Met Pro Leu Val Val Ala Glu Glu
                165                 170                 175
ggc gtc acc aag gag cag gct ctt gct ttg ctg tct gca aac aag gtg 576
Gly Val Thr Lys Glu Gln Ala Leu Ala Leu Leu Ser Ala Asn Lys Val
            180                 185                 190
gag aag ctt cct atc atc gca aag gac ggc aag ctt gtc ggt ctg atc 624
Glu Lys Leu Pro Ile Ile Ala Lys Asp Gly Lys Leu Val Gly Leu Ile
        195                 200                 205
acg gtg aag gac ttc gtt aag act gag cag cac ccg aac gca tcc aag 672
Thr Val Lys Asp Phe Val Lys Thr Glu Gln His Pro Asn Ala Ser Lys
    210                 215                 220
gat gca tca ggt cgt ctg ctg gtt gcg gct ggc atc ggc acg ggc gag 720
Asp Ala Ser Gly Arg Leu Leu Val Ala Ala Gly Ile Gly Thr Gly Glu
225                 230                 235                 240
gag tca ttc cag cga gct ggt gcg ctt gcc gac gcc ggc gtc gac att 768
Glu Ser Phe Gln Arg Ala Gly Ala Leu Ala Asp Ala Gly Val Asp Ile
                245                 250                 255
ttg gtc gta gac tct gca cac gcc cat agc cgt gga gtt ttg gac atg 816
Leu Val Val Asp Ser Ala His Ala His Ser Arg Gly Val Leu Asp Met

            260                 265                 270
gtg tcc cgc gtg aag aag tcg ttc ccc aag gtc gat atc gtt ggc ggc 864
Val Ser Arg Val Lys Lys Ser Phe Pro Lys Val Asp Ile Val Gly Gly
        275                 280                 285
aac ttg gcg acc cgc gag gct gcg cag gcc atg att gaa gct ggc gca 912
Asn Leu Ala Thr Arg Glu Ala Ala Gln Ala Met Ile Glu Ala Gly Ala
    290                 295                 300
gac gct atc aag gtg ggt att ggc cca ggt tct att tgc acc act cgc 960
Asp Ala Ile Lys Val Gly Ile Gly Pro Gly Ser Ile Cys Thr Thr Arg
305                 310                 315                 320
gtt gtc gca ggt gtc ggt gca cct cag atc act gcg atc atg gag gca 1008
Val Val Ala Gly Val Gly Ala Pro Gln Ile Thr Ala Ile Met Glu Ala
                325                 330                 335
gct gtt cca gct cac aag gct ggc gtt cct atc atc gcc gat ggc ggc 1056
Ala Val Pro Ala His Lys Ala Gly Val Pro Ile Ile Ala Asp Gly Gly
            340                 345                 350
atg cag ttc tct ggt gat atc gct aag gct ttg gct gct ggc gct aac 1104
Met Gln Phe Ser Gly Asp Ile Ala Lys Ala Leu Ala Ala Gly Ala Asn
        355                 360                 365
tcc gtg atg ctg ggc tcc atg ctg gct ggt acc gct gag gct cct ggt 1152
Ser Val Met Leu Gly Ser Met Leu Ala Gly Thr Ala Glu Ala Pro Gly
    370                 375                 380
gag acc atc acc atc aac ggc aag cag tac aag cgt tac cgc ggc atg 1200
Glu Thr Ile Thr Ile Asn Gly Lys Gln Tyr Lys Arg Tyr Arg Gly Met
385                 390                 395                 400
ggc tcc atg ggc gct atg cag ggc cgt gga ctt agt ggt gag aag cgt 1248
Gly Ser Met Gly Ala Met Gln Gly Arg Gly Leu Ser Gly Glu Lys Arg
                405                 410                 415
tcc tac tcc aag gac cgt tac ttc cag tct gac gtt aag agc gaa gac 1296
Ser Tyr Ser Lys Asp Arg Tyr Phe Gln Ser Asp Val Lys Ser Glu Asp
            420                 425                 430
aag ctc gtt cca gaa ggc atc gaa ggt cgc gtg cct ttc cgc ggt ccc 1344
Lys Leu Val Pro Glu Gly Ile Glu Gly Arg Val Pro Phe Arg Gly Pro
        435                 440                 445
atc gga gac atc att cac cag cag gtc ggt gga ctt cgt gca gca atg 1392
Ile Gly Asp Ile Ile His Gln Gln Val Gly Gly Leu Arg Ala Ala Met
    450                 455                 460
ggc tac acc ggt tcc tcc acc att gaa gag ctg cac aac gct cgt ttc 1440
Gly Tyr Thr Gly Ser Ser Thr Ile Glu Glu Leu His Asn Ala Arg Phe
465                 470                 475                 480
gtg cag atc acc agc gcg ggt ctg aag gaa tcc cac ccg cac cac atc 1488
Val Gln Ile Thr Ser Ala Gly Leu Lys Glu Ser His Pro His His Ile
                485                 490                 495
cag cag act gtg gaa gct cct aac tac cac tag 1521
Gln Gln Thr Val Glu Ala Pro Asn Tyr His
            500                 505

<210> 14
<211> 506
<212> PRT
<213> Corynebacterium glutamicum

<400> 14
Met Thr Thr Gln Ser Arg Val Ser Thr Gly Gly Asp Asn Pro Asn Lys
1               5                   10                  15
Val Ala Leu Val Gly Leu Thr Phe Asp Asp Val Leu Leu Leu Pro Asp
            20                  25                  30
Ala Ser Asp Val Val Pro Ser Glu Val Asp Thr Ser Thr Gln Leu Thr
        35                  40                  45
Arg Asn Ile Arg Leu Asn Thr Pro Ile Leu Ser Ala Ala Met Asp Thr
    50                  55                  60
Val Thr Glu Ala Arg Met Ala Ile Gly Met Ala Arg His Gly Gly Ile
65                  70                  75                  80
Gly Val Leu His Arg Asn Leu Ser Ile Gln Glu Gln Ala Glu Asn Val
                85                  90                  95
Glu Leu Val Lys Arg Ser Glu Ser Gly Met Val Thr Asp Pro Val Thr
            100                 105                 110
Cys Thr Pro Asp Met Ser Ile Gln Glu Val Asp Asp Leu Cys Ala Arg
        115                 120                 125

Phe Arg Ile Ser Gly Leu Pro Val Val Asp Glu Ala Gly Lys Leu Val
    130                 135                 140
Gly Ile Cys Thr Asn Arg Asp Met Arg Phe Glu Ser Asp Met Asn Arg
145                 150                 155                 160
Arg Val Ala Glu Val Met Thr Pro Met Pro Leu Val Val Ala Glu Glu
                165                 170                 175
Gly Val Thr Lys Glu Gln Ala Leu Ala Leu Leu Ser Ala Asn Lys Val
            180                 185                 190
Glu Lys Leu Pro Ile Ile Ala Lys Asp Gly Lys Leu Val Gly Leu Ile
        195                 200                 205
Thr Val Lys Asp Phe Val Lys Thr Glu Gln His Pro Asn Ala Ser Lys
    210                 215                 220
Asp Ala Ser Gly Arg Leu Leu Val Ala Ala Gly Ile Gly Thr Gly Glu
225                 230                 235                 240
Glu Ser Phe Gln Arg Ala Gly Ala Leu Ala Asp Ala Gly Val Asp Ile
                245                 250                 255
Leu Val Val Asp Ser Ala His Ala His Ser Arg Gly Val Leu Asp Met
            260                 265                 270
Val Ser Arg Val Lys Lys Ser Phe Pro Lys Val Asp Ile Val Gly Gly
        275                 280                 285
Asn Leu Ala Thr Arg Glu Ala Ala Gln Ala Met Ile Glu Ala Gly Ala
    290                 295                 300
Asp Ala Ile Lys Val Gly Ile Gly Pro Gly Ser Ile Cys Thr Thr Arg
305                 310                 315                 320
Val Val Ala Gly Val Gly Ala Pro Gln Ile Thr Ala Ile Met Glu Ala
                325                 330                 335
Ala Val Pro Ala His Lys Ala Gly Val Pro Ile Ile Ala Asp Gly Gly
            340                 345                 350
Met Gln Phe Ser Gly Asp Ile Ala Lys Ala Leu Ala Ala Gly Ala Asn
        355                 360                 365
Ser Val Met Leu Gly Ser Met Leu Ala Gly Thr Ala Glu Ala Pro Gly
    370                 375                 380
Glu Thr Ile Thr Ile Asn Gly Lys Gln Tyr Lys Arg Tyr Arg Gly Met
385                 390                 395                 400
Gly Ser Met Gly Ala Met Gln Gly Arg Gly Leu Ser Gly Glu Lys Arg
                405                 410                 415
Ser Tyr Ser Lys Asp Arg Tyr Phe Gln Ser Asp Val Lys Ser Glu Asp
            420                 425                 430
Lys Leu Val Pro Glu Gly Ile Glu Gly Arg Val Pro Phe Arg Gly Pro
        435                 440                 445
Ile Gly Asp Ile Ile His Gln Gln Val Gly Gly Leu Arg Ala Ala Met
    450                 455                 460
Gly Tyr Thr Gly Ser Ser Thr Ile Glu Glu Leu His Asn Ala Arg Phe
465                 470                 475                 480
Val Gln Ile Thr Ser Ala Gly Leu Lys Glu Ser His Pro His His Ile
                485                 490                 495
Gln Gln Thr Val Glu Ala Pro Asn Tyr His
            500                 505

<210>  15
<211>  1542
<212>  DNA
<213>  Corynebacterium ammoniagenes

<220>
<221>  CDS
<222>  (1)..(1539)

<400>  15
gtg cga agg aga aga ccc gta atg acc gaa aat cgt gtt tct acc ggt      48
Val Arg Arg Arg Arg Pro Val Met Thr Glu Asn Arg Val Ser Thr Gly
1               5                   10                  15
gga gat gac cca aat aag gtt gca ttg cat ggc ttg acg ttt gat gac      96
Gly Asp Asp Pro Asn Lys Val Ala Leu His Gly Leu Thr Phe Asp Asp
            20                  25                  30
gtg ctg ctg cta cct gcc gaa tcc aat gtt gtt ccg tcg gaa gta gac     144
Val Leu Leu Leu Pro Ala Glu Ser Asn Val Val Pro Ser Glu Val Asp

35                  40                  45
act tcg gcg cag ttc acc cgc aat act cgt tta ggt att cct ttg gca      192
Thr Ser Ala Gln Phe Thr Arg Asn Thr Arg Leu Gly Ile Pro Leu Ala
    50                  55                  60
tcg gct gcg atg gac acg gtt act gag gcg cgc atg gct att gcc atg      240
Ser Ala Ala Met Asp Thr Val Thr Glu Ala Arg Met Ala Ile Ala Met
65                  70                  75                  80
gca cgc cag ggt ggc att ggt gtc ttg cac cgc aac ttg tcc tcg caa      288
Ala Arg Gln Gly Gly Ile Gly Val Leu His Arg Asn Leu Ser Ser Gln
                85                  90                  95
gag cag gcg gag cag gtc gaa atc gtc aag cgc tct gag tcc ggc atg      336
Glu Gln Ala Glu Gln Val Glu Ile Val Lys Arg Ser Glu Ser Gly Met
            100                 105                 110
gtc acc gac cct gtg acc gcg aat cca gac atg act atc cag gaa gtt      384
Val Thr Asp Pro Val Thr Ala Asn Pro Asp Met Thr Ile Gln Glu Val
        115                 120                 125
gat gac ctg tgt gca cgc ttc cgc atc tct ggt ctt cct gtg gtc aac      432
Asp Asp Leu Cys Ala Arg Phe Arg Ile Ser Gly Leu Pro Val Val Asn
    130                 135                 140
gaa gac ggc acc ttg ttg ggc att tgc acc aac cgc gat atg cgc ttt      480
Glu Asp Gly Thr Leu Leu Gly Ile Cys Thr Asn Arg Asp Met Arg Phe
145                 150                 155                 160
gag cgc gac tat tcc cgc aag gtt tct gac atc atg acc gct atg ccg      528
Glu Arg Asp Tyr Ser Arg Lys Val Ser Asp Ile Met Thr Ala Met Pro
                165                 170                 175
ctg gtt gtg gca aaa gaa ggc gtc agc aag gaa gaa gcc ctg gat ctg      576
Leu Val Val Ala Lys Glu Gly Val Ser Lys Glu Glu Ala Leu Asp Leu
            180                 185                 190
ctg tcg acg aac aag gta gaa aag cta cct atc gtt gat aaa aac aac      624
Leu Ser Thr Asn Lys Val Glu Lys Leu Pro Ile Val Asp Lys Asn Asn
        195                 200                 205
aag ctg gtc ggt ctg att acc gtt aaa gac ttt gtt aag acc gaa cag      672
Lys Leu Val Gly Leu Ile Thr Val Lys Asp Phe Val Lys Thr Glu Gln
    210                 215                 220
ttc ccg aat tcc tcc aag gat gct tcg ggc cgc ttg cta gta gca gca      720
Phe Pro Asn Ser Ser Lys Asp Ala Ser Gly Arg Leu Leu Val Ala Ala
225                 230                 235                 240
ggt att ggt acc ggc gag gag tct tat gag cgt gca ggc ttg ctt gtc      768
Gly Ile Gly Thr Gly Glu Glu Ser Tyr Glu Arg Ala Gly Leu Leu Val
                245                 250                 255
gat gcc ggc gtg gac gtt ctc att gtc gac tcc gca cac gcg cac aat      816
Asp Ala Gly Val Asp Val Leu Ile Val Asp Ser Ala His Ala His Asn
            260                 265                 270
aac cgc gtg ctg gaa atg gtc tcg cgc gtc aag aat gac ttc ggc tcc      864
Asn Arg Val Leu Glu Met Val Ser Arg Val Lys Asn Asp Phe Gly Ser
        275                 280                 285
aag att gat gtt gtc ggc ggc aac ctg gca aca cgc tcg gca gca aag      912
Lys Ile Asp Val Val Gly Gly Asn Leu Ala Thr Arg Ser Ala Ala Lys
    290                 295                 300
gcg atg att gag gct ggc gca gac gcc atc aag gtg ggt att ggt cct      960
Ala Met Ile Glu Ala Gly Ala Asp Ala Ile Lys Val Gly Ile Gly Pro
305                 310                 315                 320
ggt tct atc tgc acc acc cgt gtg gtt gct ggt gtt ggt gca cca cag      1008
Gly Ser Ile Cys Thr Thr Arg Val Val Ala Gly Val Gly Ala Pro Gln
                325                 330                 335
att acc gcg atc atg gaa gca gct acc gtg gct tct gct gcg ggc gtg      1056
Ile Thr Ala Ile Met Glu Ala Ala Thr Val Ala Ser Ala Ala Gly Val
            340                 345                 350
cct ttg att gca gac ggc ggc atg cag tac tcc ggt gac gtt gct aag      1104
Pro Leu Ile Ala Asp Gly Gly Met Gln Tyr Ser Gly Asp Val Ala Lys
        355                 360                 365
gct ttg gct gct ggc gcg gac tcg gtc atg ctg ggc tcg atg ttc gca      1152
Ala Leu Ala Ala Gly Ala Asp Ser Val Met Leu Gly Ser Met Phe Ala
    370                 375                 380
ggc acc ctg gag gct cct ggt gac atc gtg att gtc ggc ggc aag cag      1200
Gly Thr Leu Glu Ala Pro Gly Asp Ile Val Ile Val Gly Gly Lys Gln
385                 390                 395                 400
tac aag cgc tac cgc ggc atg ggt tcg atg ggc gct atg caa ggc cgt      1248
Tyr Lys Arg Tyr Arg Gly Met Gly Ser Met Gly Ala Met Gln Gly Arg

                405                 410                 415
ggc ctc tcc ggc gag aag cgt tct tac tcc aag gac cgc tac ttc cag 1296
Gly Leu Ser Gly Glu Lys Arg Ser Tyr Ser Lys Asp Arg Tyr Phe Gln
            420                 425                 430
gca gat gtg cgc agc gaa gat aag ctg gtt cca gaa ggc gtg gaa ggc 1344
Ala Asp Val Arg Ser Glu Asp Lys Leu Val Pro Glu Gly Val Glu Gly
        435                 440                 445
aag gtt cct tac cgc ggc gaa att ggt cag att acc cac cag att gtg 1392
Lys Val Pro Tyr Arg Gly Glu Ile Gly Gln Ile Thr His Gln Ile Val
    450                 455                 460
ggc ggt ttg cgc gcg gca atg ggc tac act ggc tcc gct act att gaa 1440
Gly Gly Leu Arg Ala Ala Met Gly Tyr Thr Gly Ser Ala Thr Ile Glu
465                 470                 475                 480
gag ctg aag acc aag cag ttc gtg cgt att acc act gct ggc ttg gct 1488
Glu Leu Lys Thr Lys Gln Phe Val Arg Ile Thr Thr Ala Gly Leu Ala
                485                 490                 495
gag tcg cac ccg cac cac ctg cag caa act gta gaa gct ccg aac tac 1536
Glu Ser His Pro His His Leu Gln Gln Thr Val Glu Ala Pro Asn Tyr
            500                 505                 510
cgt taa 1542
Arg

<210> 16
<211> 513
<212> PRT
<213> Corynebacterium ammoniagenes

<400> 16
Val Arg Arg Arg Arg Pro Val Met Thr Glu Asn Arg Val Ser Thr Gly
1               5                   10                  15
Gly Asp Asp Pro Asn Lys Val Ala Leu His Gly Leu Thr Phe Asp Asp
            20                  25                  30
Val Leu Leu Leu Pro Ala Glu Ser Asn Val Val Pro Ser Glu Val Asp
        35                  40                  45
Thr Ser Ala Gln Phe Thr Arg Asn Thr Arg Leu Gly Ile Pro Leu Ala
    50                  55                  60
Ser Ala Ala Met Asp Thr Val Thr Glu Ala Arg Met Ala Ile Ala Met
65                  70                  75                  80
Ala Arg Gln Gly Gly Ile Gly Val Leu His Arg Asn Leu Ser Ser Gln
                85                  90                  95
Glu Gln Ala Glu Gln Val Glu Ile Val Lys Arg Ser Glu Ser Gly Met
            100                 105                 110
Val Thr Asp Pro Val Thr Ala Asn Pro Asp Met Thr Ile Gln Glu Val
        115                 120                 125
Asp Asp Leu Cys Ala Arg Phe Arg Ile Ser Gly Leu Pro Val Val Asn
    130                 135                 140
Glu Asp Gly Thr Leu Leu Gly Ile Cys Thr Asn Arg Asp Met Arg Phe
145                 150                 155                 160
Glu Arg Asp Tyr Ser Arg Lys Val Ser Asp Ile Met Thr Ala Met Pro
                165                 170                 175
Leu Val Val Ala Lys Glu Gly Val Ser Lys Glu Glu Ala Leu Asp Leu
            180                 185                 190
Leu Ser Thr Asn Lys Val Glu Lys Leu Pro Ile Val Asp Lys Asn Asn
        195                 200                 205
Lys Leu Val Gly Leu Ile Thr Val Lys Asp Phe Val Lys Thr Glu Gln
    210                 215                 220
Phe Pro Asn Ser Ser Lys Asp Ala Ser Gly Arg Leu Leu Val Ala Ala
225                 230                 235                 240
Gly Ile Gly Thr Gly Glu Glu Ser Tyr Glu Arg Ala Gly Leu Leu Val
                245                 250                 255
Asp Ala Gly Val Asp Val Leu Ile Val Asp Ser Ala His Ala His Asn
            260                 265                 270
Asn Arg Val Leu Glu Met Val Ser Arg Val Lys Asn Asp Phe Gly Ser
        275                 280                 285
Lys Ile Asp Val Val Gly Gly Asn Leu Ala Thr Arg Ser Ala Ala Lys
    290                 295                 300
Ala Met Ile Glu Ala Gly Ala Asp Ala Ile Lys Val Gly Ile Gly Pro

305                 310                 315                 320
Gly Ser Ile Cys Thr Thr Arg Val Val Ala Gly Val Gly Ala Pro Gln
                325                 330                 335
Ile Thr Ala Ile Met Glu Ala Ala Thr Val Ala Ser Ala Ala Gly Val
            340                 345                 350
Pro Leu Ile Ala Asp Gly Gly Met Gln Tyr Ser Gly Asp Val Ala Lys
        355                 360                 365
Ala Leu Ala Ala Gly Ala Asp Ser Val Met Leu Gly Ser Met Phe Ala
    370                 375                 380
Gly Thr Leu Glu Ala Pro Gly Asp Ile Val Ile Val Gly Gly Lys Gln
385                 390                 395                 400
Tyr Lys Arg Tyr Arg Gly Met Gly Ser Met Gly Ala Met Gln Gly Arg
                405                 410                 415
Gly Leu Ser Gly Glu Lys Arg Ser Tyr Ser Lys Asp Arg Tyr Phe Gln
            420                 425                 430
Ala Asp Val Arg Ser Glu Asp Lys Leu Val Pro Glu Gly Val Glu Gly
        435                 440                 445
Lys Val Pro Tyr Arg Gly Glu Ile Gly Gln Ile Thr His Gln Ile Val
    450                 455                 460
Gly Gly Leu Arg Ala Ala Met Gly Tyr Thr Gly Ser Ala Thr Ile Glu
465                 470                 475                 480
Glu Leu Lys Thr Lys Gln Phe Val Arg Ile Thr Thr Ala Gly Leu Ala
                485                 490                 495
Glu Ser His Pro His His Leu Gln Gln Thr Val Glu Ala Pro Asn Tyr
            500                 505                 510
Arg

<210>  17
<211>  1026
<212>  DNA
<213>  Escherichia coli

<220>
<221>  CDS
<222>  (1)..(1023)

<400>  17
atg gca aca ata aaa gat gta gcg aaa cga gca aac gtt tcc act aca      48
Met Ala Thr Ile Lys Asp Val Ala Lys Arg Ala Asn Val Ser Thr Thr
1               5                   10                  15
act gtg tca cac gtg atc aac aaa aca cgt ttc gtc gct gaa gaa acg      96
Thr Val Ser His Val Ile Asn Lys Thr Arg Phe Val Ala Glu Glu Thr
            20                  25                  30
cgc aac gcc gtg tgg gca gcg att aaa gaa tta cac tac tcc cct agc      144
Arg Asn Ala Val Trp Ala Ala Ile Lys Glu Leu His Tyr Ser Pro Ser
        35                  40                  45
gcg gtg gcg cgt agc ctg aag gtt aac cac acc aag tct atc ggt ttg      192
Ala Val Ala Arg Ser Leu Lys Val Asn His Thr Lys Ser Ile Gly Leu
    50                  55                  60
ctg gcg acc agc agc gaa gcg gcc tat ttt gcc gag atc att gaa gca      240
Leu Ala Thr Ser Ser Glu Ala Ala Tyr Phe Ala Glu Ile Ile Glu Ala
65                  70                  75                  80
gtt gaa aaa aat tgc ttc cag aaa ggt tac acc ctg att ctg ggc aat      288
Val Glu Lys Asn Cys Phe Gln Lys Gly Tyr Thr Leu Ile Leu Gly Asn
                85                  90                  95
gcg tgg aac aat ctt gag aaa cag cgg gct tat ctg tcg atg atg gcg      336
Ala Trp Asn Asn Leu Glu Lys Gln Arg Ala Tyr Leu Ser Met Met Ala
            100                 105                 110
caa aaa cgc gtc gat ggt ctg ctg gtg atg tgt tct gag tac cca gag      384
Gln Lys Arg Val Asp Gly Leu Leu Val Met Cys Ser Glu Tyr Pro Glu
        115                 120                 125
ccg ttg ctg gcg atg ctg gaa gag tat cgc cat atc cca atg gtg gtc      432
Pro Leu Leu Ala Met Leu Glu Glu Tyr Arg His Ile Pro Met Val Val
    130                 135                 140
atg gac tgg ggt gaa gca aaa gct gac ttc acc gat gcg gtc att gat      480
Met Asp Trp Gly Glu Ala Lys Ala Asp Phe Thr Asp Ala Val Ile Asp
145                 150                 155                 160

aac gcg ttc gaa ggc ggc tac atg gcc ggg cgt tat ctg att gaa cgc 528
Asn Ala Phe Glu Gly Gly Tyr Met Ala Gly Arg Tyr Leu Ile Glu Arg
                165                 170                 175
ggt cac cgc gaa atc ggc gtc atc ccc ggc ccg ctg gaa cgt aac acc 576
Gly His Arg Glu Ile Gly Val Ile Pro Gly Pro Leu Glu Arg Asn Thr
            180                 185                 190
ggc gca ggc cgc ctt gcc ggt ttt atg aag gcg atg gaa gaa gcg atg 624
Gly Ala Gly Arg Leu Ala Gly Phe Met Lys Ala Met Glu Glu Ala Met
        195                 200                 205
atc aag gtg ccg gaa agc tgg att gtg cag ggt gac ttt gaa cct gaa 672
Ile Lys Val Pro Glu Ser Trp Ile Val Gln Gly Asp Phe Glu Pro Glu
    210                 215                 220
tcc ggt tat cgc gcc atg cag caa atc ctg tcg cag ccg cat cgc cct 720
Ser Gly Tyr Arg Ala Met Gln Gln Ile Leu Ser Gln Pro His Arg Pro
225                 230                 235                 240
act gcc gtc ttc tgt ggt ggc gat atc atg gca atg ggc gca ctt tgt 768
Thr Ala Val Phe Cys Gly Gly Asp Ile Met Ala Met Gly Ala Leu Cys
                245                 250                 255
gct gct gat gaa atg ggc ctg cgc gtc ccg cag gat gtt tcg ctg atc 816
Ala Ala Asp Glu Met Gly Leu Arg Val Pro Gln Asp Val Ser Leu Ile
            260                 265                 270
ggt tat gat aac gtg cgc aac gcg cgc tat ttt acg ccg gcg ctg acc 864
Gly Tyr Asp Asn Val Arg Asn Ala Arg Tyr Phe Thr Pro Ala Leu Thr
        275                 280                 285
acg atc cat cag cca aaa gat tcg ctg ggt gaa aca gcg ttc aac atg 912
Thr Ile His Gln Pro Lys Asp Ser Leu Gly Glu Thr Ala Phe Asn Met
    290                 295                 300
ctg ttg gat cgt atc gtc aac aaa cgt gaa gaa ccg cag tct att gaa 960
Leu Leu Asp Arg Ile Val Asn Lys Arg Glu Glu Pro Gln Ser Ile Glu
305                 310                 315                 320
gtg cat ccg cgc ttg att gaa cgc cgc tcc gtg gct gac ggc ccg ttc 1008
Val His Pro Arg Leu Ile Glu Arg Arg Ser Val Ala Asp Gly Pro Phe
                325                 330                 335
cgc gac tat cgt cgt taa 1026
Arg Asp Tyr Arg Arg
            340

<210> 18
<211> 341
<212> PRT
<213> Escherichia coli

<400> 18
Met Ala Thr Ile Lys Asp Val Ala Lys Arg Ala Asn Val Ser Thr Thr
1               5                   10                  15
Thr Val Ser His Val Ile Asn Lys Thr Arg Phe Val Ala Glu Glu Thr
            20                  25                  30
Arg Asn Ala Val Trp Ala Ala Ile Lys Glu Leu His Tyr Ser Pro Ser
        35                  40                  45
Ala Val Ala Arg Ser Leu Lys Val Asn His Thr Lys Ser Ile Gly Leu
    50                  55                  60
Leu Ala Thr Ser Ser Glu Ala Ala Tyr Phe Ala Glu Ile Ile Glu Ala
65                  70                  75                  80
Val Glu Lys Asn Cys Phe Gln Lys Gly Tyr Thr Leu Ile Leu Gly Asn
                85                  90                  95
Ala Trp Asn Asn Leu Glu Lys Gln Arg Ala Tyr Leu Ser Met Met Ala
            100                 105                 110
Gln Lys Arg Val Asp Gly Leu Leu Val Met Cys Ser Glu Tyr Pro Glu
        115                 120                 125
Pro Leu Leu Ala Met Leu Glu Glu Tyr Arg His Ile Pro Met Val Val
    130                 135                 140
Met Asp Trp Gly Glu Ala Lys Ala Asp Phe Thr Asp Ala Val Ile Asp
145                 150                 155                 160
Asn Ala Phe Glu Gly Gly Tyr Met Ala Gly Arg Tyr Leu Ile Glu Arg
                165                 170                 175
Gly His Arg Glu Ile Gly Val Ile Pro Gly Pro Leu Glu Arg Asn Thr
            180                 185                 190
Gly Ala Gly Arg Leu Ala Gly Phe Met Lys Ala Met Glu Glu Ala Met

195                 200                 205
Ile Lys Val Pro Glu Ser Trp Ile Val Gln Gly Asp Phe Glu Pro Glu
    210                 215                 220
Ser Gly Tyr Arg Ala Met Gln Gln Ile Leu Ser Gln Pro His Arg Pro
225                 230                 235                 240
Thr Ala Val Phe Cys Gly Gly Asp Ile Met Ala Met Gly Ala Leu Cys
                245                 250                 255
Ala Ala Asp Glu Met Gly Leu Arg Val Pro Gln Asp Val Ser Leu Ile
            260                 265                 270
Gly Tyr Asp Asn Val Arg Asn Ala Arg Tyr Phe Thr Pro Ala Leu Thr
        275                 280                 285
Thr Ile His Gln Pro Lys Asp Ser Leu Gly Glu Thr Ala Phe Asn Met
    290                 295                 300
Leu Leu Asp Arg Ile Val Asn Lys Arg Glu Glu Pro Gln Ser Ile Glu
305                 310                 315                 320
Val His Pro Arg Leu Ile Glu Arg Arg Ser Val Ala Asp Gly Pro Phe
                325                 330                 335
Arg Asp Tyr Arg Arg
            340

<210>  19
<211>  25
<212>  DNA
<213>  Artificial sequence

<220>
<223>  primer1 for cloning purR

<400>  19
ttgccatttt gctaacaaac aggaa                                          25


<210>  20
<211>  25
<212>  DNA
<213>  Artificial sequence

<220>
<223>  primer2 for cloning purR

<400>  20
tttcaggaag tgttcaacgg tgtct                                          25


<210>  21
<211>  1653
<212>  DNA
<213>  Escherichia coli


<220>
<221>  CDS
<222>  (1)..(1650)

<400>  21
atg aaa tta ttg cag cgg ggc gtg gcg tta gcg ctg tta acc aca ttt      48
Met Lys Leu Leu Gln Arg Gly Val Ala Leu Ala Leu Leu Thr Thr Phe
1               5                   10                  15
aca ctg gcg agt gaa act gct ctg gcg tat gag cag gat aaa acc tac      96
Thr Leu Ala Ser Glu Thr Ala Leu Ala Tyr Glu Gln Asp Lys Thr Tyr
            20                  25                  30
aaa att aca gtt ctg cat acc aat gat cat cat ggg cat ttt tgg cgc     144
Lys Ile Thr Val Leu His Thr Asn Asp His His Gly His Phe Trp Arg
        35                  40                  45
aat gaa tat ggc gaa tat ggt ctg gcg gcg caa aaa acg ctg gtg gat     192
Asn Glu Tyr Gly Glu Tyr Gly Leu Ala Ala Gln Lys Thr Leu Val Asp
    50                  55                  60
ggt atc cgc aaa gag gtt gcg gct gaa ggc ggt agc gtg ctg cta ctt     240
Gly Ile Arg Lys Glu Val Ala Ala Glu Gly Gly Ser Val Leu Leu Leu

65                  70                  75                  80
tcc ggt ggc gac att aac act ggc gtg ccc gag tct gac tta cag gat      288
Ser Gly Gly Asp Ile Asn Thr Gly Val Pro Glu Ser Asp Leu Gln Asp
                85                  90                  95
gcc gaa cct gat ttt cgc ggt atg aat ctg gtg ggc tat gac gcg atg      336
Ala Glu Pro Asp Phe Arg Gly Met Asn Leu Val Gly Tyr Asp Ala Met
            100                 105                 110
gcg atc ggt aat cat gaa ttt gat aat ccg ctc acc gta tta cgc cag      384
Ala Ile Gly Asn His Glu Phe Asp Asn Pro Leu Thr Val Leu Arg Gln
        115                 120                 125
cag gaa aag tgg gcc aag ttc ccg ttg ctt tcc gcg aat atc tac cag      432
Gln Glu Lys Trp Ala Lys Phe Pro Leu Leu Ser Ala Asn Ile Tyr Gln
    130                 135                 140
aaa agt act ggc gag cgc ctg ttt aaa ccg tgg gcg ctg ttt aag cgt      480
Lys Ser Thr Gly Glu Arg Leu Phe Lys Pro Trp Ala Leu Phe Lys Arg
145                 150                 155                 160
cag gat ctg aaa att gcc gtt att ggg ctg aca acc gat gac aca gca      528
Gln Asp Leu Lys Ile Ala Val Ile Gly Leu Thr Thr Asp Asp Thr Ala
                165                 170                 175
aaa att ggt aac ccg gaa tac ttc act gat atc gaa ttt cgt aag ccc      576
Lys Ile Gly Asn Pro Glu Tyr Phe Thr Asp Ile Glu Phe Arg Lys Pro
            180                 185                 190
gcc gat gaa gcg aag ctg gtg att cag gag ctg caa cag aca gaa aag      624
Ala Asp Glu Ala Lys Leu Val Ile Gln Glu Leu Gln Gln Thr Glu Lys
        195                 200                 205
cca gac att att atc gcg gcg acc cat atg ggg cat tac gat aat ggt      672
Pro Asp Ile Ile Ile Ala Ala Thr His Met Gly His Tyr Asp Asn Gly
    210                 215                 220
gag cac ggc tct aac gca ccg ggc gat gtg gag atg gca cgc gcg ctg      720
Glu His Gly Ser Asn Ala Pro Gly Asp Val Glu Met Ala Arg Ala Leu
225                 230                 235                 240
cct gcc gga tcg ctg gcg atg atc gtc ggt ggt cac tcg caa gat ccg      768
Pro Ala Gly Ser Leu Ala Met Ile Val Gly Gly His Ser Gln Asp Pro
                245                 250                 255
gtc tgc atg gcg gca gaa aac aaa aaa cag gtc gat tac gtg ccg ggt      816
Val Cys Met Ala Ala Glu Asn Lys Lys Gln Val Asp Tyr Val Pro Gly
            260                 265                 270
acg cca tgc aaa cca gat caa caa aac ggc atc tgg att gtg cag gcg      864
Thr Pro Cys Lys Pro Asp Gln Gln Asn Gly Ile Trp Ile Val Gln Ala
        275                 280                 285
cat gag tgg ggc aaa tac gtg gga cgg gct gat ttt gag ttt cgt aat      912
His Glu Trp Gly Lys Tyr Val Gly Arg Ala Asp Phe Glu Phe Arg Asn
    290                 295                 300
ggc gaa atg aaa atg gtt aac tac cag ctg att ccg gtg aac ctg aag      960
Gly Glu Met Lys Met Val Asn Tyr Gln Leu Ile Pro Val Asn Leu Lys
305                 310                 315                 320
aag aaa gtg acc tgg gaa gac ggg aaa agc gag cgc gtg ctt tac act      1008
Lys Lys Val Thr Trp Glu Asp Gly Lys Ser Glu Arg Val Leu Tyr Thr
                325                 330                 335
cct gaa atc gct gaa aac cag caa atg atc tcg ctg tta tca ccg ttc      1056
Pro Glu Ile Ala Glu Asn Gln Gln Met Ile Ser Leu Leu Ser Pro Phe
            340                 345                 350
cag aac aaa ggc aaa gcg cag ctg gaa gtg aaa ata ggc gaa acc aat      1104
Gln Asn Lys Gly Lys Ala Gln Leu Glu Val Lys Ile Gly Glu Thr Asn
        355                 360                 365
ggt cgt ctg gaa ggc gat cgt gac aaa gtg cgt ttt gta cag acc aat      1152
Gly Arg Leu Glu Gly Asp Arg Asp Lys Val Arg Phe Val Gln Thr Asn
    370                 375                 380
atg ggg cgg ttg att ctg gca gcc caa atg gat cgc act ggt gcc gac      1200
Met Gly Arg Leu Ile Leu Ala Ala Gln Met Asp Arg Thr Gly Ala Asp
385                 390                 395                 400
ttt gcg gtg atg agc gga ggc gga att cgt gat tct atc gaa gca ggc      1248
Phe Ala Val Met Ser Gly Gly Gly Ile Arg Asp Ser Ile Glu Ala Gly
                405                 410                 415
gat atc agc tat aaa aac gtg ctg aaa gtg cag cca ttc ggc aat gtg      1296
Asp Ile Ser Tyr Lys Asn Val Leu Lys Val Gln Pro Phe Gly Asn Val
            420                 425                 430
gtg gtg tat gcc gac atg acc ggt aaa gag gtg att gat tac ctg acc      1344
Val Val Tyr Ala Asp Met Thr Gly Lys Glu Val Ile Asp Tyr Leu Thr

                435                     440                     445
gcc gtc gcg cag atg aag cca gat tca ggt gcc tac ccg caa ttt gcc        1392
Ala Val Ala Gln Met Lys Pro Asp Ser Gly Ala Tyr Pro Gln Phe Ala
    450                     455                     460
aac gtt agc ttt gtg gcg aaa gac ggc aaa ctg aac gac ctt aaa atc        1440
Asn Val Ser Phe Val Ala Lys Asp Gly Lys Leu Asn Asp Leu Lys Ile
465                     470                     475                     480
aaa ggc gaa ccg gtc gat ccg gcg aaa act tac cgt atg gcg aca tta        1488
Lys Gly Glu Pro Val Asp Pro Ala Lys Thr Tyr Arg Met Ala Thr Leu
                485                     490                     495
aac ttc aat gcc acc ggc ggt gat gga tat ccg cgc ctt gat aac aaa        1536
Asn Phe Asn Ala Thr Gly Gly Asp Gly Tyr Pro Arg Leu Asp Asn Lys
            500                     505                     510
ccg ggc tat gtg aat acc ggc ttt att gat gcc gaa gtg ctg aaa gcg        1584
Pro Gly Tyr Val Asn Thr Gly Phe Ile Asp Ala Glu Val Leu Lys Ala
        515                     520                     525
tat atc cag aaa agc tcg ccg ctg gat gtg agt gtt tat gaa ccg aaa        1632
Tyr Ile Gln Lys Ser Ser Pro Leu Asp Val Ser Val Tyr Glu Pro Lys
    530                     535                     540
ggt gag gtg agc tgg cag taa                                            1653
Gly Glu Val Ser Trp Gln
545                 550

<210>  22
<211>  550
<212>  PRT
<213>  Escherichia coli

<400>  22
Met Lys Leu Leu Gln Arg Gly Val Ala Leu Ala Leu Leu Thr Thr Phe
1               5                   10                  15
Thr Leu Ala Ser Glu Thr Ala Leu Ala Tyr Glu Gln Asp Lys Thr Tyr
            20                  25                  30
Lys Ile Thr Val Leu His Thr Asn Asp His His Gly His Phe Trp Arg
        35                  40                  45
Asn Glu Tyr Gly Glu Tyr Gly Leu Ala Ala Gln Lys Thr Leu Val Asp
    50                  55                  60
Gly Ile Arg Lys Glu Val Ala Ala Glu Gly Gly Ser Val Leu Leu Leu
65                  70                  75                  80
Ser Gly Gly Asp Ile Asn Thr Gly Val Pro Glu Ser Asp Leu Gln Asp
                85                  90                  95
Ala Glu Pro Asp Phe Arg Gly Met Asn Leu Val Gly Tyr Asp Ala Met
            100                 105                 110
Ala Ile Gly Asn His Glu Phe Asp Asn Pro Leu Thr Val Leu Arg Gln
        115                 120                 125
Gln Glu Lys Trp Ala Lys Phe Pro Leu Leu Ser Ala Asn Ile Tyr Gln
    130                 135                 140
Lys Ser Thr Gly Glu Arg Leu Phe Lys Pro Trp Ala Leu Phe Lys Arg
145                 150                 155                 160
Gln Asp Leu Lys Ile Ala Val Ile Gly Leu Thr Thr Asp Asp Thr Ala
                165                 170                 175
Lys Ile Gly Asn Pro Glu Tyr Phe Thr Asp Ile Glu Phe Arg Lys Pro
            180                 185                 190
Ala Asp Glu Ala Lys Leu Val Ile Gln Glu Leu Gln Gln Thr Glu Lys
        195                 200                 205
Pro Asp Ile Ile Ile Ala Ala Thr His Met Gly His Tyr Asp Asn Gly
    210                 215                 220
Glu His Gly Ser Asn Ala Pro Gly Asp Val Glu Met Ala Arg Ala Leu
225                 230                 235                 240
Pro Ala Gly Ser Leu Ala Met Ile Val Gly Gly His Ser Gln Asp Pro
                245                 250                 255
Val Cys Met Ala Ala Glu Asn Lys Lys Gln Val Asp Tyr Val Pro Gly
            260                 265                 270
Thr Pro Cys Lys Pro Asp Gln Gln Asn Gly Ile Trp Ile Val Gln Ala
        275                 280                 285
His Glu Trp Gly Lys Tyr Val Gly Arg Ala Asp Phe Glu Phe Arg Asn
    290                 295                 300
Gly Glu Met Lys Met Val Asn Tyr Gln Leu Ile Pro Val Asn Leu Lys

305                 310                 315                 320
Lys Lys Val Thr Trp Glu Asp Gly Lys Ser Glu Arg Val Leu Tyr Thr
                325                 330                 335
Pro Glu Ile Ala Glu Asn Gln Gln Met Ile Ser Leu Leu Ser Pro Phe
            340                 345                 350
Gln Asn Lys Gly Lys Ala Gln Leu Glu Val Lys Ile Gly Glu Thr Asn
        355                 360                 365
Gly Arg Leu Glu Gly Asp Arg Asp Lys Val Arg Phe Val Gln Thr Asn
    370                 375                 380
Met Gly Arg Leu Ile Leu Ala Ala Gln Met Asp Arg Thr Gly Ala Asp
385                 390                 395                 400
Phe Ala Val Met Ser Gly Gly Gly Ile Arg Asp Ser Ile Glu Ala Gly
                405                 410                 415
Asp Ile Ser Tyr Lys Asn Val Leu Lys Val Gln Pro Phe Gly Asn Val
            420                 425                 430
Val Val Tyr Ala Asp Met Thr Gly Lys Glu Val Ile Asp Tyr Leu Thr
        435                 440                 445
Ala Val Ala Gln Met Lys Pro Asp Ser Gly Ala Tyr Pro Gln Phe Ala
    450                 455                 460
Asn Val Ser Phe Val Ala Lys Asp Gly Lys Leu Asn Asp Leu Lys Ile
465                 470                 475                 480
Lys Gly Glu Pro Val Asp Pro Ala Lys Thr Tyr Arg Met Ala Thr Leu
                485                 490                 495
Asn Phe Asn Ala Thr Gly Gly Asp Gly Tyr Pro Arg Leu Asp Asn Lys
            500                 505                 510
Pro Gly Tyr Val Asn Thr Gly Phe Ile Asp Ala Glu Val Leu Lys Ala
        515                 520                 525
Tyr Ile Gln Lys Ser Ser Pro Leu Asp Val Ser Val Tyr Glu Pro Lys
    530                 535                 540
Gly Glu Val Ser Trp Gln
545                 550


<210>  23
<211>  714
<212>  DNA
<213>  Escherichia coli


<220>
<221>  CDS
<222>  (1)..(711)

<400>  23
atg cgc aag atc aca cag gca atc agt gcc gtt tgc tta ttg ttc gct      48
Met Arg Lys Ile Thr Gln Ala Ile Ser Ala Val Cys Leu Leu Phe Ala
1               5                   10                  15
cta aac agt tcc gct gtt gcc ctg gcc tca tct cct tca ccg ctt aac      96
Leu Asn Ser Ser Ala Val Ala Leu Ala Ser Ser Pro Ser Pro Leu Asn
            20                  25                  30
cct ggg act aac gtt gcc agg ctt gct gaa cag gca ccc att cat tgg      144
Pro Gly Thr Asn Val Ala Arg Leu Ala Glu Gln Ala Pro Ile His Trp
        35                  40                  45
gtt tcg gtc gca caa att gaa aat agc ctc gca ggg cgt ccg cca atg      192
Val Ser Val Ala Gln Ile Glu Asn Ser Leu Ala Gly Arg Pro Pro Met
    50                  55                  60
gcg gtg ggg ttt gat atc gat gac acg gta ctt ttt tcc agt ccg ggc      240
Ala Val Gly Phe Asp Ile Asp Asp Thr Val Leu Phe Ser Ser Pro Gly
65                  70                  75                  80
ttc tgg cgc ggc aaa aaa acc ttc tcg cca gaa agc gaa gat tat ctg      288
Phe Trp Arg Gly Lys Lys Thr Phe Ser Pro Glu Ser Glu Asp Tyr Leu
                85                  90                  95
aaa aat cct gtg ttc tgg gaa aaa atg aac aat ggc tgg gat gaa ttc      336
Lys Asn Pro Val Phe Trp Glu Lys Met Asn Asn Gly Trp Asp Glu Phe
            100                 105                 110
agc att cca aaa gag gtc gct cgc cag ctg att gat atg cat gta cgc      384
Ser Ile Pro Lys Glu Val Ala Arg Gln Leu Ile Asp Met His Val Arg
        115                 120                 125
cgc ggt gac gcg atc ttc ttt gtg act ggt cgt agc ccg acg aaa aca      432

Arg Gly Asp Ala Ile Phe Phe Val Thr Gly Arg Ser Pro Thr Lys Thr
    130                 135                 140
gaa acg gtt tca aaa acg ctg gcg gat aat ttt cat att cct gcc acc      480
Glu Thr Val Ser Lys Thr Leu Ala Asp Asn Phe His Ile Pro Ala Thr
145                 150                 155                 160
aac atg aat ccg gtg atc ttt gcg ggc gat aaa cca ggg caa aat aca      528
Asn Met Asn Pro Val Ile Phe Ala Gly Asp Lys Pro Gly Gln Asn Thr
                165                 170                 175
aaa tcg caa tgg ctg cag gat aaa aat atc cga att ttt tat ggc gat      576
Lys Ser Gln Trp Leu Gln Asp Lys Asn Ile Arg Ile Phe Tyr Gly Asp
            180                 185                 190
tct gat aat gat att acc gcc gca cgc gat gtc ggc gct cgt ggt atc      624
Ser Asp Asn Asp Ile Thr Ala Ala Arg Asp Val Gly Ala Arg Gly Ile
        195                 200                 205
cgc att ctg cgc gcc tcc aac tct acc tac aaa ccc ttg cca caa gcg      672
Arg Ile Leu Arg Ala Ser Asn Ser Thr Tyr Lys Pro Leu Pro Gln Ala
    210                 215                 220
ggt gcg ttt ggt gaa gag gtg atc gtc aat tca gaa tac tga              714
Gly Ala Phe Gly Glu Glu Val Ile Val Asn Ser Glu Tyr
225                 230                 235

<210>  24
<211>  237
<212>  PRT
<213>  Escherichia coli

<400>  24
Met Arg Lys Ile Thr Gln Ala Ile Ser Ala Val Cys Leu Leu Phe Ala
1               5                   10                  15
Leu Asn Ser Ser Ala Val Ala Leu Ala Ser Ser Pro Ser Pro Leu Asn
            20                  25                  30
Pro Gly Thr Asn Val Ala Arg Leu Ala Glu Gln Ala Pro Ile His Trp
        35                  40                  45
Val Ser Val Ala Gln Ile Glu Asn Ser Leu Ala Gly Arg Pro Pro Met
    50                  55                  60
Ala Val Gly Phe Asp Ile Asp Asp Thr Val Leu Phe Ser Ser Pro Gly
65                  70                  75                  80
Phe Trp Arg Gly Lys Lys Thr Phe Ser Pro Glu Ser Glu Asp Tyr Leu
                85                  90                  95
Lys Asn Pro Val Phe Trp Glu Lys Met Asn Asn Gly Trp Asp Glu Phe
            100                 105                 110
Ser Ile Pro Lys Glu Val Ala Arg Gln Leu Ile Asp Met His Val Arg
        115                 120                 125
Arg Gly Asp Ala Ile Phe Phe Val Thr Gly Arg Ser Pro Thr Lys Thr
    130                 135                 140
Glu Thr Val Ser Lys Thr Leu Ala Asp Asn Phe His Ile Pro Ala Thr
145                 150                 155                 160
Asn Met Asn Pro Val Ile Phe Ala Gly Asp Lys Pro Gly Gln Asn Thr
                165                 170                 175
Lys Ser Gln Trp Leu Gln Asp Lys Asn Ile Arg Ile Phe Tyr Gly Asp
            180                 185                 190
Ser Asp Asn Asp Ile Thr Ala Ala Arg Asp Val Gly Ala Arg Gly Ile
        195                 200                 205
Arg Ile Leu Arg Ala Ser Asn Ser Thr Tyr Lys Pro Leu Pro Gln Ala
    210                 215                 220
Gly Ala Phe Gly Glu Glu Val Ile Val Asn Ser Glu Tyr
225                 230                 235

<210>  25
<211>  54
<212>  DNA
<213>  Artificial sequence

<220>
<223>  primer1 for disrupting ushA

<400>  25
ggagagaagt atgaaattat tgcagcgggg cgtggccgct caagttagta taaa          54

<210> 26
<211> 54
<212> DNA
<213> Artificial sequence

<220>
<223> primer2 for disrupting ushA

<400> 26
ccagctcacc tcacctttcg gttcataaac actcactgaa gcctgctttt ttat 54


<210> 27
<211> 54
<212> DNA
<213> Artificial sequence

<220>
<223> primer1 for disrupting aphA

<400> 27
agggaaaaat atgcgcaaga tcacacaggc aatcagcgct caagttagta taaa 54


<210> 28
<211> 54
<212> DNA
<213> Artificial sequence

<220>
<223> primer2 for disrupting aphA

<400> 28
ttctgaattg acgatcacct cttcaccaaa cgcacctgaa gcctgctttt ttat 54


<210> 29
<211> 54
<212> DNA
<213> Artificial sequence

<220>
<223> primer1 for disrupting purR

<400> 29
gtgaaatgga atggcaacaa taaaagatgt agcgaacgct caagttagta taaa 54


<210> 30
<211> 54
<212> DNA
<213> Artificial sequence

<220>
<223> primer2 for disrupting purR

<400> 30
acgatagtcg cggaacgggc cgtcagccac ggagcgtgaa gcctgctttt ttat 54


<210> 31
<211> 29
<212> DNA
<213> Artificial sequence

<220>
<223> primer1 for cloning guaBA

<400>  31
cgcgagctct attcagtcga tagtaaccc                                          29


<210>  32
<211>  29
<212>  DNA
<213>  Artificial sequence

<220>
<223>  primer2 for cloning guaBA

<400>  32
cgcggtacct caatcctata attcttgaa                                          29


<210>  33
<211>  1299
<212>  DNA
<213>  Escherichia coli


<220>
<221>  CDS
<222>  (1)..(1296)

<400>  33
atg ggt aac aac gtc gtc gta ctg ggc acc caa tgg ggt gac gaa ggt       48
Met Gly Asn Asn Val Val Val Leu Gly Thr Gln Trp Gly Asp Glu Gly
1               5                   10                  15
aaa ggt aag atc gtc gat ctt ctg act gaa cgg gct aaa tat gtt gta       96
Lys Gly Lys Ile Val Asp Leu Leu Thr Glu Arg Ala Lys Tyr Val Val
            20                  25                  30
cgc tac cag ggc ggt cac aac gca ggc cat act ctc gta atc aac ggt      144
Arg Tyr Gln Gly Gly His Asn Ala Gly His Thr Leu Val Ile Asn Gly
        35                  40                  45
gaa aaa acc gtt ctc cat ctt att cca tca ggt att ctc cgc gag aat      192
Glu Lys Thr Val Leu His Leu Ile Pro Ser Gly Ile Leu Arg Glu Asn
    50                  55                  60
gta acc agc atc atc ggt aac ggt gtt gtg ctg tct ccg gcc gcg ctg      240
Val Thr Ser Ile Ile Gly Asn Gly Val Val Leu Ser Pro Ala Ala Leu
65                  70                  75                  80
atg aaa gag atg aaa gaa ctg gaa gac cgt ggc atc ccc gtt cgt gag      288
Met Lys Glu Met Lys Glu Leu Glu Asp Arg Gly Ile Pro Val Arg Glu
                85                  90                  95
cgt ctg ctg ctg tct gaa gca tgt ccg ctg atc ctt gat tat cac gtt      336
Arg Leu Leu Leu Ser Glu Ala Cys Pro Leu Ile Leu Asp Tyr His Val
            100                 105                 110
gcg ctg gat aac gcg cgt gag aaa gcg cgt ggc gcg aaa gcg atc ggc      384
Ala Leu Asp Asn Ala Arg Glu Lys Ala Arg Gly Ala Lys Ala Ile Gly
        115                 120                 125
acc acc ggt cgt ggt atc ggg cct gct tat gaa gat aaa gta gca cgt      432
Thr Thr Gly Arg Gly Ile Gly Pro Ala Tyr Glu Asp Lys Val Ala Arg
    130                 135                 140
cgc ggt ctg cgt gtt ggc gac ctt ttc gac aaa gaa acc ttc gct gaa      480
Arg Gly Leu Arg Val Gly Asp Leu Phe Asp Lys Glu Thr Phe Ala Glu
145                 150                 155                 160
aaa ctg aaa gaa gtg atg gaa tat cac aac ttc cag ttg gtt aac tac      528
Lys Leu Lys Glu Val Met Glu Tyr His Asn Phe Gln Leu Val Asn Tyr
                165                 170                 175
tac aaa gct gaa gcg gtt gat tac cag aaa gtt ctg gat gat acg atg      576
Tyr Lys Ala Glu Ala Val Asp Tyr Gln Lys Val Leu Asp Asp Thr Met
            180                 185                 190
gct gtt gcc gac atc ctg act tct atg gtg gtt gac gtt tct gac ctg      624
Ala Val Ala Asp Ile Leu Thr Ser Met Val Val Asp Val Ser Asp Leu
        195                 200                 205
ctc gac cag gcg cgt cag cgt ggc gat ttc gtc atg ttt gaa ggt gcg      672
Leu Asp Gln Ala Arg Gln Arg Gly Asp Phe Val Met Phe Glu Gly Ala

210                 215                 220
cag ggt acg ctg ctg gat atc gac cac ggt act tat ccg tac gta act         720
Gln Gly Thr Leu Leu Asp Ile Asp His Gly Thr Tyr Pro Tyr Val Thr
225                 230                 235                 240
tct tcc aac acc act gct ggt ggc gtg gcg acc ggt tcc ggc ctg ggc         768
Ser Ser Asn Thr Thr Ala Gly Gly Val Ala Thr Gly Ser Gly Leu Gly
                245                 250                 255
ccg cgt tat gtt gat tac gtt ctg ggt atc ctc aaa gct tac tcc act         816
Pro Arg Tyr Val Asp Tyr Val Leu Gly Ile Leu Lys Ala Tyr Ser Thr
            260                 265                 270
cgt gta ggt gca ggt ccg ttc ccg acc gaa ctg ttt gat gaa act ggc         864
Arg Val Gly Ala Gly Pro Phe Pro Thr Glu Leu Phe Asp Glu Thr Gly
        275                 280                 285
gag ttc ctc tgc aag cag ggt aac gaa ttc ggc gca act acg ggg cgt         912
Glu Phe Leu Cys Lys Gln Gly Asn Glu Phe Gly Ala Thr Thr Gly Arg
    290                 295                 300
cgt cgt cgt acc ggc tgg ctg gac acc gtt gcc gtt cgt cgt gcg gta         960
Arg Arg Arg Thr Gly Trp Leu Asp Thr Val Ala Val Arg Arg Ala Val
305                 310                 315                 320
cag ctg aac tcc ctg tct ggc ttc tgc ctg act aaa ctg gac gtt ctg        1008
Gln Leu Asn Ser Leu Ser Gly Phe Cys Leu Thr Lys Leu Asp Val Leu
                325                 330                 335
gat ggc ctg aaa gag gtt aaa ctc tgc gtg gct tac cgt atg ccg gat        1056
Asp Gly Leu Lys Glu Val Lys Leu Cys Val Ala Tyr Arg Met Pro Asp
            340                 345                 350
ggt cgc gaa gtg act acc act ccg ctg gca gct gac gac tgg aaa ggt        1104
Gly Arg Glu Val Thr Thr Thr Pro Leu Ala Ala Asp Asp Trp Lys Gly
        355                 360                 365
gta gag ccg att tac gaa acc atg ccg ggc tgg tct gaa tcc acc ttc        1152
Val Glu Pro Ile Tyr Glu Thr Met Pro Gly Trp Ser Glu Ser Thr Phe
    370                 375                 380
ggc gtg aaa gat cgt agc ggc ctg ccg cag gcg gcg ctg aac tat atc        1200
Gly Val Lys Asp Arg Ser Gly Leu Pro Gln Ala Ala Leu Asn Tyr Ile
385                 390                 395                 400
aag cgt att gaa gag ctg act ggt gtg ccg atc gat atc atc tct acc        1248
Lys Arg Ile Glu Glu Leu Thr Gly Val Pro Ile Asp Ile Ile Ser Thr
                405                 410                 415
ggt ccg gat cgt act gaa acc atg att ctg cgc gac ccg ttc gac gcg        1296
Gly Pro Asp Arg Thr Glu Thr Met Ile Leu Arg Asp Pro Phe Asp Ala
            420                 425                 430
taa                                                                    1299


<210>  34
<211>  432
<212>  PRT
<213>  Escherichia coli

<400>  34
Met Gly Asn Asn Val Val Val Leu Gly Thr Gln Trp Gly Asp Glu Gly
1               5                   10                  15
Lys Gly Lys Ile Val Asp Leu Leu Thr Glu Arg Ala Lys Tyr Val Val
            20                  25                  30
Arg Tyr Gln Gly Gly His Asn Ala Gly His Thr Leu Val Ile Asn Gly
        35                  40                  45
Glu Lys Thr Val Leu His Leu Ile Pro Ser Gly Ile Leu Arg Glu Asn
    50                  55                  60
Val Thr Ser Ile Ile Gly Asn Gly Val Val Leu Ser Pro Ala Ala Leu
65                  70                  75                  80
Met Lys Glu Met Lys Glu Leu Glu Asp Arg Gly Ile Pro Val Arg Glu
                85                  90                  95
Arg Leu Leu Leu Ser Glu Ala Cys Pro Leu Ile Leu Asp Tyr His Val
            100                 105                 110
Ala Leu Asp Asn Ala Arg Glu Lys Ala Arg Gly Ala Lys Ala Ile Gly
        115                 120                 125
Thr Thr Gly Arg Gly Ile Gly Pro Ala Tyr Glu Asp Lys Val Ala Arg
    130                 135                 140
Arg Gly Leu Arg Val Gly Asp Leu Phe Asp Lys Glu Thr Phe Ala Glu
145                 150                 155                 160

Lys Leu Lys Glu Val Met Glu Tyr His Asn Phe Gln Leu Val Asn Tyr
                165                 170                 175
Tyr Lys Ala Glu Ala Val Asp Tyr Gln Lys Val Leu Asp Asp Thr Met
            180                 185                 190
Ala Val Ala Asp Ile Leu Thr Ser Met Val Val Asp Val Ser Asp Leu
        195                 200                 205
Leu Asp Gln Ala Arg Gln Arg Gly Asp Phe Val Met Phe Glu Gly Ala
    210                 215                 220
Gln Gly Thr Leu Leu Asp Ile Asp His Gly Thr Tyr Pro Tyr Val Thr
225                 230                 235                 240
Ser Ser Asn Thr Thr Ala Gly Gly Val Ala Thr Gly Ser Gly Leu Gly
                245                 250                 255
Pro Arg Tyr Val Asp Tyr Val Leu Gly Ile Leu Lys Ala Tyr Ser Thr
            260                 265                 270
Arg Val Gly Ala Gly Pro Phe Pro Thr Glu Leu Phe Asp Glu Thr Gly
        275                 280                 285
Glu Phe Leu Cys Lys Gln Gly Asn Glu Phe Gly Ala Thr Thr Gly Arg
    290                 295                 300
Arg Arg Arg Thr Gly Trp Leu Asp Thr Val Ala Val Arg Arg Ala Val
305                 310                 315                 320
Gln Leu Asn Ser Leu Ser Gly Phe Cys Leu Thr Lys Leu Asp Val Leu
                325                 330                 335
Asp Gly Leu Lys Glu Val Lys Leu Cys Val Ala Tyr Arg Met Pro Asp
            340                 345                 350
Gly Arg Glu Val Thr Thr Thr Pro Leu Ala Ala Asp Asp Trp Lys Gly
        355                 360                 365
Val Glu Pro Ile Tyr Glu Thr Met Pro Gly Trp Ser Glu Ser Thr Phe
    370                 375                 380
Gly Val Lys Asp Arg Ser Gly Leu Pro Gln Ala Ala Leu Asn Tyr Ile
385                 390                 395                 400
Lys Arg Ile Glu Glu Leu Thr Gly Val Pro Ile Asp Ile Ile Ser Thr
                405                 410                 415
Gly Pro Asp Arg Thr Glu Thr Met Ile Leu Arg Asp Pro Phe Asp Ala
            420                 425                 430

<210>  35
<211>  54
<212>  DNA
<213>  Artificial sequence

<220>
<223>  primer1 for disrupting purA

<400>  35
attttgaaaa atgggtaaca acgtcgtcgt actgggcgct caagttagta taaa          54


<210>  36
<211>  54
<212>  DNA
<213>  Artificial sequence

<220>
<223>  primer2 for disrupting purA

<400>  36
gtcgaacggg tcgcgcagaa tcatggtttc agtacgtgaa gcctgctttt ttat          54


<210>  37
<211>  1002
<212>  DNA
<213>  Escherichia coli


<220>
<221>  CDS
<222>  (1)..(999)

<400> 37
atg att gat acc acc ctg cca tta act gat atc cat cgc cac ctt gat 48
Met Ile Asp Thr Thr Leu Pro Leu Thr Asp Ile His Arg His Leu Asp
1               5                   10                  15
ggc aac att cgt ccc cag acc att ctt gaa ctt ggc cgc cag tat aat 96
Gly Asn Ile Arg Pro Gln Thr Ile Leu Glu Leu Gly Arg Gln Tyr Asn
            20                  25                  30
atc tcg ctt cct gca caa tcc ctg gaa aca ctg att ccc cac gtt cag 144
Ile Ser Leu Pro Ala Gln Ser Leu Glu Thr Leu Ile Pro His Val Gln
        35                  40                  45
gtc att gcc aac gaa ccc gat ctg gtg agc ttt ctg acc aaa ctt gac 192
Val Ile Ala Asn Glu Pro Asp Leu Val Ser Phe Leu Thr Lys Leu Asp
    50                  55                  60
tgg ggc gtt aaa gtt ctc gcc tct ctt gat gcc tgt cgc cgc gtg gca 240
Trp Gly Val Lys Val Leu Ala Ser Leu Asp Ala Cys Arg Arg Val Ala
65                  70                  75                  80
ttt gaa aac att gaa gat gca gcc cgt cac ggc ctg cac tat gtc gag 288
Phe Glu Asn Ile Glu Asp Ala Ala Arg His Gly Leu His Tyr Val Glu
                85                  90                  95
ctg cgt ttt tca cca ggc tac atg gca atg gca cat cag ctg cct gta 336
Leu Arg Phe Ser Pro Gly Tyr Met Ala Met Ala His Gln Leu Pro Val
            100                 105                 110
gcg ggt gtt gtc gaa gcg gtg atc gat ggc gta cgt gaa ggt tgc cgc 384
Ala Gly Val Val Glu Ala Val Ile Asp Gly Val Arg Glu Gly Cys Arg
        115                 120                 125
acc ttt ggt gtg cag gcg aag ctt atc ggc att atg agc cgg acc ttc 432
Thr Phe Gly Val Gln Ala Lys Leu Ile Gly Ile Met Ser Arg Thr Phe
    130                 135                 140
ggc gaa gcc gcc tgt cag caa gag ctg gag gcc ttt tta gcc cac cgt 480
Gly Glu Ala Ala Cys Gln Gln Glu Leu Glu Ala Phe Leu Ala His Arg
145                 150                 155                 160
gac cag att acc gca ctt gat tta gcc ggt gat gaa ctt ggt ttc ccg 528
Asp Gln Ile Thr Ala Leu Asp Leu Ala Gly Asp Glu Leu Gly Phe Pro
                165                 170                 175
gga agt ctg ttc ctt tct cac ttc aac cgc gcg cgt gat gcg ggc tgg 576
Gly Ser Leu Phe Leu Ser His Phe Asn Arg Ala Arg Asp Ala Gly Trp
            180                 185                 190
cat att acc gtc cat gca ggc gaa gct gcc ggg ccg gaa agc atc tgg 624
His Ile Thr Val His Ala Gly Glu Ala Ala Gly Pro Glu Ser Ile Trp
        195                 200                 205
cag gcg att cgt gaa ctg ggt gcg gag cgt att gga cat ggc gta aaa 672
Gln Ala Ile Arg Glu Leu Gly Ala Glu Arg Ile Gly His Gly Val Lys
    210                 215                 220
gcc att gaa gat cgg gcg ctg atg gat ttt ctc gcc gag caa caa att 720
Ala Ile Glu Asp Arg Ala Leu Met Asp Phe Leu Ala Glu Gln Gln Ile
225                 230                 235                 240
ggt att gaa tcc tgt ctg acc tcc aat att cag acc agc acc gta gca 768
Gly Ile Glu Ser Cys Leu Thr Ser Asn Ile Gln Thr Ser Thr Val Ala
                245                 250                 255
gag ctg gct gca cat ccg ctg aaa acg ttc ctt gag cat ggc att cgt 816
Glu Leu Ala Ala His Pro Leu Lys Thr Phe Leu Glu His Gly Ile Arg
            260                 265                 270
gcc agc att aac act gac gat ccc ggc gta cag gga gtg gat atc att 864
Ala Ser Ile Asn Thr Asp Asp Pro Gly Val Gln Gly Val Asp Ile Ile
        275                 280                 285
cac gaa tat acc gtt gcc gcg cca gct gct ggg tta tcc cgc gag caa 912
His Glu Tyr Thr Val Ala Ala Pro Ala Ala Gly Leu Ser Arg Glu Gln
    290                 295                 300
atc cgc cag gca cag att aat ggt ctg gaa atg gct ttc ctc agc gct 960
Ile Arg Gln Ala Gln Ile Asn Gly Leu Glu Met Ala Phe Leu Ser Ala
305                 310                 315                 320
gag gaa aaa cgc gca ctg cga gaa aaa gtc gcc gcg aag taa 1002
Glu Glu Lys Arg Ala Leu Arg Glu Lys Val Ala Ala Lys
                325                 330

<210> 38
<211> 333
<212> PRT

<213>  Escherichia coli

<400>  38
Met Ile Asp Thr Thr Leu Pro Leu Thr Asp Ile His Arg His Leu Asp
1               5                   10                  15
Gly Asn Ile Arg Pro Gln Thr Ile Leu Glu Leu Gly Arg Gln Tyr Asn
            20                  25                  30
Ile Ser Leu Pro Ala Gln Ser Leu Glu Thr Leu Ile Pro His Val Gln
        35                  40                  45
Val Ile Ala Asn Glu Pro Asp Leu Val Ser Phe Leu Thr Lys Leu Asp
    50                  55                  60
Trp Gly Val Lys Val Leu Ala Ser Leu Asp Ala Cys Arg Arg Val Ala
65                  70                  75                  80
Phe Glu Asn Ile Glu Asp Ala Ala Arg His Gly Leu His Tyr Val Glu
                85                  90                  95
Leu Arg Phe Ser Pro Gly Tyr Met Ala Met Ala His Gln Leu Pro Val
            100                 105                 110
Ala Gly Val Val Glu Ala Val Ile Asp Gly Val Arg Glu Gly Cys Arg
        115                 120                 125
Thr Phe Gly Val Gln Ala Lys Leu Ile Gly Ile Met Ser Arg Thr Phe
    130                 135                 140
Gly Glu Ala Ala Cys Gln Gln Glu Leu Glu Ala Phe Leu Ala His Arg
145                 150                 155                 160
Asp Gln Ile Thr Ala Leu Asp Leu Ala Gly Asp Glu Leu Gly Phe Pro
                165                 170                 175
Gly Ser Leu Phe Leu Ser His Phe Asn Arg Ala Arg Asp Ala Gly Trp
            180                 185                 190
His Ile Thr Val His Ala Gly Glu Ala Ala Gly Pro Glu Ser Ile Trp
        195                 200                 205
Gln Ala Ile Arg Glu Leu Gly Ala Glu Arg Ile Gly His Gly Val Lys
    210                 215                 220
Ala Ile Glu Asp Arg Ala Leu Met Asp Phe Leu Ala Glu Gln Gln Ile
225                 230                 235                 240
Gly Ile Glu Ser Cys Leu Thr Ser Asn Ile Gln Thr Ser Thr Val Ala
                245                 250                 255
Glu Leu Ala Ala His Pro Leu Lys Thr Phe Leu Glu His Gly Ile Arg
            260                 265                 270
Ala Ser Ile Asn Thr Asp Asp Pro Gly Val Gln Gly Val Asp Ile Ile
        275                 280                 285
His Glu Tyr Thr Val Ala Ala Pro Ala Ala Gly Leu Ser Arg Glu Gln
    290                 295                 300
Ile Arg Gln Ala Gln Ile Asn Gly Leu Glu Met Ala Phe Leu Ser Ala
305                 310                 315                 320
Glu Glu Lys Arg Ala Leu Arg Glu Lys Val Ala Ala Lys
                325                 330

<210>  39
<211>  54
<212>  DNA
<213>  Artificial sequence

<220>
<223>  primer1 for disrupting add

<400>  39
gagtgcgacc atgattgata ccaccctgcc attaaccgct caagttagta taaa          54


<210>  40
<211>  54
<212>  DNA
<213>  Artificial sequence

<220>
<223>  primer2 for disrupting add

<400>  40
cttcgcggcg actttttctc gcagtgcgcg tttttctgaa gcctgctttt ttat          54

<210>  41
<211>  753
<212>  DNA
<213>  Escherichia coli

<220>
<221>  CDS
<222>  (1)..(750)

<400>  41
atg acc att aaa aat gta att tgc gat atc gac ggc gtg ctg atg cac      48
Met Thr Ile Lys Asn Val Ile Cys Asp Ile Asp Gly Val Leu Met His
1               5                   10                  15
gat aac gtc gcc gta ccg ggt gca gcg gaa ttt ttg cac ggg att atg      96
Asp Asn Val Ala Val Pro Gly Ala Ala Glu Phe Leu His Gly Ile Met
            20                  25                  30
gat aaa ggc ctg ccg ctg gtg ttg ctg acc aac tat cct tcg cag act      144
Asp Lys Gly Leu Pro Leu Val Leu Leu Thr Asn Tyr Pro Ser Gln Thr
        35                  40                  45
ggg caa gat ctg gcg aac cgc ttt gcc acc gca ggt gtc gat gta cct      192
Gly Gln Asp Leu Ala Asn Arg Phe Ala Thr Ala Gly Val Asp Val Pro
    50                  55                  60
gac agc gtg ttt tat acc tct gcg atg gcg act gcc gat ttc ctg cgt      240
Asp Ser Val Phe Tyr Thr Ser Ala Met Ala Thr Ala Asp Phe Leu Arg
65                  70                  75                  80
cgc cag gaa ggc aag aaa gcg tat gtg gtg ggc gaa ggc gca ctg att      288
Arg Gln Glu Gly Lys Lys Ala Tyr Val Val Gly Glu Gly Ala Leu Ile
                85                  90                  95
cat gaa ctg tac aaa gcc ggt ttc act att acc gat gtg aac cct gat      336
His Glu Leu Tyr Lys Ala Gly Phe Thr Ile Thr Asp Val Asn Pro Asp
            100                 105                 110
ttc gtg att gtt ggc gaa acg cgt tcc tac aac tgg gac atg atg cat      384
Phe Val Ile Val Gly Glu Thr Arg Ser Tyr Asn Trp Asp Met Met His
        115                 120                 125
aaa gca gcc tat ttc gtc gct aac ggt gca cgt ttt atc gcc acc aat      432
Lys Ala Ala Tyr Phe Val Ala Asn Gly Ala Arg Phe Ile Ala Thr Asn
    130                 135                 140
ccg gac acc cac ggg cgc ggt ttt tat ccc gct tgt ggc gcg ttg tgt      480
Pro Asp Thr His Gly Arg Gly Phe Tyr Pro Ala Cys Gly Ala Leu Cys
145                 150                 155                 160
gca ggg att gag aaa atc tcc ggg cgc aaa ccg ttc tat gtt ggt aag      528
Ala Gly Ile Glu Lys Ile Ser Gly Arg Lys Pro Phe Tyr Val Gly Lys
                165                 170                 175
ccc agc ccg tgg atc atc cgc gca gca tta aac aaa atg cag gcg cat      576
Pro Ser Pro Trp Ile Ile Arg Ala Ala Leu Asn Lys Met Gln Ala His
            180                 185                 190
tcg gaa gaa acg gtg att gtc ggc gat aac ctg cgt acc gat att ctg      624
Ser Glu Glu Thr Val Ile Val Gly Asp Asn Leu Arg Thr Asp Ile Leu
        195                 200                 205
gcc ggc ttc cag gca ggt ctg gag acg att ctg gtg ctt tct ggt gtt      672
Ala Gly Phe Gln Ala Gly Leu Glu Thr Ile Leu Val Leu Ser Gly Val
    210                 215                 220
tcg tcg ctc gac gat atc gac agt atg cct ttc cgc ccc agc tgg att      720
Ser Ser Leu Asp Asp Ile Asp Ser Met Pro Phe Arg Pro Ser Trp Ile
225                 230                 235                 240
tac ccg tca gtc gct gaa atc gac gtt atc tga                          753
Tyr Pro Ser Val Ala Glu Ile Asp Val Ile
                245                 250

<210>  42
<211>  250
<212>  PRT
<213>  Escherichia coli

<400>  42
Met Thr Ile Lys Asn Val Ile Cys Asp Ile Asp Gly Val Leu Met His
1               5                   10                  15

Asp Asn Val Ala Val Pro Gly Ala Ala Glu Phe Leu His Gly Ile Met
            20                  25                  30
Asp Lys Gly Leu Pro Leu Val Leu Leu Thr Asn Tyr Pro Ser Gln Thr
        35                  40                  45
Gly Gln Asp Leu Ala Asn Arg Phe Ala Thr Ala Gly Val Asp Val Pro
    50                  55                  60
Asp Ser Val Phe Tyr Thr Ser Ala Met Ala Thr Ala Asp Phe Leu Arg
65                  70                  75                  80
Arg Gln Glu Gly Lys Lys Ala Tyr Val Val Gly Glu Gly Ala Leu Ile
                85                  90                  95
His Glu Leu Tyr Lys Ala Gly Phe Thr Ile Thr Asp Val Asn Pro Asp
            100                 105                 110
Phe Val Ile Val Gly Glu Thr Arg Ser Tyr Asn Trp Asp Met Met His
        115                 120                 125
Lys Ala Ala Tyr Phe Val Ala Asn Gly Ala Arg Phe Ile Ala Thr Asn
    130                 135                 140
Pro Asp Thr His Gly Arg Gly Phe Tyr Pro Ala Cys Gly Ala Leu Cys
145                 150                 155                 160
Ala Gly Ile Glu Lys Ile Ser Gly Arg Lys Pro Phe Tyr Val Gly Lys
                165                 170                 175
Pro Ser Pro Trp Ile Ile Arg Ala Ala Leu Asn Lys Met Gln Ala His
            180                 185                 190
Ser Glu Glu Thr Val Ile Val Gly Asp Asn Leu Arg Thr Asp Ile Leu
        195                 200                 205
Ala Gly Phe Gln Ala Gly Leu Glu Thr Ile Leu Val Leu Ser Gly Val
    210                 215                 220
Ser Ser Leu Asp Asp Ile Asp Ser Met Pro Phe Arg Pro Ser Trp Ile
225                 230                 235                 240
Tyr Pro Ser Val Ala Glu Ile Asp Val Ile
                245                 250


<210>  43
<211>  54
<212>  DNA
<213>  Artificial sequence

<220>
<223>  primer1 for disrupting nagD

<400>  43
tgggtagtcc atgaccatta aaaatgtaat ttgcgacgct caagttagta taaa          54


<210>  44
<211>  54
<212>  DNA
<213>  Artificial sequence

<220>
<223>  primer2 for disrupting nagD

<400>  44
aacgtcgatt tcagcgactg acgggtaaat ccagcttgaa gcctgctttt ttat          54